(19) Europäisches Patentamt / European Patent Office / Office européen des brevets

(11) **EP 2 516 668 B1**

(12) **EUROPEAN PATENT SPECIFICATION**

(45) Date of publication and mention of the grant of the patent:
**06.04.2016 Bulletin 2016/14**

(21) Application number: **10813131.9**

(22) Date of filing: **22.12.2010**

(51) Int Cl.:
*C12Q 1/25* (2006.01)     *C12Q 1/48* (2006.01)

(86) International application number:
**PCT/IB2010/056006**

(87) International publication number:
**WO 2011/077387 (30.06.2011 Gazette 2011/26)**

(54) **INHIBITION OF THE ACTIVITY OF KINASE AND SYNTHETASE ENZYMES**

HEMMUNG DER AKTIVITÄT VON KINASE- UND SYNTHETASEENZYMEN

INHIBITION DE L'ACTIVITÉ D'ENZYMES KINASES ET SYNTHÉTASES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2009 ZA 200909160**

(43) Date of publication of application:
**31.10.2012 Bulletin 2012/44**

(73) Proprietor: **CSIR**
**0002 Pretoria (ZA)**

(72) Inventors:
- **ROTH, Robyn**
  **2191 Sandton (ZA)**
- **KENYON, Colin, Peter**
  **2188 Randburg (ZA)**

(74) Representative: **Boult Wade Tennant**
**Verulam Gardens**
**70 Gray's Inn Road**
**London WC1X 8BT (GB)**

(56) References cited:
**EP-A2- 1 396 491     WO-A1-01/37835**

- **TAYLOR P ET AL:** "Ligand discovery and virtual screening using the program LIDAEUS.", BRITISH JOURNAL OF PHARMACOLOGY MAR 2008 LNKD- PUBMED:18037921, vol. 153 Suppl 1, March 2008 (2008-03), pages S55-S67, XP002637439, ISSN: 0007-1188
- **KIM D K ET AL:** "Synthesis and biological evaluation of trisubstituted imidazole derivatives as inhibitors of p38alpha mitogen-activated protein kinase", BIOORGANIC & MEDICINAL CHEMISTRY LETTERS, PERGAMON, ELSEVIER SCIENCE, GB, vol. 18, no. 14, 15 July 2008 (2008-07-15) , pages 4006-4010, XP022852885, ISSN: 0960-894X, DOI: DOI:10.1016/J.BMCL.2008.06.007 [retrieved on 2008-06-06]
- **LAUFER STEFAN A ET AL:** "Design, synthesis, and biological evaluation of novel Tri- and tetrasubstituted imidazoles as highly potent and specific ATP-mimetic inhibitors of p38 MAP kinase: focus on optimized interactions with the enzyme's surface-exposed front region.", JOURNAL OF MEDICINAL CHEMISTRY 24 JUL 2008 LNKD- PUBMED:18578517, vol. 51, no. 14, 24 July 2008 (2008-07-24) , pages 4122-4149, XP002634938, ISSN: 1520-4804
- **Kai Liu ET AL:** "Solid-state deuterium NMR of imidazole ligands in cytochrome c peroxidase", Journal of the American Chemical Society [H.W. Wilson - GS], 7 October 1998 (1998-10-07), page 10199, XP055172193, Retrieved from the Internet: URL:http://search.proquest.com/docview/220 740304

## Description

THIS INVENTION relates to the inhibition of the activity of kinase and synthetase enzymes. More particularly, the invention relates to an *in vitro* method of inhibiting the activity of a kinase or a synthetase, to a method of generating a compound that inhibits the activity of a kinase or a synthetase, and to a method of screening a test compound *in vitro* to determine whether or not it inhibits the activity of a kinase or a synthetase.

Adenylate kinase (AK) contributes to the homeostasis of adenine nucleotides by maintaining intracellular nucleotide pools. Six isoenzymes of adenylate kinase have been identified in mammalian cells with different subcellular localization and substrate specificity. Adenylate kinase catalyses the reaction:

$$ATP + AMP \rightarrow 2ADP$$

where ATP is adenosine triphosphate, AMT is adenosine monophosphate and ADP is adenosine diphosphate. The adenylate kinases (ATP:AMP phosphotransferases, EC 2.7.4.3) catalyze the reversible transfer of the $\gamma$-phosphate group from a phosphate donor (ATP, GTP, CTP, ITP) to AMP. The phosphate donor is usually ATP. There is a size variation among the isoenzymes: AK1, AK5 and AK6 are short type adenylate kinases while AK2, AK3 and AK4 are long type adenylate kinases containing a 27 amino acid insertion sequence in the central portion of the peptide. The mammalian adenylate kinases have a distinct intracellular compartmentalization, with AK1 in the cytosol, AK2 in the inter-membrane space of mitochondria, AK3 in the mitochondrial matrix, AK4 being mitochondrial in nature, AK5 (unknown localization) and AK6 in the nucleus. AK3 present in the mitochondrial matrix has a preference for GTP over ATP.

It is often desired to regulate the activity of kinases and synthetases, and this can, for example, be done by binding an active site of a kinase, such as AK, or a synthetase, such as glutamine synthetase, to an imidazole moiety, eg as found in ATP. An object of this invention is to provide a means whereby the level of inhibition of the activity of a kinase or a synthetase can be enhanced. In Kim DK *et al.* (2008) Synthesis and biological evaluation of trisubstituted imidazole derivatives as inhibitors of p38alpha mitogen-activated protein kinase, Bioorganic and Medicinal Chemistry Letters 18(14): 4006-4010; Laufer SA et al. (2008) Design, synthesis, and biological evaluation of novel Tri- and tetrasubstituted imidazoles as highly potent and specific ATP-mimetic inhibitors of p38 MAP kinase: focus on optimized interactions with the enzyme's surface-exposed front region, Journal of Medicinal Chemistry 51(14): 4122-4149; EP 1 396 491 A2 *2-substituted 4,5-diaryl-imidazoles and their used as p38 MAP kinase inhibitors*; and WO 01/37835 A1 *Novel compounds,* the use of imidazole moieties as kinase inhibitors is disclosed. Thus, according to a first aspect of the invention, there is provided an *in vitro* method of inhibiting the activity of a kinase or a synthetase, the method including binding an active site of the kinase or synthetase with a deuterated imidazole moiety, thereby inhibiting the activity of the kinase or the synthetase.

The method may be applied to a kinase. Examples of suitable kinases are adenylate kinase (AK), shikimate kinase (SK), pyruvate kinase (PK), hexokinase (HXK), aspartokinase (ASK), creatine kinase (CK), glycerate kinase, acetate kinase, glutamine synthetase, such as adenylate glutamineate synthetase, and/or deadenylated glutamine synthetase, phosphofructokinase, and isoforms thereof. In a particular embodiment of the invention, the kinase may be adenylate kinase (AK). Its sequence comprises conserved residues Arg 97, Glu 98, Arg 128 and Asp 180.

Instead, the method may be applied to a synthetase. The synthetase may then, for example, be glutamine synthetase (GS).

Generally, the kinase or the synthetase, or an isoenzyme thereof, may comprise amino acid residues identical to, or similar to, conserved adenylate kinase residues Arg 97, Glu 98, Arg 128 and Asp 180, at positions equivalent to the positions of these residues in adenylate kinase.

More particularly, the deuterated imidazole moiety may be provided by a nucleotide. In one embodiment of the invention, the nucleotide may be adenosine triphosphate (ATP), adenosine diphosphate (ADP) or adenosine monophosphate (AMP), with the nucleotide having an immonium moiety induced at position N7, so that a carbene is induced at the C8 position, and with deuteration being effected at the C8 position. However, in another embodiment of the invention, the nucleotide may be a compound which is deuterated at a position equivalent to C8 in ATP, ADP and AMP.

The extent of regulation of each form (isoenzymes) of the kinase enzyme or the synthetase enzyme is different based on the level of the Kinetic Isotope Effect (KIE) and the kinetics of the enzymes and the structure of the active site of the kinase. When the kinase is adenylate kinase, the reaction catalysed by adenylate kinase is:

$$ATP + AMP \rightarrow 2ADP$$

The assay is carried out in the presence of ATP and AMP measuring the formation of ADP. The KIE is obtained when comparing the enzyme activity of the adenylate kinase enzyme when the reaction is carried out in the presence of ATP and deuterated ATP. A kinetic isotope effect is also obtained when comparing the activity of the enzyme when the reaction is carried out in the presence of AMP and deuterated AMP. A further KIE is obtained when both deuterated ATP and deuterated AMP are used in the reaction. It is significant that for all these enzymes, the KIE occurs as a result of the change in using ATP over deuterated ATP or AMP over deuterated AMP. The KIE is obtained by dividing the activity of the enzyme in the presence of the proteated species ($v_H$) by the enzyme activity in the presence of the deuterated species ($v_D$) and is $v_H/v_D = 0.5$. The KIE is found over a broad range of ATP concentration enzyme activity profile. The extent of the regulation of AK is also ATP concentration dependent. The proposed basic reaction mechanisms by which all forms of kinase are regulated occurs via either the formation of an immonium species at N7, which in turn induces the formation of a carbene at C8 or via the delocalization of electrons away from the C8 rendering the C8 more acidic. An immonium species may be induced at N7 by (1) protonation via a coordinated $HCO_3^-$, (2) carboxylation of the N7, or (3) the coordination of an amino acid side chain within the active site such as a arginine, glutamine, lysine or histidine. The different forms of regulation are then based on the mechanism by which the carbene formed at C8 is stabilized and the mechanism by which the C8-H is deprotonated or via the interaction of the protein coordinated amino acid side chains affecting the delocalization of electrons around the adenyl ring of the nucleotide.

The formation of the immonium species at N7 then facilitates the deprotonation of C8 via a coordinated amino acid residue. The resulting carbene intermediate is stabilised by the putative bond formation by a coordinated amino acid and C8. In some kinases the coordination may be mediated by the presence of a divalent metal ion such as $Mn^{2+}$ coordinated into the ATP coordination complex.

In particular, it was found that the activity of AK1 is affected by the deuteration of ATP and AMP at the C8 position. The role of deuteration of ATP/AMP in causing a KIE in AK1 demonstrates that the binding mechanism of nucleotides to the active sites of kinases is similar and the imidazole moiety is implicated in all cases. "Imidazole" moiety-containing compounds are found to affect the regulation of AK1 and when these compounds are deuterated at the position in the imidazole moiety equivalent to the C8 position in ATP.

Thus, in accordance with the invention, the nucleotides ATP, ADP and AMP will be bound to the AK active site, with the nucleotide being either protonated or unprotonated at position N7. The protonated form is positively charged while the unprotonated form is neutral. The unprotonated form may be protonated within the active site of the enzyme also inducing the formation of an immonium species. In both cases, ie when the nucleotide is in either the protonated form or the neutral form, an immonium species at the N7 position facilitates the induction of a carbene at the C8 position.

In the case of the neutral form of the nucleotide, the method may include creating an immonium species at position N7 through the donation of a protein by the AK enzyme. This facilitates the induction of the carbene at the C8 position by making the C8-H more acidic.

The rendering of the C8-H to become more acidic via the coordination of amino acid side chains and the delocalization of electrons away from C8 is another mechanism by which the regulation of kinases occurs.

According to a second aspect of the invention, there is provided a deuterated imidazole moiety for use in inhibiting the activity of a kinase or a synthetase, by binding an active site of the kinase or synthetase.

According to a third aspect of the invention, there is provided a method of generating a compound that inhibits the activity of a kinase or a synthetase, the method comprising providing a three-dimensional structure of a kinase or a synthetase; and designing, based on the three-dimensional structure, a compound capable of inhibiting the activity of the kinase or the synthetase, the compound comprising a deuterated imidazole moiety.

The compound may be a nucleotide or imidazole containing compound as hereinbefore described.

According to a fourth aspect of the invention, there is provided a method of screening a compound *in vitro* to determine whether or not it inhibits the activity of kinase or a synthetase, the method comprising contacting a kinase or a synthetase with a compound comprising a protonated imidazole moiety; contacting the kinase or the synthetase with the same compound comprising a deuterated imidazole moiety; and determining whether or not the activity of the kinase or synthetase is reduced in the presence of the compound containing the deuterated imidazole moiety relative to the activity of the same kinase or synthetase in the presence of the compound containing the protonated imidazole moiety.

The method of the third or fourth aspect may be applied to a kinase. Examples of suitable kinases are then, as hereinbefore described, adenylate kinase (AK), shikimate kinase (SK), pyruvate kinase (PK), hexokinase (HXK), aspartokinase (ASK), creatine kinase (CK), glycerate kinase, acetate kinase, glutamine synthetase, such as adenylated glutamine synthetase, and/or deadenylated glutamine synthetase, phosphofructokinase, and isoforms thereof. Instead, the method of the third or fourth aspect of the invention may be applied to a synthetase. The synthetase may then, for example, be glutamine synthetase (GC).

As also hereinbefore described, the deuterated imidazole moiety may be provided by a nucleotide or by a nucleotide analogue. The nucleotide may, in one embodiment of the invention be adenosine triphosphate (ATP), adenosine diphosphate (ADP) or adenosine monophosphate (AMP), with the nucleotide having an immonium moiety at position N7, so that a carbene is induced at the C8 position, and with deuteration being effected at the C8 position. However, in another embodiment of the invention, the nucleotide may be a compound which is deuterated at a position equivalent to C8 in ATP, ADP and AMP.

The invention will now be described in more detail with reference to the accompanying non-limiting examples and drawings.

In the drawings

Figure 1 shows, for Example 1, a protein sequence alignment of human adenylate kinase isoforms 1 to 6. KAD1 = P00568 (*SEQ ID NO. 1*), KAD2 = P54819 (*SEQ ID NO. 2), KAD3 = Q9UIJ7 (SEQ ID NO. 3*), KAD4 = P27144 (*SEQ ID NO. 4), KAD5 = Q9Y6K8 (SEQ ID NO. 5*), and KAD6 = Q9Y3D8 (*SEQ ID NO. 6*);

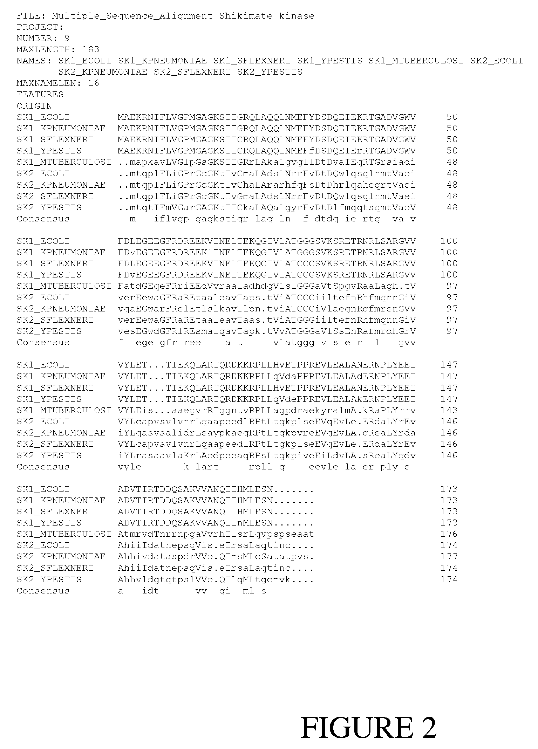

Figure 2 shows, for Example 1, a protein sequence alignment of shikimate kinase isoforms 1 and 2: *E. coli* aroK, SK1_Ecoli = P0A6D7 (*SEQ ID NO. 7), E. coli* aroL SK2_Ecoli = P0A6E1 (*SEQ ID NO. 8); Klebsiella* pneumoniae aroK, SK1_Kpueumoniae = A6TF14 (*SEQ ID NO. 9), Klebsiella* pneumoniae aroL, SK2_Kpueumoniae = A6T5B3 (*SEQ ID NO. 10); Shigella flexneri* aroK, SK1_Ypestis = A6BW25 (*SEQ ID NO. 11), Yersinia pestis* aroL, SK2_Ypestis = A4TPJ4 (*SEQ ID NO. 12); Shigella flexneri* aroK, SK1_Sflexneri = Q0SZS8 (*SEQ ID NO. 13), Shigella flexneri* aroL, SK2_Sflexneri = Q83M66 (*SEQ ID NO. 14), and *Mycobacterium tuberculosis* aroK, SK1_Mtuberculosis = P0A4Z2 (*SEQ ID NO. 15*);

Figure 3 shows, for Example 1, a protein sequence alignment of pyruvate kinase isoforms R, L, M1 and M2. R = P12928 (*SEQ ID NO. 16*), L = P04763 (*SEQ ID NO. 17*), M1 = P11980 (*SEQ ID NO. 18*) and M2 = P11981 (*SEQ ID NO. 19) ;*

Figure 4 shows, for Example 1, a protein sequence alignment of creatine kinase isoforms B-CK, cytoplasmic muscle M-CK, uMT and sMT: CKB = P12277 (*SEQ ID NO. 20*), CKM = P06732 (*SEQ ID NO. 21*), CKMT1A = P12532 (*SEQ ID NO. 22*), CKMT2 = P17540 (*SEQ ID NO. 23*);

Figure 5 shows, for Example 1, a protein sequence alignment of glycerate kinase isoforms 1; *(SEQ ID NO. 24)* and 2 *(SEQ ID NO 25).*;

Figure 6 shows, for Example 1, a protein sequence alignment of human hexokinase isoforms 1-4 (*SEQ ID NO.26 to 29, respectively*);

Figure 7 shows, for Example 1, a protein sequence alignment of *E. coli* aspartokinase isoforms 1-3 (*SEQ ID NO.30 to 32, respectively);*

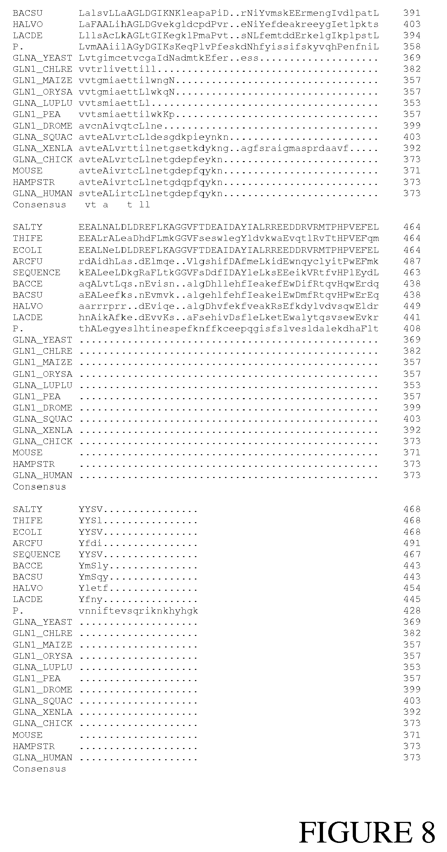

Figure 8 shows, for Example 1, conserved ATP binding domain sequence motifs in GSI-β compared to the domains in GS1-α and GSII: GLNA_SALTY = *Salmonella typhimurium* (P0A1P6, *SEQ ID NO 33*), GLNA_THIFE = *Acidithiobacillus ferrooxidans* (P07804, *SEQ ID NO 34*), GLNA_ECOLI = *E. coli* (P0A9C5; *SEQ ID NO 35*), GLNA_ARCFU = *Archaeoglobus fulgidus* ((O29380; *SEQ ID NO 36*), GLNA_AZOVI = *Azotobacter vinelandii* (P22248; *SEQ ID NO 37*), GLNA_Bacce = *Bacillus cereus* (P19064; *SEQ ID NO 38*), GLNA_BACSU = *Bacillus subtilis* (P12425; *SEQ ID NO 39), GLNA_HALVO = Halobacterium volcanii* (P43386; *SEQ ID NO 40*), GLNA_LACDE = *Lactobacillus delbrueckii* (P45627; *SEQ ID NO 41*)GLNA_PLASMO = *Plasmodium falciparum* (NCBI: XP001352097; *SEQ ID NO 42), GLNA_YEAST = Saccharomyces cerevisiae* (P32288; *SEQ ID NO 43), GLNA_CHLRE - Chlamydomonas reinhardtii* (Q42688; *SEQ ID NO 44*), GLNA_MAIZE - *Zea mays* (P49094; *SEQ ID NO 45), GLNA_ORYSA = Orysa sativa* (P14656; *SEQ ID NO 46), GLNA_LUPLU - Lupinus luteus* (P52782; *SEQ ID NO 47), GLNA_PEA = Pisum sativum* (P19251; *SEQ ID NO 48*), GLNA_DROME - *Drosophila melanogaster* (P20477; *SEQ ID NO 49*), GLNA_SQUAC = *Squalus acanthia* (P41520; *SEQ ID NO 50), GLNA_XENLA = Xenopus laevis* (P51121; *SEQ ID NO 51*), GLNA_CHICK = *Gallus gallus* (P16580; *SEQ ID NO 52), GLNA_MOUSE = Mus musculus* (P15105; *SEQ ID NO 53), HAMSTR = Cricetulus griseus* (P04773; *SEQ ID NO 54*) and GLNA_HUMAN = Homo sapiens (P15104; *SEQ ID NO 55).*

Figure 9 shows, for Example 1, stereo views of the interaction of identified Arg and Glu/Asp interaction in the active sites of in a range of kinase isoenzymes and a synthetase enzyme, namely AK (A), CK (B), PK (C), HXK, SK (D), ASK (E) and GS (F);

Figure 10 shows, for Example 1, structure and numbering of ATP;

Figure 11 shows, for Example 1, the role of Arg97 and Arg44 and the C8H of ATP in the binding of ATP and catalysing phosphoryl transfer in ATP dependent reactions;

Figure 12 shows, for Example 2, the effect of the SDM R97K, R97Q, R97A, R128K, R128Q, R128A, E98L and D180L on the specific activity of AK1 with (A): R97K, R97Q, R128K, R128Q, and (B): R97A, R128A E98L and D180L;

Figure 13 shows, for Example 3, the effect of pH and NaCl (♦), imidazole (■), histidine (▲) and 1,2 dimethyl imidazole (□) on the enzyme activity of AK1. Activity is expressed as ADP produced in mM. Effect of pH on the protonation of imidazole (◇), histidine (△) and 1,2 dimethyl imidazole (●);

Figure 14 shows, for Example 4, the effect of imidazole.HCl and histidine.HCl and deuterated imidazole.HCl and histidine.HCl at a range of pH values on the activity of AK1, (■) 2mM NaCl, (♦) imidazole.HCl, (▲) histidine.HCl, (◇) deuterated imidazole.HCl and (□) deuterated histidine.HCl;

Figure 15 shows, for Example 5, the effect of the relative concentrations of ATP, AMP and C8-D ATP on the activity of AK1;

Figure 16 shows, for Example 5, the effect of a range of concentrations of ATP (♦) and C8-D ATP (◇) on the activity of AK1 and the KIE (■);

Figure 17 shows, for Example 6, the effect of Adenosine 5'-[γ-thio]triphosphate (ATPS) (♦) and deuterated ATPS (◇) on the synthesis of ADP by AK1. The activity of AK1 in the absence of ATPS produced 0.019 mM ADP; and

Figure 18 shows, for Example 7, $^1$H and $^{15}$N NMR HSQC spectrum of AK1 in the presence of $^1$H-ATPS (light shading) and C8-D ATPS (darker shading). Arrows indicate shift changes as a result of the binding of the deuterated analogue.

Figure 19 shows, for Example 8, the effect of the concentration of ATP and C8D-ATP on the specific activity (A) and KIE (B) of *Mycobacteria tuberculosis* shikimate kinase; ● = ATP, ■ = C8D-ATP, a = KIE, b = $KIE_D$.

Figure 20 shows, for Example 8, the effect of the concentration of ATP and C8D-ATP on the specific activity (A) and KIE (B) of *Saccharomyces cerevisiae* hexokinase; ● = ATP, ■ = C8D-ATP a = KIE, b = $KIE_D$.

Figure 21 shows, for Example 8, the effect of the concentration of ATP and C8D-ATP on the specific activity (A) and KIE (B) of *Escherichia coli* acetate kinase; ● = ATP, ■ = C8D-ATP, a = KIE, b = $KIE_D$.

Figure 22 shows, for Example 8, the effect of the concentration of ATP and C8D-ATP on the specific activity (A) and KIE (B) of *Escherichia coli* $GS_0$; ● = ATP, ■ = C8D-ATP a = KIE, b = $KIE_D$.

Figure 23 shows, for Example 8, the effect of the concentration of ATP and C8D-ATP on the specific activity (A) and KIE (B) of *B. stearothermophilus* phosphofructokinase; ● = ATP, ■ = C8D-ATP, a = KIE, b = $KIE_D$.

Figure 24 shows, for Example 8, the effect of the concentration of ATP and C8D-ATP on the specific activity (A) and KIE (B) of *Escherichia coli* adenylylated glutamine synthetase; ● = ATP, ■ = C8D-ATP, a = KIE, b = $KIE_D$.

Figure 25 shows, for Example 8, the effect of low concentrations of ATP on the KIE and $KIE_D$ obtained for A: shikimate kinase, B: hexokinase, C: acetate kinase, D: $GS_0$, E: PFK and F: $GS_{12}$; a = KIE, b = $KIE_D$

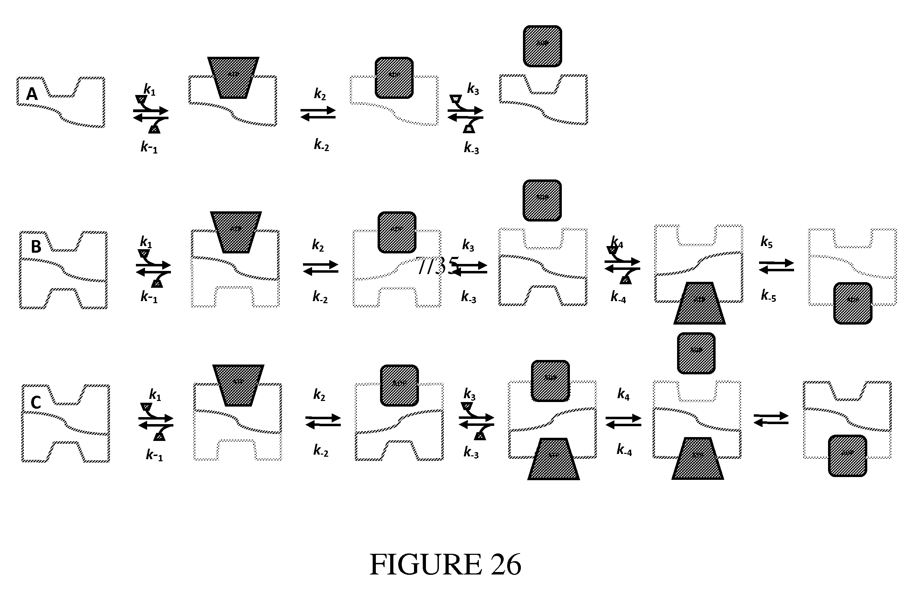

Figure 26 shows, for Example 8, models for the binding of nucleotides to kinases and synthetase enzymes. A: Model for the binding of ATP and release of ADP from monomeric kinase. The current model for oligomeric kinases is based on nonequivalent ligand binding where binding to one monomer affects binding to a second monomer, or coordinated active sites. B: Model based on the rate reaction in the conversion of ATP to ADP with the concomitant conversion of the second binding site to the ATP-binding-form as a result of the release of the ADP. Once the ADP has been released from the first site this changes the affinity of the second site from an ADP binding structure to an ATP binding structure. C: Model based on the rate reaction in the conversion of ATP to ADP with the concomitant conversion of the second binding site to the ATP-binding-form as a result of the conversion of ATP to ADP in the first binding site. Once the ATP is converted ADP this changes the affinity of the second site from an ADP-binding structure to an ATP-binding-form.

EXAMPLE 1

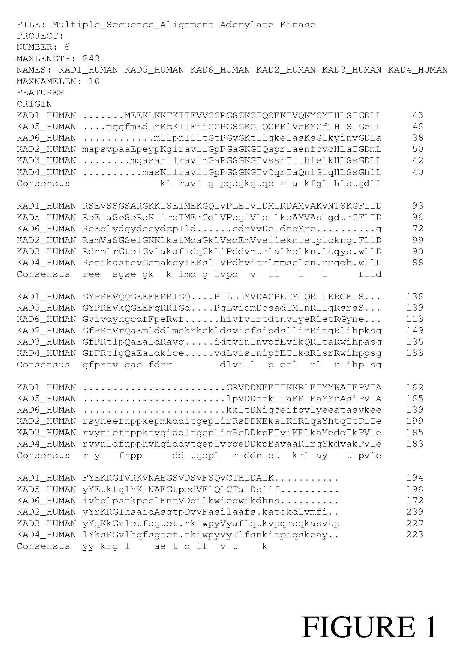

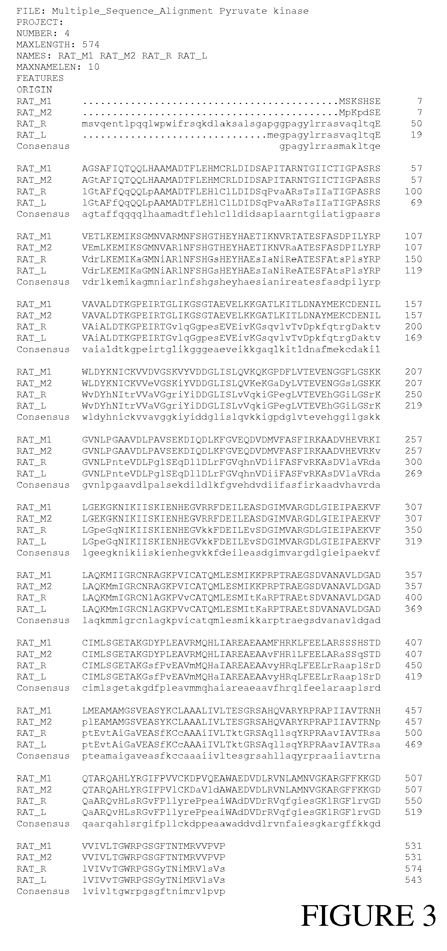

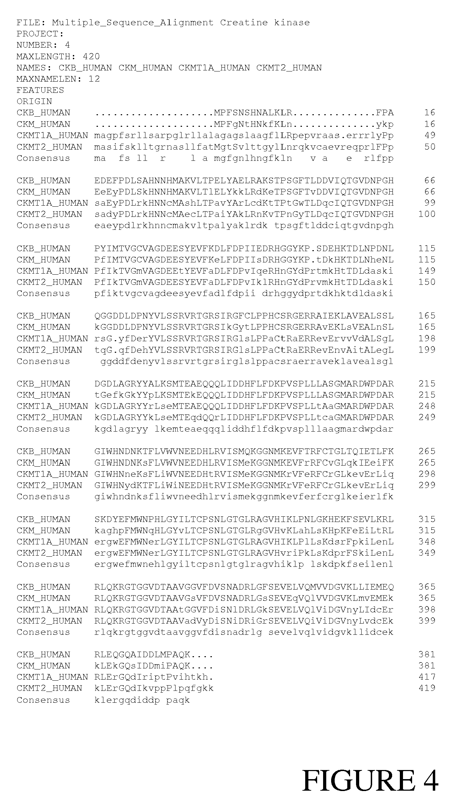

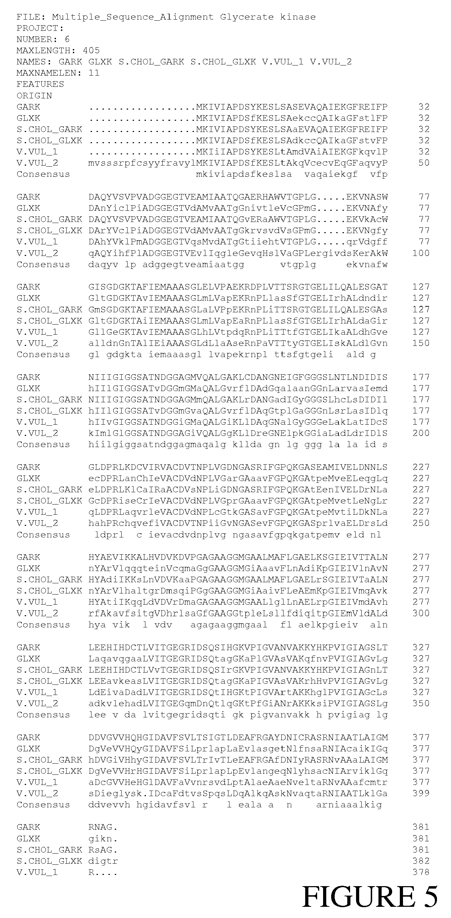

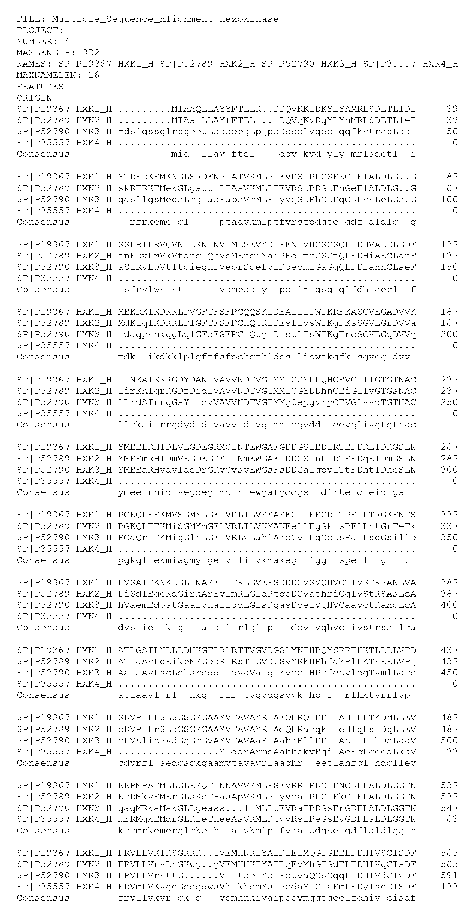

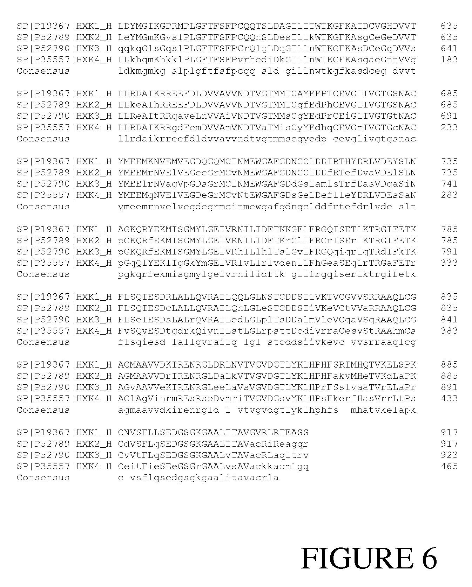

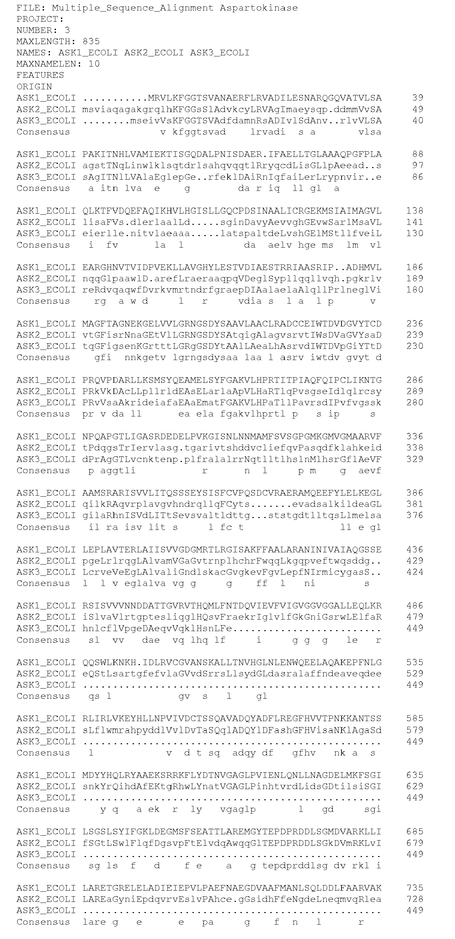

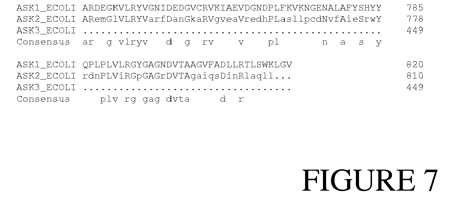

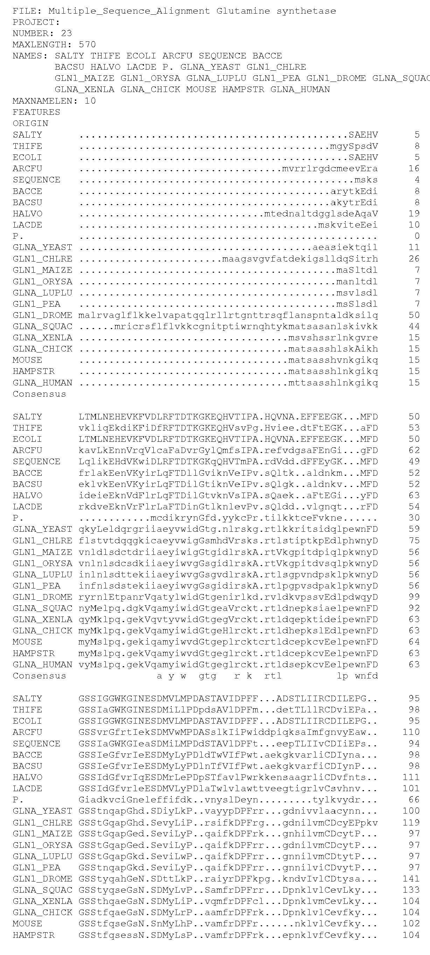

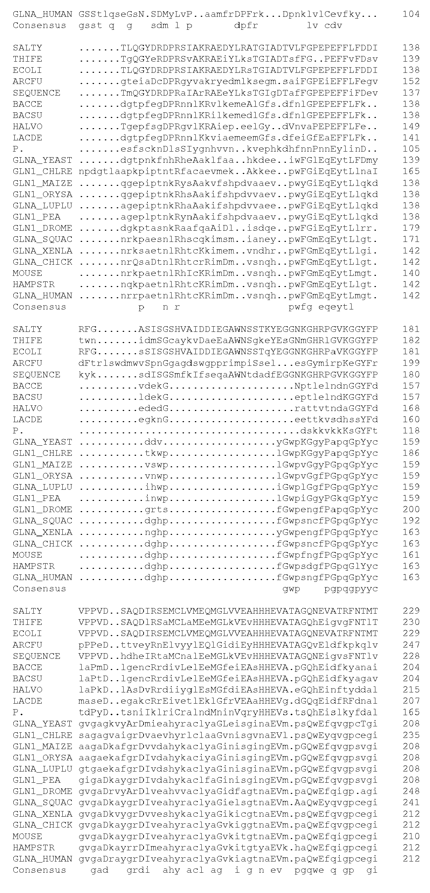

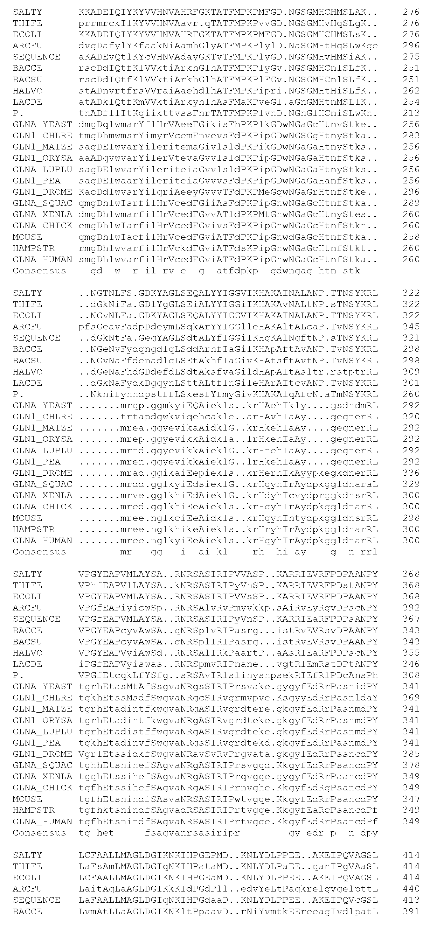

The kinases are a large number of structurally diverse enzymes that play a critical role in numerous metabolic and signalling pathways and whose substrates may be a small molecule, lipid, or protein. The kinases have been classified into 25 families of homogenous proteins, with the families assembled into 12 fold-groups based on the similarity of their structural folds. The enzymes transferring high energy phosphate bonds from nucleotides fall into two divisions, namely, transferases (kinases) and ligases (synthetases). The catalytic and regulatory mechanisms employed in nucleotide binding and phosphoryl transfer, within a single kinase group from both prokaryotic and eukaryotic organisms, as well as the specific kinase isoenzymes, are kinetically and functionally distinct based on the rate of phosphoryl transfer and the regulation thereof. An initial structural comparison of key amino acid residues associated with phosphoryl transfer activity and the regulation of kinase enzymes and their isoenzymes, as depicted in Figures 1 to 8 and in Table 1, demonstrated the level of structural homology of the residues that are associated with phosphoryl transfer activity and/or the regulation of phosphoryl transfer activity, and whether functionality may be differentiated based on the conservation of key amino acids in the active sites of these kinases and the role of the conserved amino acid residues in phosphoryl transfer. The sequence and structural analysis of a range of kinase and synthetase enzymes was carried out using the Accelrys INSIGHTII and Discovery Studio 2.5 suite of molecular modelling software - see Figures 1 to 9. The protein sequence alignments of each enzymes isoforms were carried using the DNAMAN sequence alignment software using the dynamic alignment with a gap penalty of 10, a gap extension penalty of 5 and the BLOSUM protein weight.

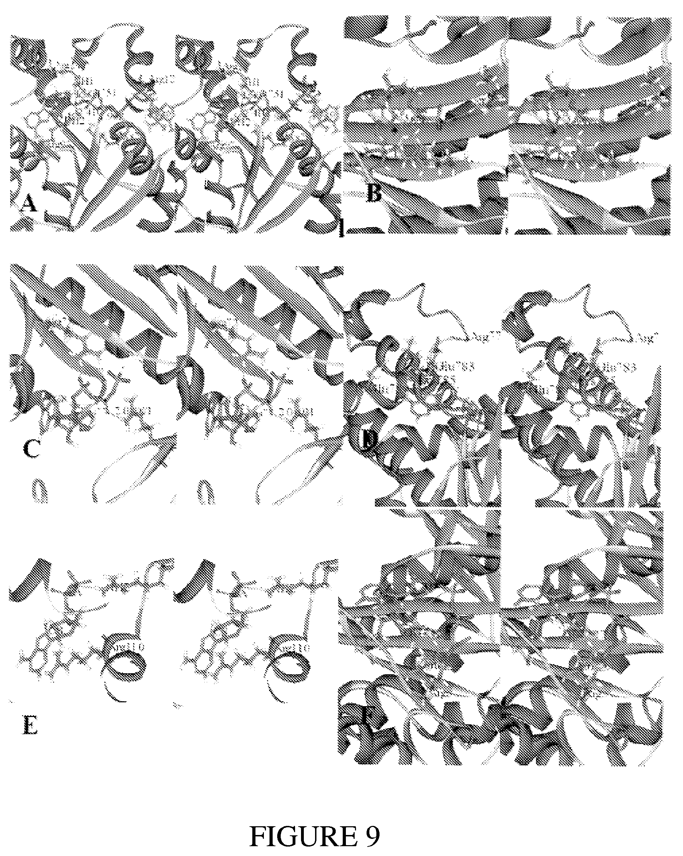

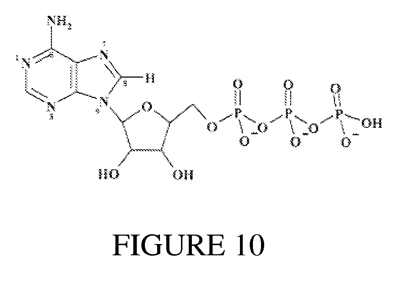

Based on the structural analysis of the nucleotide binding site of a range of diverse kinase enzymes, as depicted in Figure 9, the role of key amino acids involved in nucleotide binding was defined. The protein sequence alignments for each enzyme isoform and structural investigation of the active sites of adenylate kinase (AK), shikimate kinase (SK), pyruvate kinase (PK), hexokinase (HXK), aspartokinase (ASK) and creatine kinase (CK) indicated conserved paired Arg and Glu/Asp in the active site associated with the binding of the imidazole moiety and the $\alpha$-PO$_4$ of the nucleotide (Table 1 and Figure 9) (Sequence alignments: Figures 1-8, Structural comparison of conserved residues: Figure 9 and Table 1). The AK group of enzymes isoforms served as a template for the identification of key amino acid residues that could be required for the binding and/or regulation of phosphoryl transfer within the active site of kinases. The mechanism of binding and the role of the imidazole moiety of the nucleotide in the binding and its role in the regulation of catalysis was defined. Within the active site of AK, SK, PK, HXK, ASK and CK, Arg and Glu/Asp residues associated with the binding ATP to the active site of the enzymes (Table 1), are conserved. The NH2 of the Arg97 is within a requisite 3.5 A of the C-8 proton (ATP atom numbering: Figure 10) of the ATP analogue. As the active sites of AK1 and AK4 were not distorted in the crystal structure, these sites were used to identify the imidazole binding domain within the active site. AK1 contains "diadenyl tetraphosphate" in the active site while AK4 contains "diadenyl pentaphosphate" in the active site. Hydrogen bonding occurs between conserved Arg and Glu or Asp acid residues and the imidazole moiety of the nucleotide. AK1 has the Arg 97 and Glu 98 and the pair Arg 128 and Asp 180 associated with the equivalent N7/C8 atoms of the diadenyl tetraphosphate. The equivalent residues in AK2-AK6 are outlined in Table 1. In CK, the Arg/Glu pair hydrogen bonded to the imidazole moiety of the nucleotide ATP are Arg 292 and Asp 335. Outlined in Table 1 and Figure 9 are similar interactions between the C8-H of the imidazole moiety of the nucleotide and the conserved Arg residue within the active sites of AK, SK, PK, HXK, ASK, CK and GS isoenzymes. In all enzymes investigated, other than HXK I-III, a conserved Arg was identified within approximately 3.5 A of the C8-H of the imidazole moiety of the associated nucleotide. In HXK I-III, the Arg is replaced by a Lys whereas in HXK IV the Arg is conserved. The identified Arg residue was also found to be H-bonding distance of the $\alpha$-PO$_4$ of the nucleotide. It is proposed the concomitant association of the Arg with the C8-H of the imidazole moiety and the $\alpha$-PO$_4$ of the nucleotide has mechanistic implications in the regulation of the phosphoryl transfer activity of the enzyme. In all cases additional arginine residue/s are associated with the primary arginine residue which is associated with the imidazole moiety of the nucleotide. The second arginine residue is also associated with the phosphate backbone of the nucleotide. The arginine residues may be replaced by either glutamine or lysine.

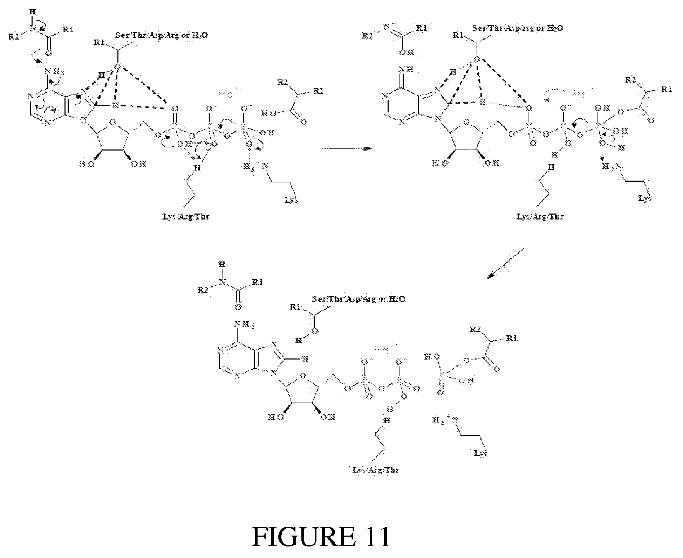

It was therefore proposed that the C-8 proton of ATP plays a direct role in the binding of ATP to the active site as well as in the catalysis of phosphorylation by general acid-base catalysis as outlined in Figure 11. The role of the Arg97 is to act as a general base catalyst in the abstraction of the C-8 proton from ATP which in turn, via resonance stabilization, acts to protonate the $\alpha$-phosphate of the ATP. The second arginine in the active site, Arg 44, then acts to transfer the proton to the $\beta$-phosphate. This acid-catalysed stabilization of the $\alpha$- and $\beta$-phosphates of the ATP acts to withdraw electrons into the "backbone" of the, $\alpha$- $\beta$-$\gamma$-phosphate chain of ATP, allowing for resonance stabilization of the phosphate backbone and facilitating that the $\gamma$-phosphate acts as a better leaving group in the phosphorylation of the substrate molecules.

EP 2 516 668 B1

TABLE 1

| PK-R | PK-L | PK-M1/M2[a] | AK1[a] (Cyto) | AK5 (Cyto) | AK6 (Cyto) | AK2 (Mito) | AK3 (Mito) | AK4 (Mito) | M-CK (Cyto) | uMt-CK (Mito) | sMt-CK (Mito)[a] |
|---|---|---|---|---|---|---|---|---|---|---|---|
| His121 | His89 | **His77** | **Arg97** | Arg100 | Arg ? | Arg103 | Arg94 | Arg92 | Arg292 | Arg325 | Arg326 |
| His $ND_1$ to ATP-C8H = 4.57 A | | | Arg $NH_1$ to ATP-$\alpha$-$PO_4$ = 2.41 A | | | | | | Arg $NH_2$ to ATP-C8H = 2.58 A | | |
| His NE2 to ATP-$\alpha$-$PO_4$ = 1.92 A | | | Arg $NH_2$ to ATP-C8H = 3.46 A | | | | | | Arg $NH_2$ to ATP-$\alpha$-$PO_4$ = 3.58 A | | |
| His NE2 to Arg120 NH1 = 1.92 A | | | | | | | | | Arg HE to ATP-$\beta$-$PO_4$ = 1.67 A | | |
| Arg116 | Arg84 | **Arg72** | Glu98 | Glu101 | Asp77? | Glu109 | Glu100 | Glu98? | Arg236 | Arg269 | **Arg270** |
| Arg $NH_1$ to ATP-C8H = 4.60 A | | | | | | | | | Arg $NH_1$ to ATP-$\alpha$-$PO_4$ = 3.15 A | | |
| Arg $NH_1$ to ATP-$\alpha$-$PO_4$ = 1.92 A | | | **Arg44** | Arg47 | Arg39 | Arg51 | Arg43 | Arg41 | Arg320 | Arg352 | **Arg354** |
| | | | Arg $NH_1$ to ATP-$\alpha$-$PO_4$ = 1.90 A | | | | | | Arg $NH_1$ to ATP-$\alpha$-$PO_4$ = 1.92 A | | |
| | | | Arg $NH_1$ to ATP-$\alpha$-$PO_4$ = 3.75 A | | | | | | Asp335 | Asp368 | **Asp369** |
| Arg163 | Arg132 | **Arg120** | **Arg 128** | Arg131 | Arg105 | Arg138 | Arg124 | Arg122 | Asp $\delta$-$OD_1$ to Arg326 $NH_1$ = 1.85 A | | |
| Arg $NH_1$ to ATP-$\beta$-$PO_4$ = 2.05 A | | | Arg $NH_1$ to ATP-C8H = 6.40 A | | | | | | | | |
| | | | Asp180 | Asp184 | Asp157 | Glu? | Glu200 | Glu198 | | | |
| **SK1** [a,b] | | **SK2**[b] | **HK1/HK2** [a] | HK3 | **HK4** | **ASK1** | **ASK2** | **ASK3** | **GS** | | |
| Arg113 | | Arg NE | **Lys785**[c] | Lys791 | Arg333 | Arg238 | Arg241 | **Arg232** | Arg 355 | | |
| Arg HE to Glu108 $\delta$-$OD_1$ = 2.39 A | | | | | | Arg $NH_1$ to ATP-C8H = 4.33A | | | Arg $NH_2$ to ATP-C8H = 3.95 A | | |
| Arg $NH_1$ to ATP-C8H = 4.00 A | | | | | | | | | Arg $NH_2$ to His271 ND1 = 3.33 A | | |
| **Glu108** | | Glu NE | Gln789 | Gln NE | Gln337 | Arg306 | Arg309 | **Arg300** | | | |
| Arg120 | | Arg117 | **Arg30**[d] | Lys41 | NE | | | | Glu 357 | | |
| Arg $NH_1$ to ATP-$\alpha$-$PO_4$ = 2.75 A | | | Arg $NH_2$ to ATP-C8H = 3.44 A | | | Asp228 | Asp231 | **Asp222** | | | |
| Glu? | | | | | NE | | | | | | |
| | | | Asp $OD_1$ to Arg30 $NH_1$ = 3.25 A | | | | | | | | |

Table 1: Identified Arg and Glu/Asp residues responsible for the regulation of phosphoryl transfer in a range of kinase isoenzymes and a synthetase enzyme. The rows contain the conserved amino acid residues from various isoenzymes of each enzyme. Where structures contained a co-crystallized nucleotide the inter-atomic distances between the C8-H of the imidazole moiety, $\alpha$-$PO_4$ of the nucleotide and the amino acid were measured (Atoms and inter-atomic distances outlined below each residue). Within each isoenzyme the Glu/Asp residues associated with each Arg residue follow the Arg residue in the column of the table. In PK, AK and CK the Arg is also in H-bonding distance of the C8-H of ATP as well the $\alpha$-$PO_4$ of ATP. In SK1 a second Arg residue is responsible for the interaction with C8-H of ATP as well the $\alpha$-$PO_4$ of ATP.

The PDB accession codes for protein structures used, pyruvate kinase (PK); 1A49, adenylate kinase (AK1); 2C95, creatine kinase (CK); 2GL6, Shikimate kinase (SK1), 1 L4U, hexokinase (HXK1) catalytic site; 1DGK, hexokinase (HXK1) allosteric regulation site; 1 QHA, glutamine synthetase (GS): 1 F52.
[a] Isoenzyme from which the associated inter-atomic distance data was obtained.
[b] Residue number taken from *E. coli* sequence however Arg/Glu/Asp identification in structure taken from *Mycobacteria tuberculosis* structure as ADP is co-crystallized into the active site of this structure.
[c] Catalytic subunit active site residue.
[d] Allosteric regulation subunit binding site residue.
NE = No equivalent residue in this isoenzyme

## EXAMPLE 2: Determination of the role of key amino in AK1 using Site-Directed Mutagenesis

The role of key amino acids involved in the nucleotide binding was demonstrated by site-directed mutagenesis of AK1 and enzymatic analysis of the mutated enzymes demonstrating the necessity of these amino acids in enzyme activity and nucleotide binding.

Construct AK1A-c001, encoding a N-terminal His-tagged human adenylate kinase 1 gene, was obtained from the Structural Genomics Consortium, University of Oxford, United Kingdom. Site-directed mutagenesis was carried out using Finnzymes' Phusion Site-Directed Mutagenesis kit. The mutations created were: Arg97 and Arg128 to Ala, Gin and Lys, and Glu98 and Asp180 to Leu. All resulting constructs were sequenced to confirm mutations. Wild-type and mutated versions of His-tagged AK1 were expressed in *E. coli* Origami(DE3) (Novagen), and purified using Bio-Rad's Profinia Purification System. Purified proteins were dialysed against 50 mM $KH_2PO_4$/$K_2HPO_4$ buffer (pH 6.8) containing 1.5 mM $MgCl_2$ and 120 mM KCl.

The effect of the SDM on the specific activity of AK1 was determined in assays containing 50 mM $KH_2PO_4$/$K_2HPO_4$, 0.6 mM ADP, 0.6 mM ATP and 0.66 mM $MgCl_2$ at 37°C at pH 6.9. The reaction was stopped by the addition of trichloroacetic acid to a final pH of 2 to 3. The assay solutions were centrifuged prior to HPLC analysis. The assays for adenosine, AMP, ADP and ATP were carried out using a Phenomenex $5\mu$ LUNA $C_{18}$ column with the mobile phase containing PIC A® (Waters Corporation), 250 ml acetonitrile and 0.7% (w/v) $KH_2PO_4$. The flow rate of the mobile phase was 1 ml/min with UV detection.

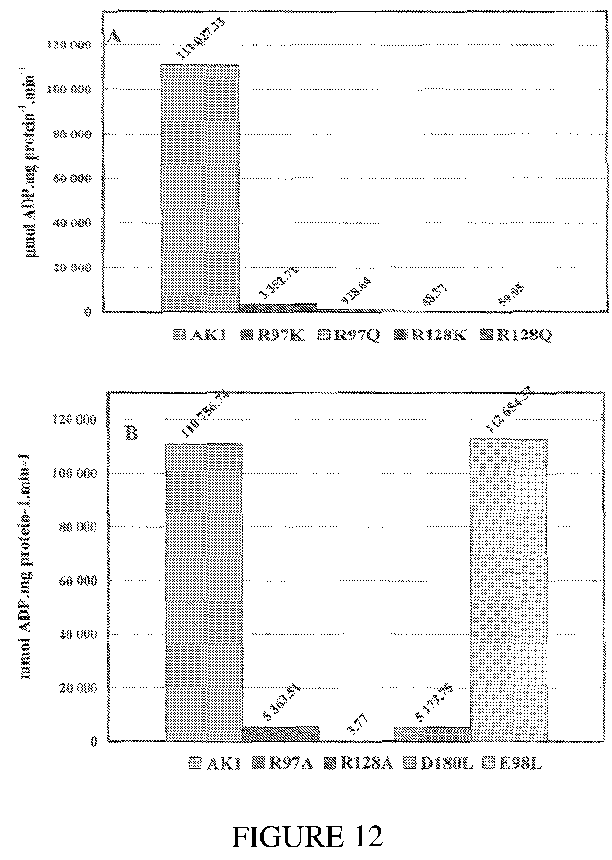

The effect of the following SDM; R97K, R97Q, R97A, R128K, R128Q, R128A, E98L and D180L on the specific activity of AK1 was determined - see Figures 12 A and B. In the case of both Arg97 and Arg128 mutations to Lys or Gin gave a significant decrease in the specific activity of the enzyme with the effect being 32 fold for R97K, 120 fold for R97Q, and 2200 fold in the case of R128K and R128Q. The R97A mutation gave a 100 fold reduction in activity while the R128A enzyme did not give detectable activity. The D180L mutation gave a 20 fold reduction in activity while the E98L mutation had no effect on the specific activity. The effect of the mutations on the Arg128/D180 pair was greater than the Arg97/Glu98 pair.

## EXAMPLE 3: Effect of pH on the inhibition of AK1 by imidazole.HCl, histidine.HCl and 1,2 dimethyl imidazole.HCl

The effect of the enzyme assay pH and the presence of imidazole.HCl, histidine.HCl and 1,2 dimethyl imidazole.HCl on the activity of AK1 was determined. The assays contained 50 mM $KH_2PO_4$/$K_2HPO_4$ (at the equivalent pH), 0.6 mM ADP, 0.6 mM ATP, 0.66 mM $MgCl_2$ and either imidazole, histidine. or 1,2 dimethyl imidazole at a concentration of 2 mM.

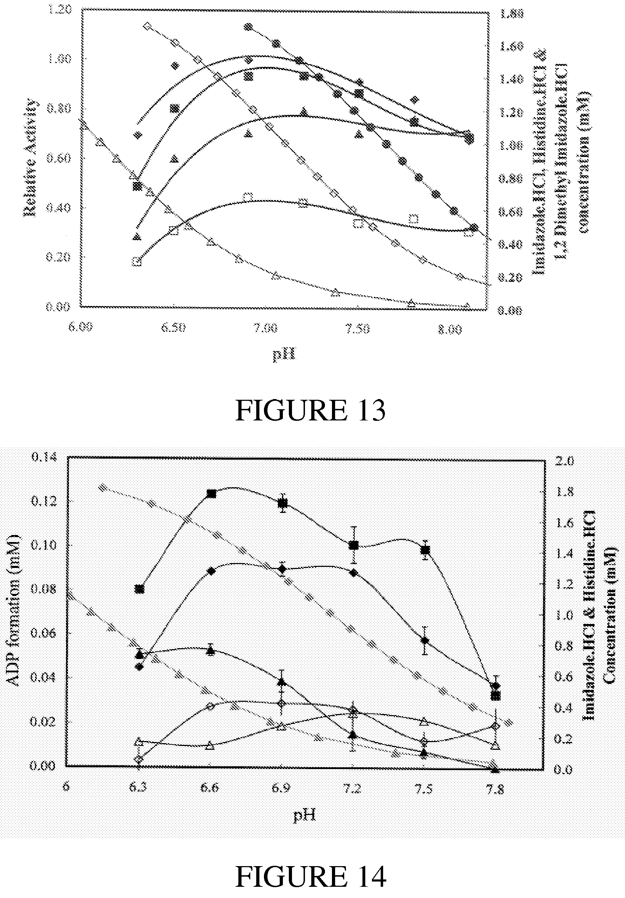

The effect of imidazole.HCl, histidine.HCl and 1,2 dimethyl imidazole.HCl concentration on the activity of AK1 was determined and expressed as a relative activity, relative to the enzyme activity obtained in the presence of NaCl - see Figure 13. The pH optimum for AK1 is of the order of pH 6.9. As each imidazole compound gave distinctive inhibition of the enzymes, the inhibition of AK1 appears to be related to the protonation of the imidazole moiety, with AK1 being inhibited by the deprotonated form of the imidazole - Figure 13. As the concentration of the protonated imidazole in solution increases with decreasing pH there is a concomitant increase in the activity of the enzyme. At a pH above pH 6.9 the deprotonated imidazole acts as an inhibitor of AK1.

**EXAMPLE 4: Effect of pH on the inhibition of AK1 by imidazole.HCl and histidine.HCl and deuterated imidazole.HCl and histidine.HCl.**

The enzyme activity of AK1 was determined over a pH range of pH 6.3 to pH 8.1 in the presence of 2mM NaCl, imidazole.HCl and histidine.HCl and compared under the same conditions in the presence of deuterated imidazole.HCl and histidine.HCl - see Figure 14. The imidazole.HCl and histidine.HCl was deuterated at position 5 which is equivalent to position C8 in the nucleotides. As outlined in Example 3, both imidazole.HCl and histidine.HCl inhibited the activity of AK1 when compared with the activity in the presence of NaCl. In both the case of imidazole.HCl and histidine.HCl there was a further reduction in the activity in excess of 50% of both adenylate kinases in the presence of deuterated imidazole.HCl and histidine.HCl.

Kinase inhibitors labelled at the carbon equivalent to C8 would therefore be at least double as inhibitory as unlabelled inhibitors.

**EXAMPLE 5: Effect of Deuterated AMP and ATP on the activity of AK1.**

The enzyme activity of AK1 was determined using undeuterated nucleotides and compared with the activity in the presence of deuterated AMP, deuterated ATP and deuterated AMP and ATP. The assays contained 50 mM $KH_2PO_4/K_2HPO_4$ (at the equivalent pH), 0.6 mM ADP, 0.6 mM ATP and 0.66 mM $MgCl_2$.

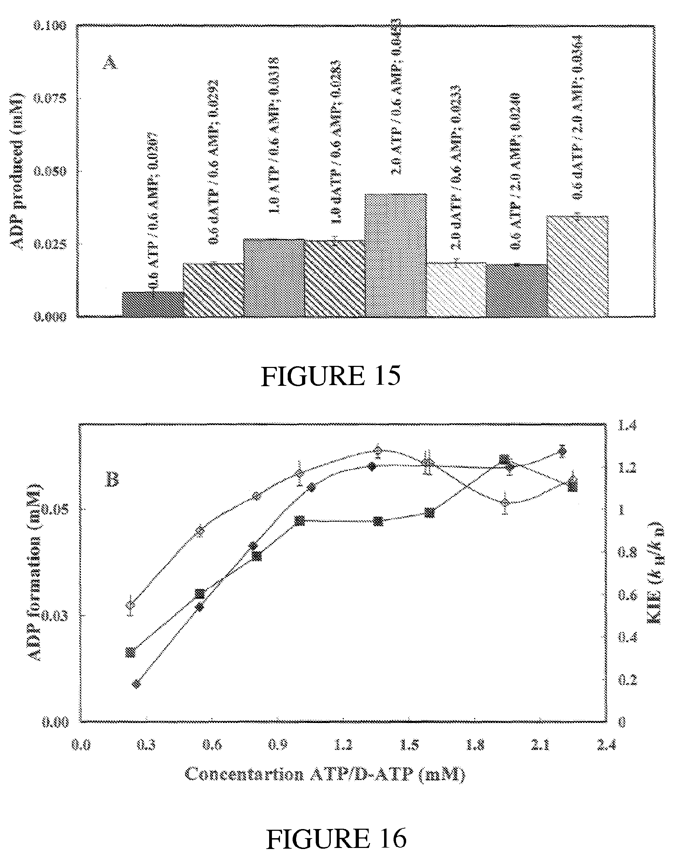

The enzyme activity of AK1 was determined using undeuterated nucleotides and compared with the activity in the presence of deuterated ATP and undeuterated ATP (Figures 15 and 16). At low ATP concentrations the presence of undeuterated ATP gave lower activity than when deuterated ATP was used. At high ATP concentrations there is at least a 50% reduction in the activity as is the case in the presence deuterated ATP when compared with deuterated ATP.

**EXAMPLE 6: The effect of Adenosine 5'-[γ-thio]triphosphate (ATPS) and deuterated ATPS on the activity of AK1**

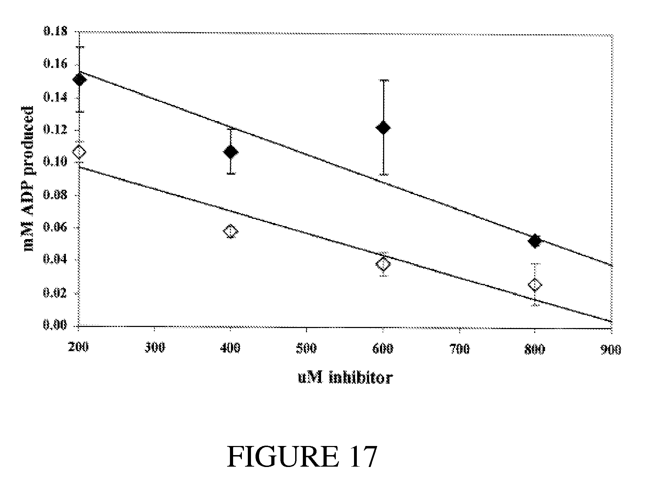

The effect of ATPS and deuterated ATPS (C8-D ATPS) on the activity of AK1 was determined. Assays contained 1 nM human AK1, 0.66 mM $MgCl_2$, 0.6 mM ATP and 0.6 mM AMP in 50 mM potassium phosphate buffer, pH 7.2. Increasing concentrations of ATPS or C8-D ATPS were added; 0.2, 0.4, 0.6, 0.8, and 1.0 mM. Assays, in a final volume of 1 ml, were allowed to proceed for 45 mins at 37°C, before 6 μl 100% TCA was added to stop the reaction. Both ATPS and C8-D ATPS were found to inhibit AK1; however the C8-D ATPS gave significantly more inhibition than the ATPS- see Figure 17. The assay where no ATPS or C8-D ATPS was added produced 0.19 mM ADP.

**EXAMPLE 7**

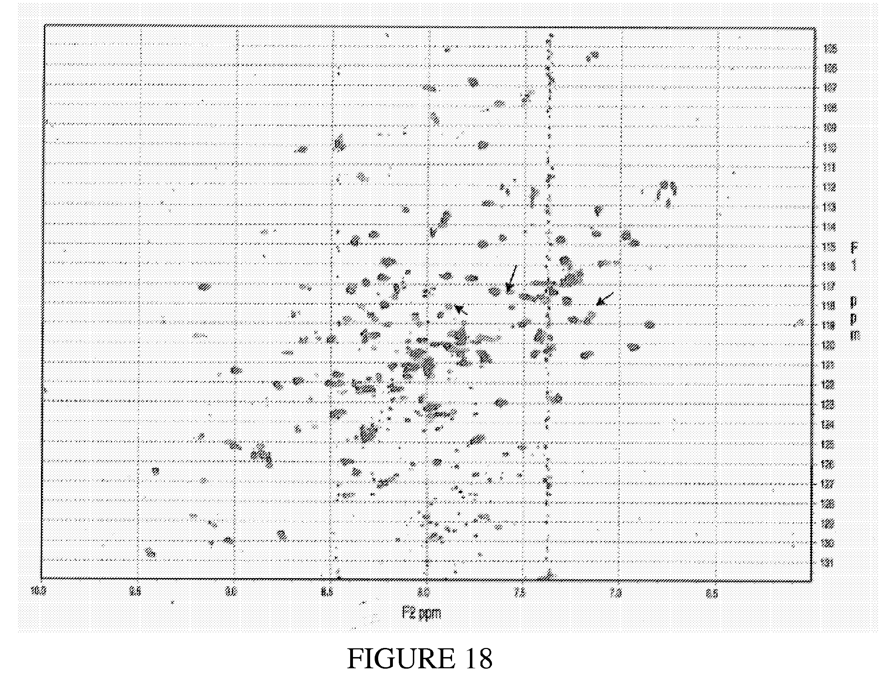

AK1 was labelled by culturing the *E. coli* expression cells in the presence of $^{15}$N $NH_4Cl$ and purified. A Heteronuclear Single Quantum Coherence (HSQC) $^1$H and $^{15}$N NMR spectrum was obtained of AK1 in the presence of ATPS and C8-D ATPS at a concentration of ≅ 200 μM AK1 and 100mM of ATPS or C8-D ATPS. From the HSQC spectrum it is clearly evident that a number of the shifts in the active site of AK1 change their relative position as a result of the binding of the nucleotide in either the ATPS or C8-D ATPS forms - see Figure 18).

A non-classical kinetic isotope effect is thus found in the enzyme kinetics of AK1 at low ATP concentrations, with a 50% reduction in enzyme activity, when the reactions are carried out using ATP when compared with ATP deuterated at position C8. A 50% reduction in enzyme activity is also obtained in the case where the kinase and synthase enzymes perform reversible reactions employing ADP or AMP.

All nucleotides bind into the active site of adenylate kinase enzymes by this mechanism which forms part of catalysis by inducing of C8-H to become more acidic.

A number of "imidazole" moiety-containing compounds inhibit adenylate kinase enzymes. A further reduction in enzyme activity by at least 50% is obtained when the "imidazole" moiety-containing compounds are deuterated at the position equivalent to C8 in AMP/ADP/ATP.

In accordance with the invention, a coupling mechanism is therefore provided whereby nucleotides bind to the active site of enzymes at the imidazole moiety by the inter-conversion of the C8-H. This coupling mechanism also plays a role in imidazole inhibition of adenylate kinases.

"Imidazole" moiety-containing compounds mimic the imidazole moiety of nucleotides and bond transiently but covalently to the active site of adenylate kinase enzymes in the same manner as nucleotides do.

**Example 8: The effect of the ATP and C8D-ATP concentration on the specific activity of *Saccharomyces cerevisiae* hexokinase, *Escherichia coli* acetate kinase, *Escherichia coli* phosphofructokinase, *Escherichia coli* deadenylylated glutamine synthetase, *Escherichia coli* adenylylated glutamine synthetase and *Mycobacterium tuberculosis* shikimate kinase**

Based on the studies carried out on AK1 as presented in Examples 1 to 7, further comparative assays were run to determine the effect of ATP and C8D-ATP on the specific activity of a range of kinase enzymes. The kinase enzymes investigated were hexokinase, acetate kinase, shikimate kinase, phosphofructokinase, glutamine synthetase [(GS$_{12}$) and (GSo)].

**Acetate kinase** (EC **2.7.2.1**) is a homodimer which catalyses the Mg$^{2+}$-dependent, reversible transfer of phosphate from ATP to acetate according to the following reaction:

$$CH_3COO^- + ATP \leftrightarrows CH_3CO_2PO_3^{2-} + ADP$$

Acetate kinase forms part of the acetate and sugar kinase/Hsc70/actin (ASKHA) structural superfamily (PFam Clan: *Actin_ATPase*:CL0108). The enzyme is a homodimer, and monomer interaction plays a role in the regulation of the enzyme activity and ligand binding with the enzyme active sites functioning in a coordinated half-the-sites manner. The acting ATPase clan contains both the acetate kinases and sugar kinases, and are all known to undergo a catalytically essential domain closure upon ligand binding.

**Hexokinase (ATP: D-hexose 6-phosphotransferase, EC 2.7.1.1)** catalyses the Mg$^{2+}$-dependent phosphorylation of glucose, from ATP:

$$C_6H_{12}O_6 + ATP \leftrightarrows C_6H_{12}O_6 \, PO_3^{2-} + ADP$$

The two isoenzymes of yeast hexokinase, designated P-I and P-II, are dimers of subunit molecular mass 52 kDa. Hexokinase also forms part of the acetate and sugar structural superfamily (PFam Clan: *Actin_ATPase*:CL0108). Yeast hexokinase enzymes are structurally well characterised with each subunit of the homodimer comprising two domains and in the open conformation these domains are separated by a cleft containing the sugar binding site. Binding of glucose induces a large conformational change in which the two lobes of the subunit rotate relative to each. The enzymes also exist in a monomer-dimer association-dissociation equilibrium that is influenced by pH, ionic strength and substrates. There are major differences in the glucose binding behaviour of both forms where binding to dimeric P-I shows strong positive cooperativity, whereas in P-II the two sites are equivalent and binding is non-cooperative.

**Shikimate kinase.** The shikimate pathway is a seven-step biosynthetic route that links the metabolism of carbohydrates to the synthesis of aromatic amino acids via the conversion of erythrose-4-phosphate to chorismic acid. Chorismic acid is an essential intermediate for the synthesis of aromatic compounds, such as aromatic amino acids, p-aminobenzoic acid, folate, and ubiquinone. Shikimate kinase (SK, EC 2.7.1.71), the fifth enzyme in the shikimate biosynthetic, catalyzes phosphate transfer from ATP to the carbon-3-hydroxyl group of shikimate, forming shikimate 3-phosphate. SK belongs to the nucleoside monophosphate (NMP) kinase structural family, with characteristic features of the NMP kinases being that they undergo large conformational changes during catalysis and belong to the P-loop containing nucleoside triphospahte hydrolase superfamily (Pfam Clan: *AAA*:CL0023). The NMP kinases are composed of three domains: the CORE, which contains a highly conserved phosphate-binding loop (P-loop); the LID domain, which undergoes substantial conformational changes upon substrate binding, and the NMP-binding domain, which is responsible for the recognition and binding of a specific substrate. MgADP induces concerted hinged movements of the shikimate binding and LID domains causing the two domains to move towards each other in the presence of this ligand. The SK crystal structures show that SK exists as a monomer with a single ATP binding site.

**Phosphofructokinase (PFK, fructose-6-phosphate 1-kinase)** (EC 2.7.1.11) is a classical allosteric enzyme that catalyzes the phosphorylation of D-fructose 6-phosphate (Fru-6-P) by Mg-ATP to form D-fructose 1,6-bisphosphate and MgADP. PFK from *B. stearothermophilus* is a homo-tetramer in which each subunit has a molecular weight of 34 000, and which undergoes a concerted two-state allosteric transition. PFK belongs to the PFK-like superfamily (Pfam Clan: *PFK*:CL0240) The enzyme from Bacillus stearothermophilus (Bs-PFK) shows hyperbolic Michaelis-Menten kinetics with respect to both Fru-6-P and Mg-ATP, but cooperative kinetics in the presence of allosteric inhibitor phosphoenolpyruvate(PEP). Unliganded Bs-PFK is in the active R state, which has high affinity for substrate, switching to the inactive T state with low affinity for substrate only in the presence of PEP.

**Glutamine synthetase.** Glutamine synthetase (GS) (EC 6.3.1.2) catalyzes the reversible conversion of L-glutamic acid, ATP and ammonia to L-glutamine, ADP and inorganic phosphate via a γ-glutamyl phosphate intermediate. As GS is a central enzyme in nitrogen metabolism the enzyme is regulated by at least four different mechanisms: (a) adenylylation and deadenylylation of the tyrosine 397 residue, (b) conversion between a relaxed (inactive) and taut (active) state depending on the divalent metal cation present, (c) cumulative feedback inhibition by multiple end products of glutamine metabolism, and (d) repression and derepression of GS biosynthesis in response to nitrogen availability. *Escherichia coli* GS is a large, metalloenzyme (~624 kDa) comprising 12 identical subunits arranged in two face-to-face hexagonal rings. *E. coli* GS belongs to the glutamine synthetase 1-$\beta$ group of enzymes that are regulated via adenylylation of a single tyrosine residue, with each subunit requiring two structurally implicated divalent cations (either $Mg^{2+}$ or $Mn^{2+}$) for its catalytic activity. The extent of adenylylation of the *E. coli* GS in response to an excess or deficiency of nitrogen in the growth environment is regulated in response to the intracellular concentrations of 2-ketoglutarate and glutamine, via the reversible adenylylation of a tyrosine residue (Tyr397) in each subunit of GS. The presence of adenylylated GS ($GS_{12}$) predominates in a nitrogen-rich, carbon-limited media, while the deadenylylated form ($GS_0$) tends to predominate under conditions of nitrogen limitation. The regulation of the adenylylation state of GS is accomplished by three proteins, uridylyltransferase/uridylyl-removing enzyme, the signal transduction protein $P_{II}$, and adenylyltransferase. High intracellular concentrations of glutamine activate the uridylyl-removing enzyme which causes the deuridylylation of $P_{II}$. This interacts with the adenylyltransferase which then catalyses the adenylylation of the GS. High intracellular concentrations of 2-ketoglutarate activate uridylyltransferase, which transfers UMP to each subunit of $P_{II}$, forming $P_{II}$-UMP. The $P_{II}$-UMP interacts with the adenylyltransferase, which in turn catalyses the removal of AMP from the GS.

**Materials and Methods**

*Enzyme source, and protein expression and purification*

Hexokinase from Saccharomyces cerevisiae Type F-300 (Sigma, H4502) and acetate kinase from *E.coli* (Sigma, A7437) were purchased. The human adenylate kinase (AK1) gene in vector pLIG-SC1 was obtained from the Structural Genomics Consortium (code AK1 A). The His-tagged AK1 was produced in *Escherichia coli* Origami (DE3) and purified using the Bio-Rad Profinia Purification System. The pure protein was dialysed against 50 mM $KH_2PO_4/K_2HPO_4$ buffer (pH 6.8), 1.5 mM $MgCl_2$, 120 mM KCl. The *Mycobacterium tuberculosis* shikimate kinase gene in pET15b (Novagen) was obtained from the group of Chris Abell, Cambridge University, UK. The His-tagged MtSK was produced in *E. coli* BL21 (DE3) and purified using the Bio-Rad Profinia Purification System. The pure protein was dialysed against 50 mM Tris (pH 7.5) and 1,000 mM NaCl. Adenylylated ($GS_{12}$) and deadenylylated ($GS_0$) glutamine synthetase were prepared as outlined below.

*Production of glnD and glnE Knockout Strains*

Knockout strains for the production of fully adenylylated (*glnD* knockout) or fully deadenylylated GS (*glnE* kockout) were made from the *E.coli* YMC11 using the Quick & Easy *E.coli* Gene Deletion Kit (Gene Bridges GmbH), designed to knockout or alter genes on the *E.coli* chromosome. Red/ET recombination allows the exchange of genetic information in a base pair precise and specific manner. An FRT-flanked kanamycin resistance marker cassette is supplied with the kit which can be used to replace a gene on the *E.coli* chromosome. The use of a FRT-flanked resistance cassette for the replacement of the targeted gene allows the subsequent removal of the selection marker by a FLP-recombinase step, involving the transformation of an FLP-expression plasmid into the cells and subsequent expression of an FLP site-specific recombinase. The genes for the recombination proteins are under the control of an inducible promoter and the plasmid carries a temperature sensitive origin of replication for a convenient removal of the plasmid after recombination. In order to produce fully adenylylated GS, it is necessary to knockout the uridylyltransferase, coded by the *gln*D gene. Primers were designed to the *E.coli gln*D gene. These primers contained a region specific to the *gln*D gene adjoining a sequence specific to the FRT cassette (underlined, see below). In a similar fashion, to produce fully deadenylylated GS, the adenylyltrasnferase, coded by the *gln*E gene, needs to be knocked out. Primers were therefore designed to the *E.coli gln*E gene. These primers contained a region specific to the *gln*E gene adjoining a sequence specific to the FRT cassette (underlined, see below). Both knockout strains were produced using the primers as described in the kit protocol. The only deviation from the protocol, was that *Bam*HI restriction sites were incorporated in the ends of the primers (shown in bold). This enabled the PCR product to be cloned into pGEM T-Easy (Promega Corporation), and then cut out of the pGEM construct as a *Bam*HI fragment. This facilitated production of the cassette in sufficient quantity for the transformation step, as it was found to be extremely difficult to produce enough of the cassette by PCR alone. Once integration of the cassette was confirmed by selection on kanamycin plates, a PCR product was produced using primers designed to the sequence of the *gln*D or *gln*E gene, either side of the integration site. This PCR product was then sequenced to confirm integration. The kanamycin resistance marker was removed using the 706-FLP plasmid carrying the site-specific recombinase. The removal of the marker was also confirmed by sequencing, as above. Primers used to create *glnD* and *glnE* knockout strains of *E.coli* YMC11 were:

*glnD* sense primer,

5'-

ga**ggatcc**cagaaccagcgccatcagcgttaccatggcaccagctacaaccttgaacc<u>aattaaccctc</u>
<u>actaaagggcg</u>-3'(SEQ ID NO.56);

*glnD* antisense primer,

5'-

gt**ggatcc**gcgatatcgtgaaacagcgcggcgatgaaaatcagctcagttgacggcag<u>taatacgactc</u>
<u>actatagggctc</u>-3'(SEQ ID NO.57);

*glnE* sense primer,

5'-

ga**ggatcc**tgcgcctgtttgaactgacgcagcgcctcaagctgttgctcttcgtcatc<u>aattaaccctcactaa</u>
<u>agggcg</u>-3'(SEQ ID NO.58);

*glnE* antisense primer,

5'-

gt**ggatcc**aggtgttccagctcattcgcggcggacgcgaaccgtcgctgcaatcgcgc<u>taatacgactcac</u>
<u>tatagggctc</u>-3'(SEQ ID NO.59).

***Purification of E. coli glutamine synthetase***

$GS_{12}$ and $GS_0$ were purified from recombinant *E. coli* YMC11 *glnD* and *glnE* knockout strains. *E. coli* YMC11 *glnD-* strain producing $GS_{12}$ and the *E. coli* YMC11 *glnE-* strain producing $GS_0$. The culturing protocols used were as outlined in supplementary information. The enzyme concentration and purity were determined by Quant -IT™ Protein Assay Kit (Invitrogen, USA) and SDS-PAGE.

***C8-D ATP synthesis.***

The synthesis ATP and ADP deuterated at the C8 position (C8-D ATP and C8-D ADP) was carried out based on the method of (49). A 20 mM solution of $Na_2ATP$ in $D_2O$ containing 60 mM triethylamine (TEA) was incubated at 60°C for 144 hours. The TEA was removed by twice passing the solution over a Dowex 20W ion-exchange resin in the acid form. The pH of the solution was adjusted to pH 12 with NaOH prior to the second pass over the resin. The pH of the solution was adjusted to pH 6.3 prior to freeze drying. The extent of the deuteration of the C8 proton was determined by $^1H$ NMR and mass spectroscopy.

***Steady-State Kinetic Analysis***

**$GS_{12}$, and $GS_0$** assay. The effect of the concentration of ATP and C8D-ATP on the specific activity of $GS_{12}$, and $GS_0$ was determined at concentrations ranging from 150 to 3000 $\mu$M ATP and C8D-ATP in assays containing 4 mM Na-glutamate, 4 mM $NH_4Cl$, 5.4 mM $NaHCO_3$ in 20 mM imidazole buffer. The $GS_0$ assay was carried out at pH 7.4 ($\pm$ pH 0.05), and at $MgCl_2$ concentrations equivalent to 3 times the ATP concentration. The $GS_{12}$ assay was carried out at pH 6.6 ($\pm$ pH 0.05), and at $MnCl_2$ concentrations equivalent to 3 times the ATP concentration. The reaction was stopped by the addition of trichloroacetic acid to give a pH of 2-3. The forward reaction rate was determined by measuring the ADP concentration in solution HPLC.

**Hexokinase assay:** 100mM Phosphate buffer pH 6.8, 10mM D-Glucose, 250mM KCl, $MgCl_2$ and ATP were kept at a 1:1 ratio at concentration between 0.2mM-3mM. The assay was incubated at 37°C for 15 minutes and stopped by the addition of 1$\mu$l of 50% TCA. The formation of ADP was analysed by HPLC.

**Acetatekinase assay:** 100mM Phosphate buffer pH 6.8, 10mM Sodium Acetate, 250mM KCl, MgCl$_2$ and ATP were kept at a 1:1 ratio at concentration between 0.2mM-3mM. The assay was incubated at 30°C for 30 minutes and stopped by the addition of 1μl of 50% TCA. The formation of ADP was analysed by HPLC.

**Phosphofructokinase assay:** 100mM Phosphate buffer pH 6.8, 10mM Fructose-6-Phosphate, 250mM KCl, MgCl$_2$ and ATP were kept at a 1:1 ratio at concentration between 0.2mM-3mM. The assay was incubated at 37°C for 15-30 minutes and stopped by the addition of 1μl of 50% TCA. The formation of ADP was analysed by HPLC.

**Shikimate kinase assay:** Assays comprised 100 mM potassium phosphate buffer (pH 6.8), 500 mM KCl, 10 nM enzyme, and varying amounts of ATP, shikimic acid and MgCl$_2$. These were kept at a constant ratio of 1:1:2 for ATP: MgCl$_2$: shikimic acid. The ATP concentrations ranged between 0.2 and 10 mM. The final volumes were 100 μl, and the reactions were incubated at 37°C for 20 minutes, before being terminated by the addition of 5 μl 200 mM EDTA.

In all assays, the production of ADP was analysed by HPLC. The assay solutions were centrifuged prior to HPLC analysis. The assays for adenosine, AMP, ADP ATP were carried out using Phenomenex 5μ LUNA C$_{18}$ column with the mobile phase containing PIC A$^®$ (Waters Coorporation), 250 ml acetonitrile, 7g KH$_2$PO$_4$ per litre water. The flow rate of the mobile phase was 1 ml/minute with UV detection.

**Results**

ATP was deuterated specifically at position C8 as hereinbefore described and the deuteration was assessed by $^1$H NMR.

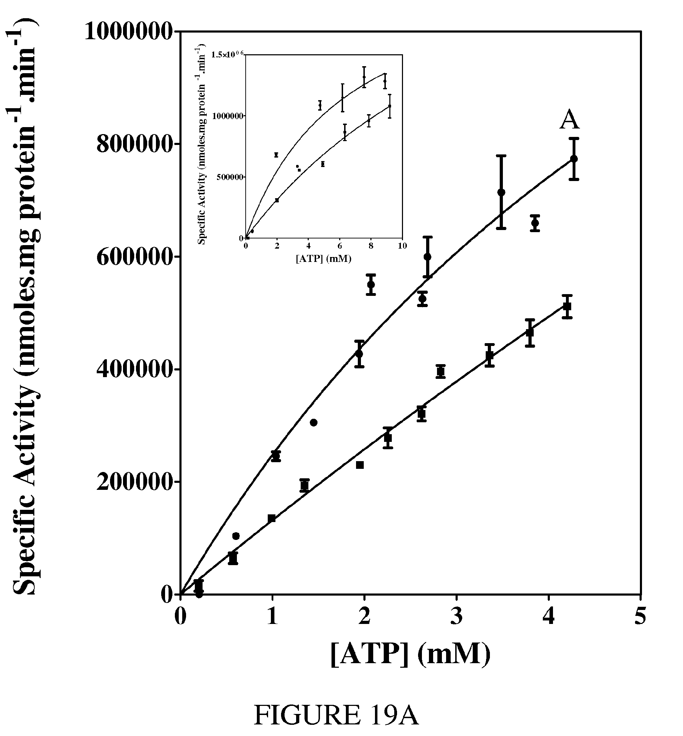

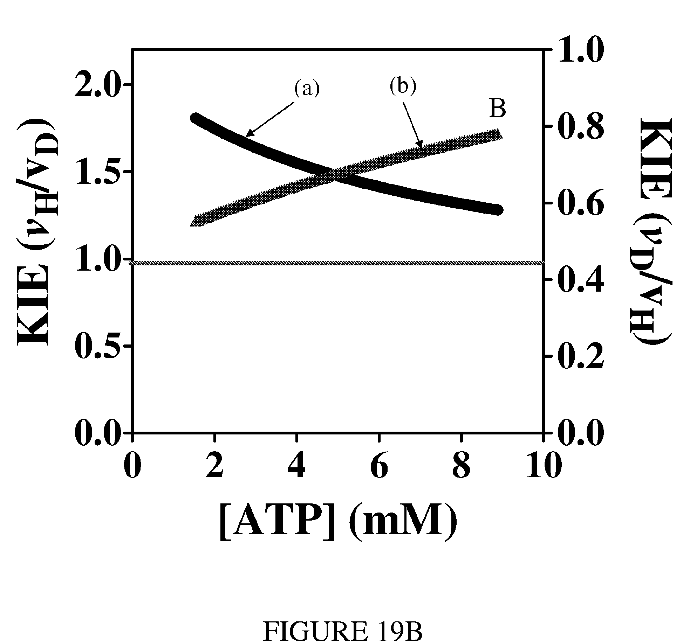

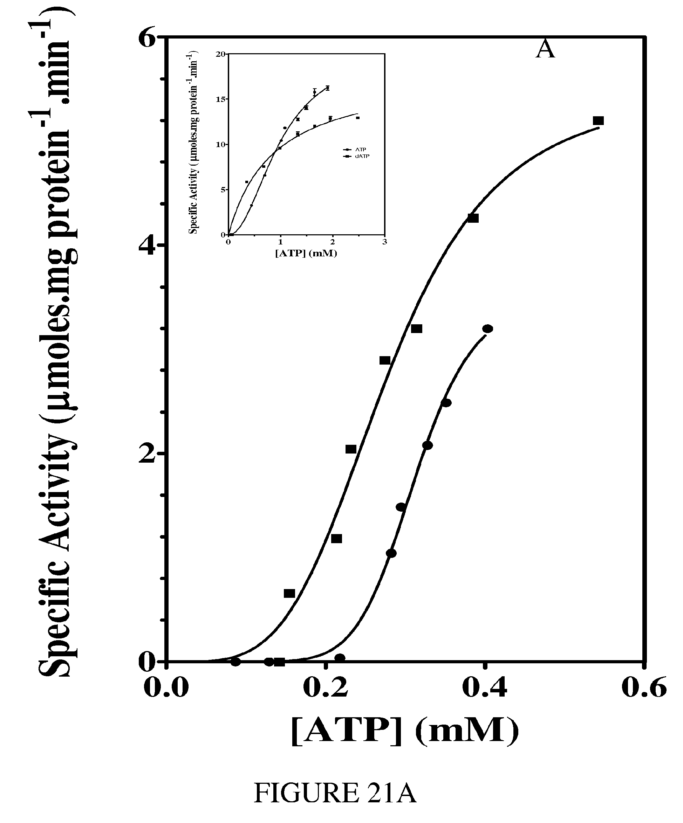

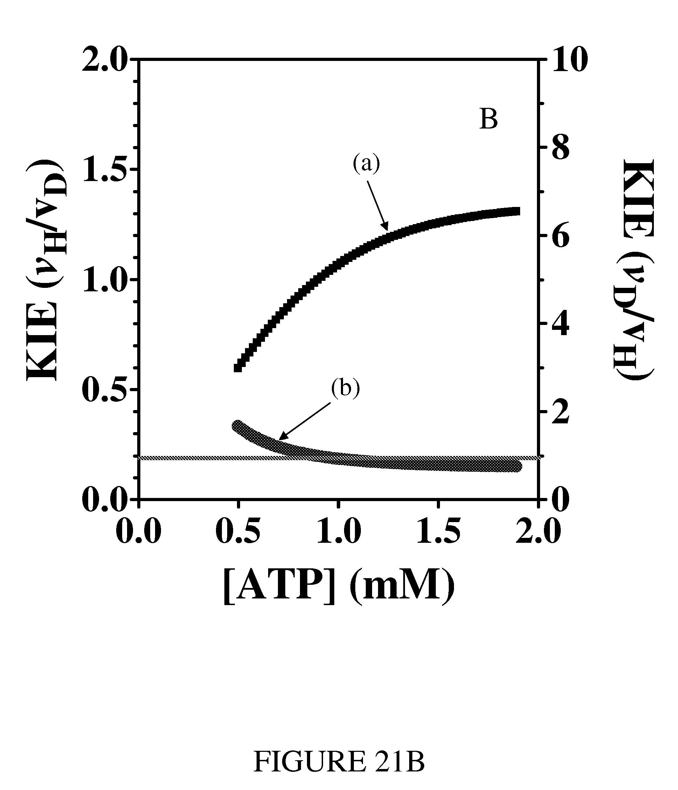

The effect of the ATP and C8D-ATP concentration on the specific activity of *Saccharomyces cerevisiae* hexokinase, *Escherichia coli* acetate kinase, *Escherichia coli* phosphofructokinase, *Escherichia coli* deadenylylated glutamine synthetase, *Escherichia coli* adenylylated glutamine synthetase and *Mycobacterium tuberculosis* shikimate kinase was determined. The results are reflected in Figures 19 to 25. Where possible, the effect of the ATP and C8D-ATP on the specific activity of the enzyme was expressed over a concentration profile that included the ATP or C8D-ATP concentrations that would allow $v_{max}$ to be calculated as well as an ATP or C8D-ATP concentration profile at low concentrations that would allow for the accurate determination of the KIE. The best-fit to the data was obtained for the specified kinetic model using the non-linear regression algorithms as outlined using the GraphPad Prism$^®$ 5 software. As part of the software output, a data-table was created containing 150 data-points defining the best kinetic fit for each enzymes response to the presence of either ATP or C8D-ATP **(see table 2 for kinetic model).** These response curves were then used to define the KIE by the conventional estimation of KIE from KIE = $v_H/v_D$. The KIE$_D$ was also determined using the following function:

$$KIE_D = \frac{v_D}{v_H}$$

Where $v_D$ = specific activity in the presence of C8D-ATP

$v_H$ = specific activity in the presence of ATP.

The calculation of KIE$_D$ was used as the data obtained is instructive in a putative role that the C8H of ATP plays in the regulation of phosphoryl transfer.

In all 6 cases defined:

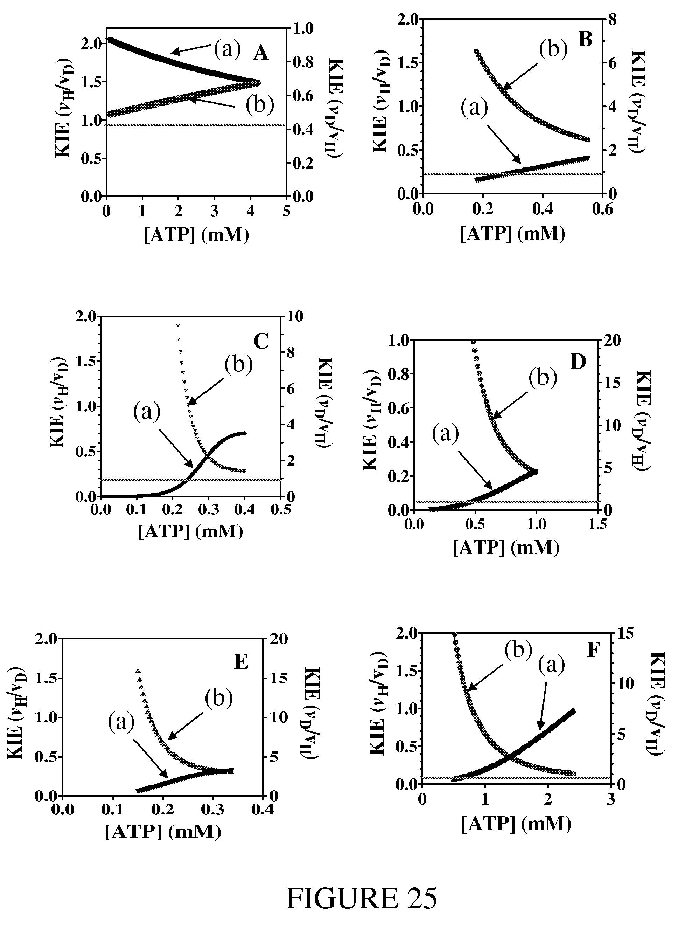

❖ A KIE was obtained in response to presence of C8D-ATP (Figures 19-24).
❖ In all cases other than shikimate kinase, the KIE$_D$ at low ATP concentrations is in excess of 5 (Figure 25).
❖ In monomeric enzymes, such as shikimate kinase, as the concentration of ATP and C8D-ATP was increased, there was a concomitant increase in the KIE$_D$ while in oligomeric enzymes a there was a decrease in the KIE$_D$ (Figure 19).
❖ In all cases the KIE obtained was a primary KIE as extent of the KIE was two-fold or significantly in excess of two-fold at low concentrations.
❖ The KIE$_D$ over the full ATP/C8D-ATP concentration range appeared to be indicative of the mode of regulation of the enzyme as in all cases the KIE either positively or negatively asymptotes to a specific constant value.
❖ The KIE of shikimate kinase asymptotes positively to a KIE of 1.0 as the specific activity tends towards $v_{max}$. The KIE giving a classical KIE effect with the KIE being 2 at low ATP concentrations asymptoting to a level of 1 (Figure 19, Table 2). Shikimate kinase exists as a monomer and therefore no regulation occurs via the interaction of the subunits that may affect the overall KIE.

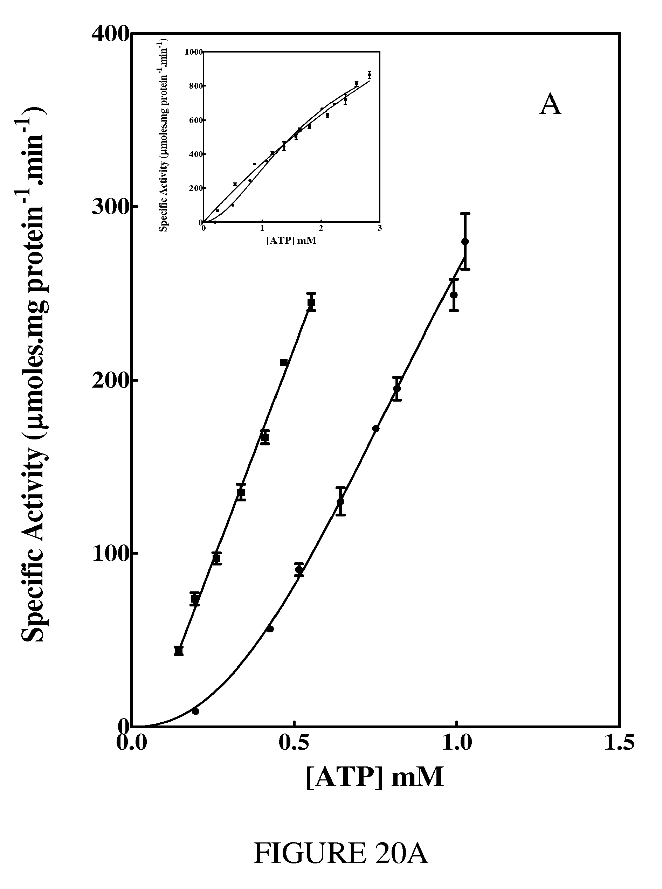

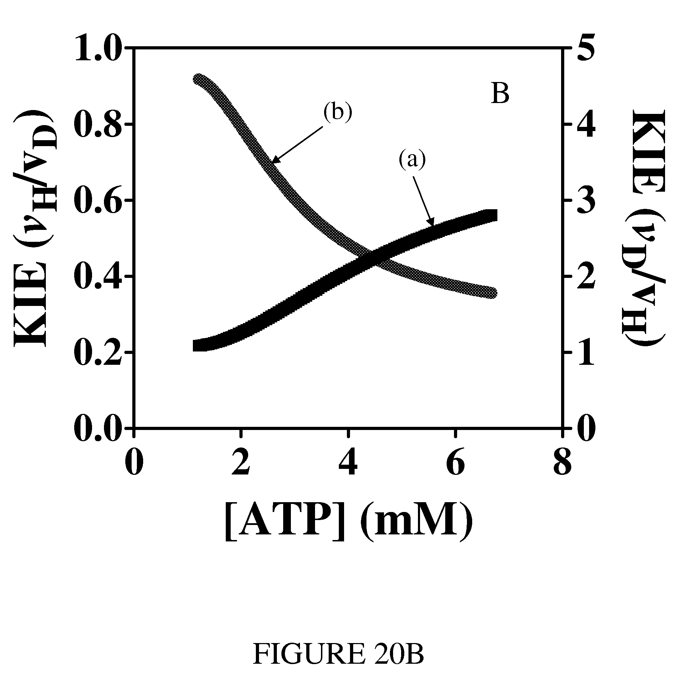

❖ Hexokinase, acetate kinase and $GS_0$ use the same mechanism for regulation. The $KIE_D$ of these enzymes negatively asymptote to 1 at $v_{max}$ (Figures 20-22, Table 2). All three of these enzymes are multi-meric and allosteric regulation may occur via the interaction of subunits. The hexokinase and acetate kinase are both homodimers and monomer interaction plays a role in the regulation of the enzyme activity and ligand binding with the enzyme active sites functioning in a coordinated half-the-sites manner.

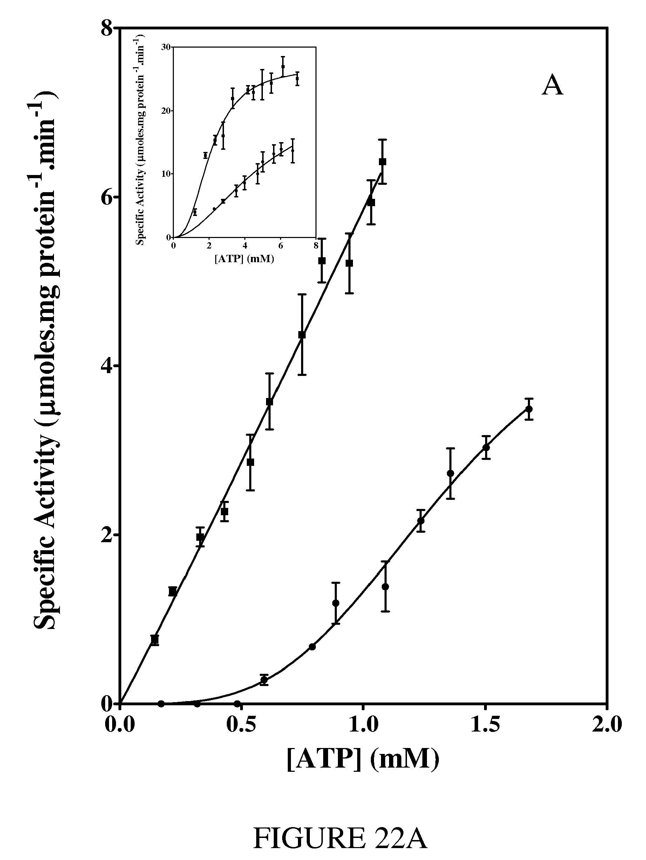

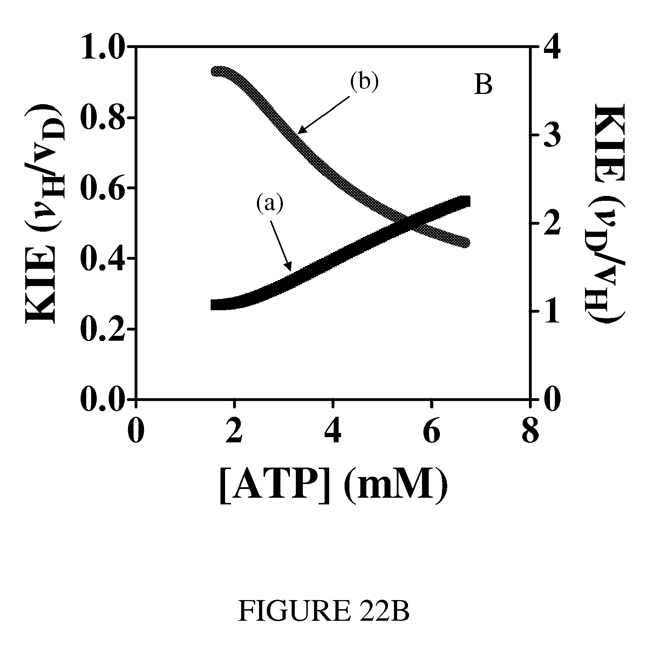

❖ Phosphofructokinase and $GS_{12}$ use a similar mechanism with the $KIE_D$ asymptoting to a level of 0.5 at $v_{max}$ (KIE = 2) (Figures 22-24, Table 2). *E. coli* $GS_{12}$ is a dodecamer consisting of two stacked hexameric structures consisting of 12 identical subunits. The subunits probably interact allosterically on the binding of ATP as occurs in phosphofructokinase. The slow rate of release of C8D-ADP from the interacting active site of $GS_{12}$ probably impacting on the binding of ATP in the adjacent site.

❖ **Table 2.** Effect of the concentration of ATP and C8D-ATP on the fit of the enzyme kinetic model of hexokinase, acetate kinase, adenylylated GS, deadenylylated GS and shikimate kinase. The response of each enzyme to change in the ATP and C8D-ATP concentration was tested for the fit to either an allosteric sigmoidal model or to the Michaelis-Menton model of enzyme kinetics by non-linear regression using the GraphPrism 5 software. The root mean square deviation of the data from the model is as outlined. The Hill factor for the allosteric sigmoidal model is as indicated. $KIE_{vmax}$ is equal to the KIE attained at ATP and C8D-ATP concentrations at maximum enzyme activities.

| Enzyme | Kinetic Model | ATP fit to kinetic model | C8D-ATP fit to kinetic model | $h_{ATP}$ | $h_{C8D}$ | RMSD[a] | | $KIE_{vmax}$ |
|---|---|---|---|---|---|---|---|---|
| | | | | | | ATP | C8D | |
| Hexokinase | Allosteric sigmoidal | Allosteric sigmoidal | Fit ambiguous | 1.75 | | 0.9963 | | 1 |
| | Michaelis-Menton | Fit ambiguous | Michaelis-Menton | | | | 0.9885 | |
| Acetate kinase | Allosteric sigmoidal | Allosteric sigmoidal | Fit ambiguous | 2.08 | | 0.9865 | | 1 |
| | Michaelis-Menton | Fit ambiguous | Michaelis-Menton | | | | 0.9847 | |
| Dedenylylated GS | Allosteric sigmoidal | Allosteric sigmoidal | Allosteric sigmoidal | 3.10 | 4.08 | 0.9972 | 0.9827 | 1 |
| | Michaelis-Menton | Fit ambiguous | Fit ambiguous | | | | | |
| PFK[b] | Allosteric sigmoidal | Allosteric sigmoidal | Allosteric sigmoidal | 1.37 | 1.79 | 0.9982 | 0.9879 | 2 |
| | Michaelis-Menton | Fit ambiguous | Fit ambiguous | | | | | |
| Adenylylated GS | Allosteric sigmoidal | Allosteric sigmoidal | Allosteric sigmoidal | 1.77 | 0.92 | 0.9940 | 0.9990 | 2 |
| | Michaelis-Menton | Fit ambiguous | Fit ambiguous | | | | | |
| Shikimate kinase | Allosteric sigmoidal | Allosteric sigmoidal | Allosteric sigmoidal | 1.22 | 1.06 | 0.9870 | 0.9770 | 1 |
| | Michaelis-Menton | Fit ambiguous | Fit ambiguous | | | | | |

❖
❖ [a] Root mean square deviation of the data defining the kinetic model.
❖ [b] Phosphofructokinase

**Discussion**

The role of the KIE in the kinetics of the enzymes investigated lead to models for the regulation of the binding of ATP being proposed, as set out in Figure 25.

In classical steady-state kinetics as represented by the Briggs-Haldane modification of the Michael-Menton formulation (Equation 1),

$$E + S \underset{k_{on}}{\overset{k_{off}}{\rightleftharpoons}} ES \xrightarrow{k_{cat}} E + P \qquad \text{(Eq. 1)}$$

and $k_{on} = k_1$, $k_{off} = k_{-1}$ and $k_{cat} = k_2$, $k_2 \gg k_{-1}$, and the Michaelis constant, $K_M$ is obtained from

$$\frac{[E][S]}{[ES]} = \frac{k_{-1} + k_2}{k_1} = K_M \qquad \text{(Eq. 2)}$$

In monomeric enzymes such a shikimate kinase $K_M$ is dependent only on $k_2$. The effect of the increase in the ATP/C8D-ATP concentration on the KIE therefore only manifests as the classical effect with the KIE being of the order of 2.0 as determined by $v_H/v_D$, at low concentrations, asymptoting to 1 at high ATP concentrations. At low concentrations of ATP the enzyme activity is dominated by the impact of the C8H/C8D on the equilibrium of binding. At high ATP concentrations the impact of the increase in the ATP concentration on the equilibrium overrides the effect of the C8H/C8D on binding resulting in a decrease in the KIE. As the classical H/D KIE is of the order of 2, as the concentration of ATP tends towards the concentration at the maximum specific activity, $v_{max}$, where the concentration effect is at its maximum the effect of the C8H/C8D on the KIE is at a minimum and the KIE tends towards 1.

In oligomeric enzymes it is proposed that the deuteration of ATP not only affects the binding of ATP to the site where catalysis is occurring but the deuteration also affects the interaction between sites. In oligomeric kinases it is proposed that mechanistically two modes of regulation occur, one which is dependent on the release of ADP from the first active site before ATP binds to the second active site (Figure 26B) and the second mode of regulation depends on the conversion of ATP to ADP prior to the binding of the ATP to the second active site (Figure 26C). In the mechanism outlined in Figure 25C binding to the second site can occur prior to the release of ATP from the first site.

It is proposed that in enzymes such as acetate kinase, hexokinase and $GS_{12}$ the enzyme kinetics follows classical Michaelis-Menton kinetics where an equilibrium is set up between the enzyme concentration [E] and the substrate concentration [S] and binding of the second ATP is dependent on the conversion of the second active site into an ATP binding form by the release of ATP from the first active site, as defined by the coordinated half-sites mechanism. In enzymes using this mechanism of regulation, $K_M$ is dependent on $k_{-1}$ and $k_2$. The KIE obtained in these enzymes asymptotes to a value of 1. At low ATP concentrations the effect of the deuteration of C8 is to allow binding to occur for long enough to allow the reaction to occur and negate the effect of $k_{-1}$, thereby shifting the equilibrium to $k_2$. At low ATP concentrations therefore the impact of the deuteration on the binding is to retard the release of the ATP. At high ATP concentrations the impact of the ATP concentration relative to the impact of ATP binding on the rate of reaction is significantly higher and as a result there is a concomitant increase in the KIE. The impact of binding and the reaction rate however equilibrate to a KIE of 1. The maximum rate of binding can only ever be equivalent to the maximum rate at which the second ATP binding site is converted to the ATP binding form by the release of ATP from the first site (Figure 26B). The classical impact of deuteration on the KIE when the KIE is a primary effect, as determined by $v_H/v_D$, should yield a KIE of 2 or more. As the regulation of the enzyme activity and ligand binding in these enzymes function in a coordinated half-the-sites manner binding in the second site only occurs on release of the ADP from the first site, it is therefore proposed that deuteration of the ATP improves the binding characteristics. As the equilibrium shifts towards the impact of increasing ATP concentration on the enzyme activity the deuterated ATP binds effectively twice as efficiently as the non-deuterated ATP thereby negating the impact of the deuteration on the apparent enzyme activity at high ATP concentrations, yielding a KIE of 1.

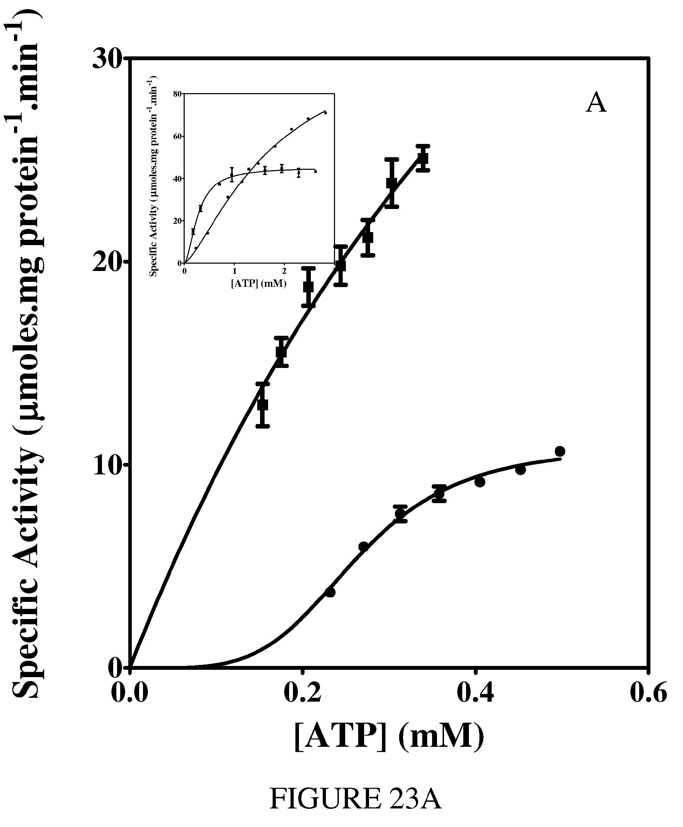

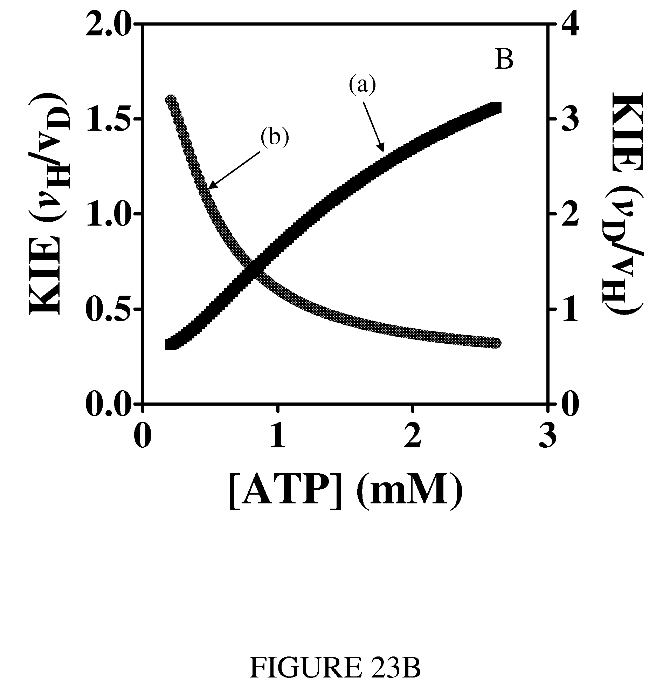

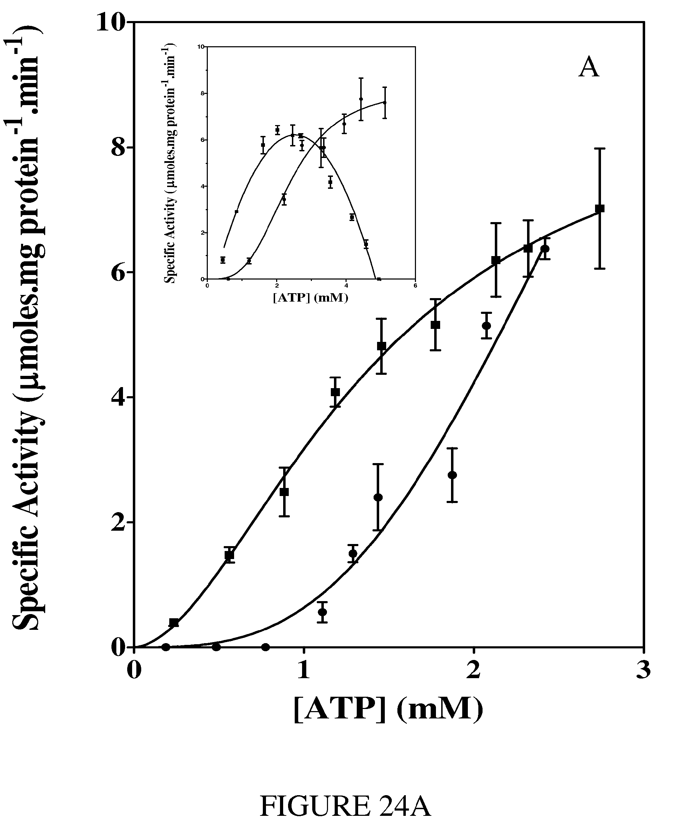

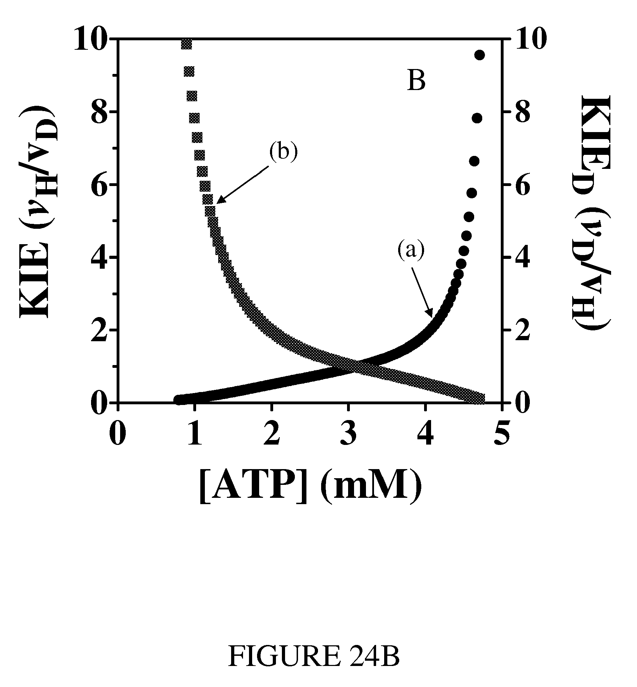

In enzymes where the second active site is made amenable to ATP binding by the conversion of ATP to ADP, in other words binding may occur to the second site prior to the release of the ATP from the first site, the $K_M$ is dependent on $k_1$ and $k_2$. This occurs in the case of phosphofructokinase and $GS_{12}$ where the KIE becomes 2 at $v_{max}$ (Figure 23 & 24). The impact of this binding is that at any point in time up to two or more reactions might be occurring simultaneously in two active sites. In multi-meric enzymes this effect might be greater. As the deuterated ATP binds twice as efficiently as the non-deuterated ATP this allows the KIE to asymptote to 2 or more. It is proposed that a result of the adenylylation of GS it allows for the regulation of the enzyme by a similar mechanism as occurs in phosphofructokinase. Bacterial PFK is a homoteramer, with the four subunits assembled as a dimer of dimers. It is conceivable that on adenylylation of GS the interaction between two-subunits effectively creates a dimer of dimer interaction.

The data outlined clearly demonstrates the role of C8H of ATP in the kinetics of a number of kinase and synthetase enzymes. The KIE is clearly a primary KIE however the extremely high values of the KIE obtained at low at concentrations in the case of the oligomeric enzymes does not appear to be as a result of the impact of the deuterium on the rate the phosphoryl transfer mechanism *per se* but rather as a result of the role that the C8H plays in the equilibrium of binding of the ATP to the active site (Figure 25). Clearly the regulation of enzyme activity in kinases and synthetases is complex which manifests in the apparent $K_m$ of the kinases ranges from less than 0.4 $\mu$M to in excess of 1000 $\mu$M for ATP (Carna Biosciences, Inc., Kinase Profiling Book:www.carnabio.com). The findings of this investigation have discovered that the C8H of ATP plays a direct role in binding of ATP to the active site of enzymes.

The deuteration of compounds containing imidazole moieties that are currently used as drugs, will increase their efficacy. With the increase in the efficacy of the deuterated forms of current drugs containing imidazole moieties either in use or in clinical trails, dosage levels of these compounds may be reduced to alleviate the toxicity.

**Claims**

1. An *in vitro* method of inhibiting the activity of a kinase or a synthetase, the method including binding an active site of the kinase or synthetase with a deuterated imidazole moiety, thereby inhibiting the activity of the kinase or the synthetase.

2. The method of claim 1, wherein it is applied to a kinase, with the kinase being selected from the group consisting of adenylate kinase (AK), shikimate kinase (SK), pyruvate kinase (PK), hexokinase (HXK), aspartokinase (ASK), creatine kinase (CK), glycerate kinase, acetate kinase and phosphofructokinase.

3. The method of claim 2, wherein the kinase is adenylate kinase, whose sequence comprises conserved residues Arg 97, Glu 98, Arg 128 and Asp 180.

4. The method of Claim 1, wherein it is applied to a synthetase, with the synthetase being a glutamine synthetase.

5. The method of claim 1, wherein the kinase or the synthetase, or an isoenzyme thereof, comprises amino acid residues identical to conserved adenylate kinase residues Arg 97, Glu 98, Arg 128 and Asp 180, at positions equivalent to the positions of these residues in adenylate kinase.

6. The method of any one of Claims 1 to 5 inclusive, wherein the deuterated imidazole moiety is provided by a nucleotide, preferably wherein the nucleotide is adenosine triphosphate (ATP), adenosine diphosphate (ADP) or adenosine monophosphate (AMP), with the nucleotide having the C8-H induced to be more acidic, and with deuteration being effected at the C8 position.

7. A deuterated imidazole moiety for use in inhibiting the activity of a kinase or a synthetase, by binding an active site of the kinase or synthetase.

8. The deuterated imidazole moiety of claim 7, wherein it is for application to a kinase, with the kinase being selected from the group consisting of adenylate kinase (AK), shikimate kinase (SK), pyruvate kinase (PK), hexokinase (HXK), aspartokinase (ASK), creatine kinase (CK), glycerate kinase, acetate kinase and phosphofructokinase.

9. The deuterated imidazole moeity of claim 8, wherein the kinase is adenylate kinase, whose sequence comprises conserved residues Arg 97, Glu 98, Arg 128 and Asp 180.

10. The deuterated imidazile moiety claim 7, wherein it is for application to a synthetase, with the synthetase being a glutamine synthetase.

11. A method of generating a compound that inhibits the activity of a kinase or a synthetase, the method comprising providing a three-dimensional structure of a kinase or a synthetase; and designing, based on the three-dimensional structure, a compound capable of inhibiting the activity of the kinase or the synthetase, the compound comprising a deuterated imidazole moiety.

**12.** A method of screening a compound *in vitro* to determine whether or not it inhibits the activity of kinase or a synthetase, the method comprising contacting a kinase or a synthetase with a compound comprising a protonated imidazole moiety; contacting the kinase or the synthetase with the same compound comprising a deuterated imidazole moiety; and determining whether or not the activity of the kinase or synthetase is reduced in the presence of the compound containing the deuterated imidazole moiety relative to the activity of the same kinase or synthetase in the presence of the compound containing the protonated imidazole moiety.

**13.** The method of Claim 11 or Claim 12 wherein, when it is applied to a kinase, the kinase is selected from a group consisting of adenylate kinase (AK), shikimate kinase (SK), pyruvate kinase (PK), hexokinase (HXK), aspartokinase (ASK), creatine kinase (CK), glycerate kinase, acetate kinase, phosphofructokinase, and wherein, when it is applied to a synthetase, the synthetase is a glutamine synthetase.

**14.** The method of any one of Claims 11 to 13 inclusive, wherein the deuterated imidazole moiety is provided by a nucleotide or by a nucleotide analogue preferably wherein the nucleotide is adenosine triphosphate (ATP), adenosine diphosphate (ADP) or adenosine monophosphate (AMP), with the nucleotide having an immonium moiety at position N7, so that a carbene is induced at the C8 position, and with deuteration being effected at the C8 position.

**15.** The method of Claim 6 or 14, wherein the nucleotide is a compound which is deuterated at a position equivalent to C8 in ATP, ADP and AMP.

## Patentansprüche

**1.** Ein *in vitro* Verfahren zur Hemmung der Aktivität einer Kinase oder Synthetase, umfassend das Binden an einem aktiven Zentrum der Kinase oder Synthetase mit einem deuterierten Imidazolrest, wobei die Aktivität der Kinase oder der Synthetase gehemmt wird.

**2.** Das Verfahren nach Anspruch 1, worin dieses bei einer Kinase angewendet wird und die Kinase ausgewählt ist aus der Gruppe, bestehend aus Adenylatkinase (AK), Shikimatkinase (SK), Pyruvatkinase (PK), Hexokinase (HXK), Aspartokinase (ASK), Creatinkinase (CK), Glyceratkinase, Acetatkinase und Phosphofruktokinase.

**3.** Das Verfahren nach Anspruch 2, worin die Kinase Adenylatkinase ist, deren Sequenz die konservierten Reste Arg 97, Glu 98, Arg 128 und Asp 180 umfasst.

**4.** Das Verfahren nach Anspruch 1, worin dieses auf eine Synthetase angewendet wird und die Synthetase eine Glutaminsynthetase ist.

**5.** Das Verfahren nach Anspruch 1, worin die Kinase oder die Synthetase oder ein Isoenzym davon Aminosäurereste umfasst, die identisch sind mit den konservierten Adenylatkinaseresten Arg 97, Glu 98, Arg 128 und Asp 180, an äquivalenten Positionen zu den Positionen dieser Reste in der Adenylatkinase.

**6.** Das Verfahren nach einem der Ansprüche 1 bis 5, worin der deuterierte Imidazolrest durch ein Nukleotid bereitgestellt wird, vorzugsweise worin das Nukleotid Adenosintriphosphat (ATP), Adenosindiphosphat (ADP) oder Adenosinmonophosphat (AMP) ist, wobei das Nukleotid das C8-H induziert aufweist, um saurer zu sein und wobei die Deuterierung an der C8-Position erfolgt ist.

**7.** Ein deuterierter Imidazolrest zur Verwendung bei der Hemmung der Aktivität einer Kinase oder Synthetase durch Bindung an einem aktiven Zentrum der Kinase oder Synthetase.

**8.** Der deuterierte Imidazolrest nach Anspruch 7, worin dieses zur Anwendung an einer Kinase dient, wobei die Kinase ausgewählt ist aus der Gruppe, bestehend aus Adenylatkinase (AK), Shikimatkinase (SK), Pyruvatkinase (PK), Hexokinase (HXK), Aspartokinase (ASK), Creatinkinase (CK), Glyceratkinase, Acetatkinase und Phosphofruktokinase.

**9.** Der deuterierte Imidazolrest nach Anspruch 8, worin die Kinase Adenylatkinase ist, deren Sequenz die konservierten Reste Arg 97, Glu 98, Arg 128 und Asp 180 umfasst.

**10.** Der deuterierte Imidazolrest nach Anspruch 7, worin dieser zur Anwendung an einer Synthetase dient und die Synthetase eine Glutaminsynthetase ist.

**11.** Ein Verfahren zur Herstellung einer Verbindung, die die Aktivität einer Kinase oder einer Synthetase hemmt, umfassend das Bereitstellen einer dreidimensionalen Struktur einer Kinase oder einer Synthetase und Entwerfen einer Verbindung, basierend auf der dreidimensionalen Struktur, die geeignet ist die Aktivität der Kinase oder der Synthetase zu hemmen, wobei die Verbindung einen deuterierten Imidazolrest umfasst.

**12.** Ein Verfahren zur Selektion einer Verbindung *in vitro,* um zu ermitteln, ob diese die Aktivität einer Kinase oder einer Synthetase hemmt oder nicht, umfassend das Inkontaktbringen einer Kinase oder einer Synthetase mit einer Verbindung, umfassend einen protonierten Imidazolrest, Inkontaktbringen der Kinase oder der Synthetase mit derselben Verbindung, umfassend einen deuterierten Imidazolrest und Ermittlung, ob die Aktivität der Kinase oder Synthetase in Gegenwart einer Verbindung, enthaltend den deuterierten Imidazolrest, relativ zu der Aktivität derselben Kinase oder Synthetase in Gegenwart der Verbindung, enthaltend den protonierten Imidazolrest, reduziert wurde oder nicht.

**13.** Das Verfahren nach Anspruch 11 oder 12, worin, wenn dieses bei einer Kinase angewendet wird und die Kinase ausgewählt ist aus einer Gruppe, bestehend aus Adenylatkinase (AK), Shikimatkinase (SK), Pyruvatkinase (PK), Hexokinase (HXK), Aspartokinase (ASK), Creatinkinase (CK), Glyceratkinase, Acetatkinase und Phosphofruktokinase und wobei, wenn dieses bei einer Synthetase angewendet wird, die Synthetase eine Glutaminsynthetase ist.

**14.** Das Verfahren nach einem der Ansprüche 11 bis 13, worin der deuterierte Imidazolrest durch ein Nukleotid oder ein Nukleotidanalogon bereitgestellt wird, vorzugsweise worin das Nukleotid Adenosintriposphat (ATP), Adenosindipohsphat (ADP) oder Adenosinmonophosphat (AMP) ist, wobei das Nukleotid einen Iminiumrest an Position N7 aufweist, so dass ein Carben in C8 Position induziert ist und wobei die Deuterierung an der C8-Position erfolgt.

**15.** Das Verfahren nach Anspruch 6 oder 14, worin das Nukleotid eine Verbindung ist, die an einer äquivalenten Position zu C8 in ATP, ADP und AMP deuteriert ist.

## Revendications

**1.** Procédé *in vitro* d'inhibition de l'activité d'une kinase ou d'une synthétase, le procédé comprenant la liaison d'un site actif de la kinase ou synthétase avec une fraction imidazole deutérée, inhibant ainsi l'activité de la kinase ou la synthétase.

**2.** Procédé selon la revendication 1, **caractérisé en ce qu'**il est appliqué à une kinase, la kinase étant choisie dans le groupe constitué par de l'adénylate kinase (AK), de la shikimate kinase (SK), de la pyruvate kinase (PK), de l'hexokinase (HXK), de l'aspartokinase (ASK), de la créatine kinase (CK), de la glycérate kinase, de l'acétate kinase et de la phospho-fructokinase.

**3.** Procédé selon la revendication 2, **caractérisé en ce que** la kinase est l'adénylate kinase, dont la séquence comprend les résidus conservés Arg 97, Glu 98, Arg 128 et Asp 180.

**4.** Procédé selon la revendication 1, **caractérisé en ce qu'**il est appliqué à une synthétase, la synthétase étant une glutamine synthétase.

**5.** Procédé selon la revendication 1, dans lequel la kinase ou la synthétase, ou bien une isoenzyme de celle-ci, comprend des résidus d'acides aminés identiques aux résidus d'adénylate kinase conservés Arg 97, Glu 98, Arg 128 et Asp 180, en des positions équivalentes aux positions de ces résidus dans l'adénylate kinase.

**6.** Procédé selon l'une quelconque des revendications 1 à 5 incluse, dans lequel la fraction imidazole deutérée est fournie par un nucléotide, de préférence dans lequel le nucléotide est l'adénosine triphosphate (ATP), l'adénosine diphosphate (ADP) ou l'adénosine monophosphate (AMP), le nucléotide ayant le C8-H induit pour être plus acide, et la deutérisation étant effectuée à la position C8.

**7.** Fraction imidazole deutérée destinée à être utilisée dans l'inhibition de l'activité d'une kinase ou d'une synthétase en se liant à un site actif de la kinase ou synthétase.

**8.** Fraction imidazole deutérée selon la revendication 7, dans laquelle elle est destinée à être appliquée à une kinase, la kinase étant choisie dans le groupe constitué de l'adénylate kinase (AK), de la shikimate kinase (SK), de la pyruvate kinase (PK), de l'hexokinase (HXK), de l'aspartokinase (ASK), de la créatine kinase (CK), de la glycérate kinase, de l'acétate kinase et de la phospho-fructokinase.

**9.** Fraction imidazole deutérée selon la revendication 8, dans laquelle la kinase est l'adénylate kinase, dont la séquence comprend les résidus conservés Arg 97, Glu 98, Arg 128 et Asp 180.

**10.** Fraction imidazole deutérée selon la revendication 7, dans laquelle elle est destinée à une application à une synthétase, la synthétase étant une glutamine synthétase.

**11.** Procédé de génération d'un composé qui inhibe l'activité d'une kinase ou d'une synthétase, le procédé comprenant la fourniture d'une structure tridimensionnelle d'une kinase ou d'une synthétase ; et la conception, sur la base de la structure tridimensionnelle, d'un composé étant capable d'inhiber l'activité de la kinase ou synthétase, le composé comprenant une fraction imidazole deutérée.

**12.** Procédé de criblage d'un composé in vitro pour déterminer s'il inhibe ou non l'activité d'une kinase ou d'une synthétase, le procédé comprenant la mise en contact d'une kinase ou d'une synthétase avec un composé comprenant une fraction imidazole protonnée ; la mise en contact de la kinase ou de la synthétase avec le même composé comprenant une fraction imidazole deutérée ; et la détermination de si l'activité de la kinase ou synthétase est réduite ou non en présence du composé contenant la fraction imidazole deutérée, par rapport à l'activité de la même kinase ou synthétase en présence du composé contenant la fraction imidazole protonnée.

**13.** Procédé selon la revendication 11 ou la revendication 12, **caractérisé en ce que** lorsqu'il est appliqué à une kinase, la kinase est choisie dans le groupe constitué par de l'adénylate kinase (AK), de la shikimate kinase (SK), de la pyruvate kinase (PK), de l'hexokinase (HXK), de l'aspartokinase (ASK), de la créatine kinase (CK), de la glycérate kinase, de l'acétate kinase, de la phospho-fructokinase, et **caractérisé en ce que** lorsqu'il est appliqué à une synthétase, la synthétase est une glutamine synthétase.

**14.** Procédé selon l'une quelconque des revendications 11 à 13 incluse, **caractérisé en ce que** la fraction imidazole deutérée est fournie par un nucléotide ou par un analogue nucléotidique, de préférence dans lequel le nucléotide est l'adénosine triphosphate (ATP), l'adénosine diphosphate (ADP) ou l'adénosine monophosphate (AMP), le nucléotide ayant une fraction d'immonium en position N7 de sorte qu'un carbène soit induit à la position C8, la deutérisation étant effectuée à la position C8.

**15.** Procédé selon la revendication 6 ou 14, dans lequel le nucléotide est un composé qui est deutéré en une position équivalente à la position C8 dans l'ATP, l'ADP et l'AMP.

```
FILE: Multiple_Sequence_Alignment Adenylate Kinase
PROJECT:
NUMBER: 6
MAXLENGTH: 243
NAMES: KAD1_HUMAN KAD5_HUMAN KAD6_HUMAN KAD2_HUMAN KAD3_HUMAN KAD4_HUMAN
MAXNAMELEN: 10
FEATURES
ORIGIN
KAD1_HUMAN .......MEEKLKKTKIIFVVGGPGSGKGTQCEKIVQKYGYTHLSTGDLL      43
KAD5_HUMAN ....mggfmEdLrKcKIIFiiGGPGSGKGTQCEKlVeKYGfTHLSTGeLL      46
KAD6_HUMAN ............mllpnIlltGtPGvGKtTlgkelasKsGlkyinvGDLa      38
KAD2_HUMAN mapsvpaaEpeypKgiravllGpPGaGKGTQaprlaenfcvcHLaTGDmL      50
KAD3_HUMAN ........mgasarllravimGaPGSGKGTvssrItthfelkHLSsGDLL      42
KAD4_HUMAN ..........masKllravilGpPGSGKGTvCqrIaQnfGlqHLSsGhfL      40
Consensus            kl ravi g pgsgkgtqc ria kfgl hlstgdll


KAD1_HUMAN RSEVSSGSARGKKLSEIMEKGQLVPLETVLDMLRDAMVAKVNTSKGFLID      93
KAD5_HUMAN ReElaSeSeRsKlirdIMErGdLVPsgiVLelLkeAMVAslgdtrGFLID      96
KAD6_HUMAN ReEqlydgydeeydcpIld......edrVvDeLdnqMre..........g      72
KAD2_HUMAN RamVaSGSelGKKLkatMdaGkLVsdEmVvelieknletplckng.FL1D      99
KAD3_HUMAN RdnmlrGteiGvlakafidqGkLiPddvmtrlalhelkn.ltqys.wLlD      90
KAD4_HUMAN RenikastevGemakqyiEKslLVPdhvitrlmmselen.rrgqh.wLlD      88
Consensus  ree  sgse gk  k imd g lvpd  v  ll   l   l      flld


KAD1_HUMAN GYPREVQQGEEFERRIGQ....PTLLLYVDAGPETMTQRLLKRGETS...     136
KAD5_HUMAN GYPREVkQGEEFgRRIGd....PqLvicmDcsadTMTnRLLqRsrsS...     139
KAD6_HUMAN GvivdyhgcdfFpeRwf......hivfvlrtdtnvlyeRLetRGyne...     113
KAD2_HUMAN GfPRtVrQaEmlddlmekrkekldsviefsipdsllirRitgRlihpksg     149
KAD3_HUMAN GfPRtlpQaEaldRayq.....idtvinlnvpfEvikQRLtaRwihpasg     135
KAD4_HUMAN GfPRtlgQaEaldkice.....vdLvislnipfETlkdRLsrRwihppsg     133
Consensus  gfprtv qae fdrr        dlvi l  p etl  rl  r ihp sg


KAD1_HUMAN .......................GRVDDNEETIKKRLETYYKATEPVIA     162
KAD5_HUMAN .......................lpVDDttkTIaKRLEaYYrAsiPVIA     165
KAD6_HUMAN .......................kkltDNiqceifqvlyeeatasykee     139
KAD2_HUMAN rsyheefnppkepmkdditgeplirRsDDNEkalKiRLqaYhtqTtPlIe     199
KAD3_HUMAN rvyniefnppktvgiddltgepliqReDDkpETviKRLkaYedqTkPVle     185
KAD4_HUMAN rvynldfnpphvhgiddvtgeplvqqeDDkpEavaaRLrqYkdvakPVIe     183
Consensus   r  y   fnpp     dd tgepl  r ddn et  krl ay    t pvie


KAD1_HUMAN FYEKRGIVRKVNAEGSVDSVFSQVCTHLDALK...........     194
KAD5_HUMAN yYEtktqlhKiNAEGtpedVFlQlCTaiDsiif..........     198
KAD6_HUMAN ivhqlpsnkpeelEnnVDqilkwieqwikdhns..........     172
KAD2_HUMAN yYrKRGIhsaidAsqtpDvVFasilaafs.katckdlvmfi..     239
KAD3_HUMAN yYqKkGvletfsgtet.nkiwpyVyafLqtkvpqrsqkasvtp     227
KAD4_HUMAN lYksRGvlhqfsgtet.nkiwpyVyTlfsnkitpiqskeay..     223
Consensus  yy krg l     ae t d if  v  t    k
```

# FIGURE 1

```
FILE: Multiple_Sequence_Alignment Shikimate kinase
PROJECT:
NUMBER: 9
MAXLENGTH: 183
NAMES: SK1_ECOLI SK1_KPNEUMONIAE SK1_SFLEXNERI SK1_YPESTIS SK1_MTUBERCULOSI SK2_ECOLI
      SK2_KPNEUMONIAE SK2_SFLEXNERI SK2_YPESTIS
MAXNAMELEN: 16
FEATURES
ORIGIN
SK1_ECOLI        MAEKRNIFLVGPMGAGKSTIGRQLAQQLNMEFYDSDQEIEKRTGADVGWV        50
SK1_KPNEUMONIAE  MAEKRNIFLVGPMGAGKSTIGRQLAQQLNMEFYDSDQEIEKRTGADVGWV        50
SK1_SFLEXNERI    MAEKRNIFLVGPMGAGKSTIGRQLAQQLNMEFYDSDQEIEKRTGADVGWV        50
SK1_YPESTIS      MAEKRNIFLVGPMGAGKSTIGRQLAQQLNMEFfDSDQEIErRTGADVGWV        50
SK1_MTUBERCULOSI ..mapkavLVGlpGsGKSTIGRrLAkaLgvgllDtDvaIEqRTGrsiadi       48
SK2_ECOLI        ..mtqplFLiGPrGcGKtTvGmaLAdsLNrrFvDtDQwlqsqlnmtVaei       48
SK2_KPNEUMONIAE  ..mtqpIFLiGPrGcGKtTvGhaLArarhfqFsDtDhrlqaheqrtVaei       48
SK2_SFLEXNERI    ..mtqplFLiGPrGcGKtTvGmaLAdsLNrrFvDtDQwlqsqlnmtVaei       48
SK2_YPESTIS      ..mtqtIFmVGarGAGKtTIGkaLAQaLgyrFvDtDlfmqqtsqmtVaeV       48
Consensus          m   iflvgp gagkstigr laq ln  f dtdq ie rtg  va v

SK1_ECOLI        FDLEGEEGFRDREEKVINELTEKQGIVLATGGGSVKSRETRNRLSARGVV      100
SK1_KPNEUMONIAE  FDvEGEEGFRDREEKiINELTEKQGIVLATGGGSVKSRETRNRLSARGVV      100
SK1_SFLEXNERI    FDLEGEEGFRDREEKVINELTEKQGIVLATGGGSVKSRETRNRLSARGVV      100
SK1_YPESTIS      FDvEGEEGFRDREEKVINELTEKQGIVLATGGGSVKSRETRNRLSARGVV      100
SK1_MTUBERCULOSI FatdGEqeFRriEEdVvraaladhdgVLslGGGaVtSpgvRaaLagh.tV       97
SK2_ECOLI        verEewaGFRaREtaaleavTaps.tViATGGGiiltefnRhfmqnnGiV       97
SK2_KPNEUMONIAE  vqaEGwarFRelEtlslkavTlpn.tViATGGGiVlaegnRqfmrenGVV       97
SK2_SFLEXNERI    verEewaGFRaREtaaleavTaas.tViATGGGiiltefnRhfmqnnGiV       97
SK2_YPESTIS      vesEGwdGFRlREsmalqavTapk.tVvATGGGaVlSsEnRafmrdhGrV       97
Consensus          f  ege gfr ree   a t    vlatggg v s er  l   gvv

SK1_ECOLI        VYLET...TIEKQLARTQRDKKRPLLHVETPPREVLEALANERNPLYEEI      147
SK1_KPNEUMONIAE  VYLET...TIEKQLARTQRDKKRPLLqVdaPPREVLEALAdERNPLYEEI      147
SK1_SFLEXNERI    VYLET...TIEKQLARTQRDKKRPLLHVETPPREVLEALANERNPLYEEI      147
SK1_YPESTIS      VYLET...TIEKQLARTQRDKKRPLLqVdePPREVLEALAkERNPLYEEI      147
SK1_MTUBERCULOSI VYLEis...aaegvrRTggntvRPLLagpdraekyralmA.kRaPLYrrv      143
SK2_ECOLI        VYLcapvsvlvnrLqaapeedlRPtLtgkplseEVqEvLe.ERdaLYrEv      146
SK2_KPNEUMONIAE  iYLqasvsalidrLeaypkaeqRPtLtgkpvreEVgEvLA.qReaLYrda      146
SK2_SFLEXNERI    VYLcapvsvlvnrLqaapeedlRPtLtgkplseEVgEvLe.ERdaLYrEv      146
SK2_YPESTIS      iYLrasaavlaKrLAedpeeaqRPsLtgkpiveEiLdvLA.sReaLYqdv      146
Consensus          vyle     k lart   rpll g   eevle la er ply e

SK1_ECOLI        ADVTIRTDDQSAKVVANQIIHMLESN.......                      173
SK1_KPNEUMONIAE  ADVTIRTDDQSAKVVANQIIHMLESN.......                      173
SK1_SFLEXNERI    ADVTIRTDDQSAKVVANQIIHMLESN.......                      173
SK1_YPESTIS      ADVTIRTDDQSAKVVANQIInMLESN.......                      173
SK1_MTUBERCULOSI AtmrvdTnrrnpgaVvrhIlsrLqvpspseaat                      176
SK2_ECOLI        AhiiIdatnepsqVis.eIrsaLaqtinc....                      174
SK2_KPNEUMONIAE  AhhivdataspdrVVe.QImsMLcSatatpvs.                      177
SK2_SFLEXNERI    AhiiIdatnepsqVis.eIrsaLaqtinc....                      174
SK2_YPESTIS      Ahhvldgtqtpsl VVe.QIlqMLtgemvk....                     174
Consensus          a  idt    vv  qi  ml s
```

# FIGURE 2

```
FILE: Multiple_Sequence_Alignment Pyruvate kinase
PROJECT:
NUMBER: 4
MAXLENGTH: 574
NAMES: RAT_M1 RAT_M2 RAT_R RAT_L
MAXNAMELEN: 10
FEATURES
ORIGIN
RAT_M1      .........................................MSKSHSE      7
RAT_M2      .........................................MpKpdSE      7
RAT_R       msvqentlpqqlwpwifrsqkdlaksalsgapggpagylrrasvaqltqE    50
RAT_L       ............................megpagylrrasvaqltqE        19
Consensus                            gpagylrrasmakltqe

RAT_M1      AGSAFIQTQQLHAAMADTFLEHMCRLDIDSAPITARNTGIICTIGPASRS    57
RAT_M2      AGtAFIQTQQLHAAMADTFLEHMCRLDIDSAPITARNTGIICTIGPASRS    57
RAT_R       lGtAFfQqQQLpAAMADTFLEHlClLDIDSqPvaARsTsIIaTIGPASRS   100
RAT_L       lGtAFfQqQQLpAAMADTFLEHlClLDIDSqPvaARsTsIIaTIGPASRS    69
Consensus   agtaffqqqqlhaamadtflehlclldidsapiaarntgiiatigpasrs

RAT_M1      VETLKEMIKSGMNVARMNFSHGTHEYHAETIKNVRTATESFASDPILYRP   107
RAT_M2      VEmLKEMIKSGMNVARlNFSHGTHEYHAETIKNVRaATESFASDPILYRP   107
RAT_R       VdrLKEMIKaGMNiARlNFSHGsHEYHAEsIaNiReATESFAtsPlsYRP   150
RAT_L       VdrLKEMIKaGMNiARlNFSHGsHEYHAEsIaNiReATESFAtsPlsYRP   119
Consensus   vdrlkemikagmniarlnfshgsheyhaesianireatesfasdpilyrp

RAT_M1      VAVALDTKGPEIRTGLIKGSGTAEVELKKGATLKITLDNAYMEKCDENIL   157
RAT_M2      VAVALDTKGPEIRTGLIKGSGTAEVELKKGATLKITLDNAYMEKCDENIL   157
RAT_R       VAiALDTKGPEIRTGvlqGgpesEVEivKGsqvlvTvDpkfqtrgDaktv   200
RAT_L       VAiALDTKGPEIRTGvlqGgpesEVEivKGsqvlvTvDpkfqtrgDaktv   169
Consensus   vaialdtkgpeirtglikgggeaeveikkgaqlkitldnafmekcdakil

RAT_M1      WLDYKNICKVVDVGSKVYVDDGLISLQVKQKGPDFLVTEVENGGFLGSKK   207
RAT_M2      WLDYKNICKVVeVGSKiYVDDGLISLQVKeKGaDyLVTEVENGGsLGSKK   207
RAT_R       WvDYhNItrVVaVGgriYiDDGLISLvVqkiGPegLVTEVEhGGiLGSrK   250
RAT_L       WvDYhNItrVVaVGgriYiDDGLISLvVqkiGPegLVTEVEhGGiLGSrK   219
Consensus   wldyhnickvvavggkiyiddglislqvkkigpdglvtevehggilgskk

RAT_M1      GVNLPGAAVDLPAVSEKDIQDLKFGVEQDVDMVFASFIRKAADVHEVRKI   257
RAT_M2      GVNLPGAAVDLPAVSEKDIQDLKFGVEQDVDMVFASFIRKAADVHEVRKv   257
RAT_R       GVNLPnteVDLPglSEqDllDLrFGVqhnVDiiFASFvRKAsDVlaVRda   300
RAT_L       GVNLPnteVDLPglSEqDllDLrFGVqhnVDiiFASFvRKAsDVlaVRda   269
Consensus   gvnlpgaavdlpalsekdildlkfgvehdvdiifasfirkaadvhavrda

RAT_M1      LGEKGKNIKIISKIENHEGVRRFDEILEASDGIMVARGDLGIEIPAEKVF   307
RAT_M2      LGEKGKNIKIISKIENHEGVRRFDEILEASDGIMVARGDLGIEIPAEKVF   307
RAT_R       LGpeGqNIKIISKIENHEGVkkFDEILEvSDGIMVARGDLGIEIPAEKVF   350
RAT_L       LGpeGqNIKIISKIENHEGVkkFDEILEvSDGIMVARGDLGIEIPAEKVF   319
Consensus   lgeegknikiiskienhegvkkfdeileasdgimvargdlgieipaekvf

RAT_M1      LAQKMIIGRCNRAGKPVICATQMLESMIKKPRPTRAEGSDVANAVLDGAD   357
RAT_M2      LAQKMmIGRCNRAGKPVICATQMLESMIKKPRPTRAEGSDVANAVLDGAD   357
RAT_R       LAQKMmIGRCNlAGKPVvCATQMLESMItKaRPTRAEtSDVANAVLDGAD   400
RAT_L       LAQKMmIGRCNlAGKPVvCATQMLESMItKaRPTRAEtSDVANAVLDGAD   369
Consensus   laqkmmigrcnlagkpvicatqmlesmikkarptraegsdvanavldgad

RAT_M1      CIMLSGETAKGDYPLEAVRMQHLIAREAEAAMFHRKLFEELARSSSHSTD   407
RAT_M2      CIMLSGETAKGDYPLEAVRMQHLIAREAEAAvFHRlLFEELARaSSqSTD   407
RAT_R       CIMLSGETAKGsfPvEAVmMQHaIAREAEAAvyHRqLFEELrRaaplSrD   450
RAT_L       CIMLSGETAKGsfPvEAVmMQHaIAREAEAAvyHRqLFEELrRaaplSrD   419
Consensus   cimlsgetakgdfpleavmmqhaiareaeaavfhrqlfeelaraaplsrd

RAT_M1      LMEAMAMGSVEASYKCLAAALIVLTESGRSAHQVARYRPRAPIIAVTRNH   457
RAT_M2      plEAMAMGSVEASYKCLAAALIVLTESGRSAHQVARYRPRAPIIAVTRNp   457
RAT_R       ptEvtAiGaVEASfKCcAAAiIVLTktGRSAqllsqYRPRAavIAVTRsa   500
RAT_L       ptEvtAiGaVEASfKCcAAAiIVLTktGRSAqllsqYRPRAavIAVTRsa   469
Consensus   pteamaigaveasfkccaaaiivltesgrsahllaqyrpraaiiavtrna

RAT_M1      QTARQAHLYRGIFPVVCKDPVQEAWAEDVDLRVNLAMNVGKARGFFKKGD   507
RAT_M2      QTARQAHLYRGIFPVlCKDaVldAWAEDVDLRVNLAMNVGKARGFFKKGD   507
RAT_R       QaARQvHLsRGvFPllyrePpeaiWAdDVDrRVqfgiesGKlRGFlrvGD   550
RAT_L       QaARQvHLsRGvFPllyrePpeaiWAdDVDrRVqfgiesGKlRGFlrvGD   519
Consensus   qaarqahlsrgifpllckdppeaawaddvdlrvnfaiesgkargffkkgd

RAT_M1      VVIVLTGWRPGSGFTNTMRVVPVP   531
RAT_M2      VVIVLTGWRPGSGFTNTMRVVPVP   531
RAT_R       lVIVvTGWRPGSGyTNiMRVlsVs   574
RAT_L       lVIVvTGWRPGSGyTNiMRVlsVs   543
Consensus   lvivltgwrpgsgftnimrvlpvp
```

# FIGURE 3

```
FILE: Multiple_Sequence_Alignment Creatine kinase
PROJECT:
NUMBER: 4
MAXLENGTH: 420
NAMES: CKB_HUMAN CKM_HUMAN CKMT1A_HUMAN CKMT2_HUMAN
MAXNAMELEN: 12
FEATURES
ORIGIN
CKB_HUMAN     ....................MPFSNSHNALKLR..............FPA      16
CKM_HUMAN     ....................MPFgNtHNkfKLn..............ykp      16
CKMT1A_HUMAN  magpfsrllsarpglrllalagagslaagflLRpepvraas.errrlyPp      49
CKMT2_HUMAN   masifsklltgrnasllfatMgtSvlttgylLnrqkvcaevreqprlFPp      50
Consensus     ma  fs ll  r     l a mgfgnlhngfkln   v a   e  rlfpp


CKB_HUMAN     EDEFPDLSAHNNHMAKVLTPELYAELRAKSTPSGFTLDDVIQTGVDNPGH      66
CKM_HUMAN     EeEyPDLSkHNNHMAKVLTlELYkkLRdKeTPSGFTvDDVIQTGVDNPGH      66
CKMT1A_HUMAN  saEyPDLrkHNNcMAshLTPavYArLcdKtTPtGwTLDqcIQTGVDNPGH      99
CKMT2_HUMAN   sadyPDLrkHNNcMAecLTPaiYAkLRnKvTPnGyTLDqcIQTGVDNPGH     100
Consensus     eaeypdlrkhnncmakvltpalyaklrdk tpsgftlddciqtgvdnpgh


CKB_HUMAN     PYIMTVGCVAGDEESYEVFKDLFDPIIEDRHGGYKP.SDEHKTDLNPDNL     115
CKM_HUMAN     PfIMTVGCVAGDEESYEVFKeLFDPIIsDRHGGYKP.tDkHKTDLNheNL     115
CKMT1A_HUMAN  PfIkTVGmVAGDEEtYEVFaDLFDPvIqeRHnGYdPrtmkHtTDLdaski     149
CKMT2_HUMAN   PfIkTVGmVAGDEESYEVFaDLFDPvIklRHnGYdPrvmkHtTDLdaski     150
Consensus     pfiktvgcvagdeesyevfadlfdpii drhggydprtdkhktdldaski


CKB_HUMAN     QGGDDLDPNYVLSSRVRTGRSIRGFCLPPHCSRGERRAIEKLAVEALSSL     165
CKM_HUMAN     kGGDDLDPNYVLSSRVRTGRSIkGytLPPHCSRGERRAvEKLsVEALnSL     165
CKMT1A_HUMAN  rsG.yfDerYVLSSRVRTGRSIRGlsLPPaCtRaERRevErvvVdALSgL     198
CKMT2_HUMAN   tqG.qfDehYVLSSRVRTGRSIRGlsLPPaCtRaERRevEnvAitALegL     199
Consensus      ggddfdenyvlssrvrtgrsirglslppacsraerraveklavealsgl


CKB_HUMAN     DGDLAGRYYALKSMTEAEQQQLIDDHFLFDKPVSPLLLASGMARDWPDAR     215
CKM_HUMAN     tGefkGkYYpLKSMTEkEQQQLIDDHFLFDKPVSPLLLASGMARDWPDAR     215
CKMT1A_HUMAN  kGDLAGRYYrLseMTEAEQQQLIDDHFLFDKPVSPLLtAaGMARDWPDAR     248
CKMT2_HUMAN   kGDLAGRYYkLseMTEqdQQrLIDDHFLFDKPVSPLLtcaGMARDWPDAR     249
Consensus     kgdlagryy lkemteaeqqqliddhflfdkpvsplllaagmardwpdar


CKB_HUMAN     GIWHNDNKTFLVWVNEEDHLRVISMQKGGNMKEVFTRFCTGLTQIETLFK     265
CKM_HUMAN     GIWHNDNKsFLVWVNEEDHLRVISMeKGGNMKEVFrRFCvGLqkIEeiFK     265
CKMT1A_HUMAN  GIWHNneKsFLiWVNEEDHtRVISMeKGGNMKrVFeRFCrGLkevErLiq     298
CKMT2_HUMAN   GIWHNydKTFLiWiNEEDHtRVISMeKGGNMKrVFeRFCrGLkevErLiq     299
Consensus     giwhndnksfliwvneedhlrvismekggnmkevferfcrglkeierlfk


CKB_HUMAN     SKDYEFMWNPHLGYILTCPSNLGTGLRAGVHIKLPNLGKHEKFSEVLKRL     315
CKM_HUMAN     kaghpFMWNqHLGYvLTCPSNLGTGLRgGVHvKLahLsKHpKFeEiLtRL     315
CKMT1A_HUMAN  ergwEFMWNerLGYILTCPSNLGTGLRAGVHIKLPlLsKdsrFpkiLenL     348
CKMT2_HUMAN   ergwEFMWNerLGYILTCPSNLGTGLRAGVHvriPkLsKdprFSkiLenL     349
Consensus     ergwefmwnehlgyiltcpsnlgtglragvhiklp lskdpkfseilenl


CKB_HUMAN     RLQKRGTGGVDTAAVGGVFDVSNADRLGFSEVELVQMVVDGVKLLIEMEQ     365
CKM_HUMAN     RLQKRGTGGVDTAAVGsVFDVSNADRLGsSEVEqVQlVVDGVKLmvEMEk     365
CKMT1A_HUMAN  RLQKRGTGGVDTAAtGGVFDiSNlDRLGkSEVELVQlViDGVnyLIdcEr     398
CKMT2_HUMAN   RLQKRGTGGVDTAAVadVyDiSNiDRiGrSEVELVQiViDGVnyLvdcEk     399
Consensus     rlqkrgtggvdtaavggvfdisnadrlg sevelvqlvidgvkllidcek


CKB_HUMAN     RLEQGQAIDDLMPAQK....                                 381
CKM_HUMAN     kLEkGQsIDDmiPAQK....                                 381
CKMT1A_HUMAN  RLErGQdIriptPvihtkh.                                 417
CKMT2_HUMAN   kLErGQdIkvppPlpqfgkk                                 419
Consensus     klergqdiddp paqk
```

# FIGURE 4

```
FILE: Multiple_Sequence_Alignment Glycerate kinase
PROJECT:
NUMBER: 6
MAXLENGTH: 405
NAMES: GARK GLXK S.CHOL_GARK S.CHOL_GLXK V.VUL_1 V.VUL_2
MAXNAMELEN: 11
FEATURES
ORIGIN
GARK         .................MKIVIAPDSYKESLSASEVAQAIEKGFREIFP    32
GLXK         .................MKIVIAPDSfKESLSAekccQAIkaGFstlFP    32
S.CHOL_GARK  .................MKIVIAPDSYKESLSAaEVAQAIEKGFREIFP    32
S.CHOL_GLXK  .................MKIVIAPDSfKESLSAdkccQAIkaGFstvFP    32
V.VUL_1      .................MKIiIAPDSYKESLtAmdVAiAIEKGFkqvlP    32
V.VUL_2      mvsssrpfcsyyfravylMKIVIAPDSfKESLtAkqVcecvEqGFaqvyP    50
Consensus                     mkiviapdsfkeslsa vaqaiekgf  vfp

GARK         DAQYVSVPVADGGEGTVEAMIAATQGAERHAWVTGPLG.....EKVNASW    77
GLXK         DAnYiclPiADGGEGTVdAMvAATgGnivtleVcGPmG.....EKVNAfy    77
S.CHOL_GARK  DAQYVSVPVADGGEGTVEAMIAATQGvERaAWVTGPLG.....EKVkAcW    77
S.CHOL_GLXK  DArYVclPiADGGEGTVdAMvAATgGkrvsvdVsGPmG.....EKVNgfy    77
V.VUL_1      DAhYVklPmADGGEGTVqsMvdATgGtiiehtVTGPLG.....qrVdgff    77
V.VUL_2      qAQYihfPlADGGEGTVevlIqgleGevqHslVaGPLergivdsKerAkW   100
Consensus    daqyv lp adggegtveamiaatgg    vtgplg    ekvnafw

GARK         GISGDGKTAFIEMAAASGLELVPAEKRDPLVTTSRGTGELILQALESGAT   127
GLXK         GltGDGKTAvIEMAAASGLmLVapEKRnPLlasSfGTGELIrhALdndir   127
S.CHOL_GARK  GmSGDGKTAFIEMAAASGLaLVPpEKRnPLiTTSRGTGELILQALESGAs   127
S.CHOL_GLXK  GltGDGKTAiIEMAAASGLmLVapEaRnPLlasSfGTGELIrhALdaGir   127
V.VUL_1      GllGeGKTAvIEMAAASGLhLVtpdqRnPLiTTtfGTGELIkaALdhGve   127
V.VUL_2      alldnGnTAlIEiAAASGLdLlaAseRnPaVTTtyGTGELIskALdlGvn   150
Consensus    gl gdgkta iemaaasgl lvapekrnpl ttsfgtgeli  ald g

GARK         NIIIGIGGSATNDGGAGMVQALGAKLCDANGNEIGFGGGSLNTLNDIDIS   177
GLXK         hIIlGIGGSATvDGGmGMaQALGvrflDAdGqalaanGGnLarvasIemd   177
S.CHOL_GARK  NIIIGIGGSATNDGGAGMmQALGAKLrDANGadIGyGGGSLhcLsDIDIl   177
S.CHOL_GLXK  hIIlGIGGSATvDGGmGvaQALGvrflDAqGtplGaGGGnLsrLasIDlq   177
V.VUL_1      hIIvGIGGSATNDGGiGMaQALGiKLlDAqGNalGyGGGeLakLatIDcS   177
V.VUL_2      kImlGlGGSATNDGGAGiVQALGgKLlDreGNElpkGGiaLadLdrIDlS   200
Consensus    hiilgiggsatndggagmaqalg kllda gn lg ggg la la id s

GARK         GLDPRLKDCVIRVACDVTNPLVGDNGASRIFGPQKGASEAMIVELDNNLS   227
GLXK         ecDPRLanChIeVACDVdNPLVGarGAaavFGPQKGAtpeMveELeqgLq   227
S.CHOL_GARK  eLDPRLKlCaIRaACDVsNPLiGDNGASRIFGPQKGAtEenIVELDrNLa   227
S.CHOL_GLXK  GcDPRiseCrIeVACDVdNPLVGprGAaavFGPQKGAtpeMvetLeNgLr   227
V.VUL_1      qLDPRLaqvrleVACDVdNPLcGtkGASavFGPQKGAtpeMvtiLDkNLa   227
V.VUL_2      hahPRchqvefiVACDVTNPiiGvNGASevFGPQKGASprlvaELdrsLd   250
Consensus     ldprl  c ievacdvdnplvg ngasavfgpqkgatpemv eld nl

GARK         HYAEVIKKALHVDVKDVPGAGAAGGMGAALMAFLGAELKSGIEIVTTALN   277
GLXK         nYArVlqqqteinVcqmaGgGAAGGMGiAaavFLnAdiKpGIEIVlnAvN   277
S.CHOL_GARK  HYAdiIKKsLnVDVKaaPGAGAAGGMGAALMAFLGAELrSGIEIVTaALN   277
S.CHOL_GLXK  nYArVlhaltgrDmsqiPGgGAAGGMGiAaivFLeAEmKpGIEIVmqAvk   277
V.VUL_1      HYAtiIKqqLdVDVrDmaGAGAAGGMGAALlglLnAELrpGIEIVmdAvh   277
V.VUL_2      rfAkavfsitgVDhrlsaGfGAAGGtpleLsllfdiqitpGIEmVldAALd   300
Consensus    hya vik  l vdv  agagaaggmgaal  fl aelkpgieiv  aln

GARK         LEEHIHDCTLVITGEGRIDSQSIHGKVPIGVANVAKKYHKPVIGIAGSLT   327
GLXK         LaqavqgaaLVITGEGRIDSQtagGKaPlGVAsVAKqfnvPVIGIAGvLg   327
S.CHOL_GARK  LEEHIHDCTLVvTGEGRIDSQSIrGKVPIGVANVAKKYHKPVIGIAGnLT   327
S.CHOL_GLXK  LEEavkeasLVITGEGRIDSQtagGKaPIGVAsVAKrhHvPVIGIAGvLg   327
V.VUL_1      LdEivaDadLVITGEGRIDSQtIHGKtPIGVArtAKKHglPVIGIAGcLs   327
V.VUL_2      adkvlehadLVITGEGqmDnQtlqGKtPfGiANrAKKksiPVIGIAGSLg   350
Consensus    lee v da lvitgegridsqti gk pigvanvakk h pvigiag lg

GARK         DDVGVVHQHGIDAVFSVLTSIGTLDEAFRGAYDNICRASRNIAATLAIGM   377
GLXK         DgVeVVHQyGIDAVFSiLprlapLaEvlasgetNlfnsaRNIAcaikIGq   377
S.CHOL_GARK  hDVGiVHhyGIDAVFSVLTrIvTLeEAFRGAfDNIyRASRNvAAaLAIGM   377
S.CHOL_GLXK  DgVeVVHrHGIDAVFSiLprlapLpEvlangeqNlyhsacNIArviklGq   377
V.VUL_1      aDcGVVHeHGlDAVFaVvnrsvdLptAlaeAaeNveltaRNvAAafcmtr   377
V.VUL_2      sDieglysk.IDcaFdtvsSpqsLDqAlkqAskNvaqtaRNIAATLklGa   399
Consensus    ddvevvh hgidavfsvl r   l eala a  n    arniaaalkig

GARK         RNAG.                                                381
GLXK         gikn.                                                381
S.CHOL_GARK  RsAG.                                                381
S.CHOL_GLXK  digtr                                                382
V.VUL_1      R....                                                378
```

# FIGURE 5

153

```
FILE: Multiple_Sequence_Alignment Hexokinase
PROJECT:
NUMBER: 4
MAXLENGTH: 932
NAMES: SP|P19367|HXK1_H SP|P52789|HXK2_H SP|P52790|HXK3_H SP|P35557|HXK4_H
MAXNAMELEN: 16
FEATURES
ORIGIN
SP|P19367|HXK1_H .........MIAAQLLAYYFTELK..DDQVKKIDKYLYAMRLSDETLIDI         39
SP|P52789|HXK2_H .........MIAshLLAYfFTELn..hDQVqKvDqYLYhMRLSDETLleI         39
SP|P52790|HXK3_H mdsigssglrqgeetLscseegLpgpsDsselvqecLqqfkvtraqLqqI         50
SP|P35557|HXK4_H .................................................          0
Consensus              mia  llay ftel   dqv kvd yly mrlsdetl i


SP|P19367|HXK1_H MTRFRKEMKNGLSRDFNPTATVKMLPTFVRSIPDGSEKGDFIALDLG..G         87
SP|P52789|HXK2_H skRFRKEMekGLgatthPTAaVKMLPTFVRStPDGtEhGeFlALDLG..G         87
SP|P52790|HXK3_H qasllgsMeqaLrgqasPapaVrMLPTyVgStPhGtEqGDFvvLeLGatG        100
SP|P35557|HXK4_H .................................................          0
Consensus              rfrkeme gl    ptaavkmlptfvrstpdgte gdf aldlg  g


SP|P19367|HXK1_H SSFRILRVQVNHEKNQNVHMESEVYDTPENIVHGSGSQLFDHVAECLGDF        137
SP|P52789|HXK2_H tnFRvLwVkVtdnglQkVeMEnqiYaiPEdImrGSGtQLFDHiAECLanF        137
SP|P52790|HXK3_H aSlRvLwVtltgieghrVeprSqefviPqevmlGaGqQLFDfaAhCLseF        150
SP|P35557|HXK4_H .................................................          0
Consensus              sfrvlwv vt   q vemesq y ipe im gsg qlfdh aecl  f


SP|P19367|HXK1_H MEKRKIKDKKLPVGFTFSFPCQQSKIDEAILITWTKRFKASGVEGADVVK        187
SP|P52789|HXK2_H MdKlqIKDKKLPlGFTFSFPChQtKlDEsfLvsWTKgFKsSGVEGrDVVa        187
SP|P52790|HXK3_H ldaqpvnkqgLqlGFsFSFPChQtglDrstLIsWTKgFrcSGVEGqDVVq        200
SP|P35557|HXK4_H .................................................          0
Consensus              mdk  ikdkklplgftfsfpchqtkldes liswtkgfk sgveg dvv


SP|P19367|HXK1_H LLNKAIKKRGDYDANIVAVVNDTVGTMMTCGYDDQHCEVGLIIGTGTNAC        237
SP|P52789|HXK2_H LirKAIqrRGDfDidIVAVVNDTVGTMMTCGYDDhnCEiGLIvGTGsNAC        237
SP|P52790|HXK3_H LLrdAIrrqGaYnidvVAVVNDTVGTMMgCepgvrpCEVGLvvdTGTNAC        250
SP|P35557|HXK4_H .................................................          0
Consensus              llrkai rrgdydidivavvndtvgtmmtcgydd cevglivgtgtnac


SP|P19367|HXK1_H YMEELRHIDLVEGDEGRMCINTEWGAFGDDGSLEDIRTEFDREIDRGSLN        287
SP|P52789|HXK2_H YMEEmRHIDmVEGDEGRMCINmEWGAFGDDGSLnDIRTEFDqEIDmGSLN        287
SP|P52790|HXK3_H YMEEaRHvavldeDrGRvCvsvEWGsFsDDGaLgpvlTtFDhtlDheSLN        300
SP|P35557|HXK4_H .................................................          0
Consensus              ymee rhid vegdegrmcin ewgafgddgsl dirtefd eid gsln


SP|P19367|HXK1_H PGKQLFEKMVSGMYLGELVRLILVKMAKEGLLFEGRITPELLTRGKFNTS        337
SP|P52789|HXK2_H PGKQLFEKMiSGMYmGELVRLILVKMAKEeLLFgGklsPELLNtGrFeTk        337
SP|P52790|HXK3_H PGaQrFEKMigGlYLGELVRLvLahlArcGvLFgGctsPaLLsqGsille        350
SP|P35557|HXK4_H .................................................          0
Consensus              pgkqlfekmisgmylgelvrlilvkmakegllfgg  spell  g f t


SP|P19367|HXK1_H DVSAIEKNKEGLHNAKEILTRLGVEPSDDDCVSVQHVCTIVSFRSANLVA        387
SP|P52789|HXK2_H DiSdIEgeKdGirkArEvLmRLGldPtqeDCVathriCqIVStRSAsLcA        387
SP|P52790|HXK3_H hVaemEdpstGaarvhaILqdLGlsPgasDvelVQHVCaaVctRaAqLcA        400
SP|P35557|HXK4_H .................................................          0
Consensus              dvs ie  k g   a eil rlgl p  dcv vqhvc ivstrsa lca


SP|P19367|HXK1_H ATLGAILNRLRDNKGTPRLRTTVGVDGSLYKTHPQYSRRFHKTLRRLVPD        437
SP|P52789|HXK2_H ATLaAvLqRikeNKGeeRLRsTiGVDGSvYKkHPhfakRlHKTvRRLVPg        437
SP|P52790|HXK3_H AaLaAvLscLqhsreqqtLqvaVatgGrvcerHPrfcsvlqgTvmlLaPe        450
SP|P35557|HXK4_H .................................................          0
Consensus              atlaavl rl  nkg  rlr tvgvdgsvyk hp f  rlhktvrrlvp


SP|P19367|HXK1_H SDVRFLLSESGSGKGAAMVTAVAYRLAEQHRQIEETLAHFHLTKDMLLEV        487
SP|P52789|HXK2_H cDVRFLrSEdGSGKGAAMVTAVAYRLAdQHRarqkTLeHlqLshDqLLEV        487
SP|P52790|HXK3_H cDVslipSvdGgGrGvAMVTAVAaRLAahrRllEETLApFrLnhDqLaaV        500
SP|P35557|HXK4_H ................MlddrArmeAakkekvEqiLAeFqLqeedLkkV         33
Consensus              cdvrfl sedgsgkgaamvtavayrlaaqhr  eetlahfql hdqllev


SP|P19367|HXK1_H KKRMRAEMELGLRKQTHNNAVVKMLPSFVRRTPDGTENGDFLALDLGGTN        537
SP|P52789|HXK2_H KrRMkvEMErGLsKeTHasApVKMLPtyVcaTPDGtEkGDFLALDLGGTN        537
SP|P52790|HXK3_H qaqMRkaMakGLRgeass...lrMLPtFVRaTPDGsErGDFLALDLGGTN        547
SP|P35557|HXK4_H mrRMqkEMdrGLRleTHeeAsVKMLPtyVRsTPeGsEvGDFLsLDLGGTN         83
Consensus              krrmrkemerglrketh  a vkmlptfvratpdgse gdflaldlggtn


SP|P19367|HXK1_H FRVLLVKIRSGKKR..TVEMHNKIYAIPIEIMQGTGEELFDHIVSCISDF        585
SP|P52789|HXK2_H FRVLLVrvRnGKwg..gVEMHNKIYAIPqEvMhGTGdELFDHIVqCIaDF        585
SP|P52790|HXK3_H FRVLLVrvttG......VqitseIYsIPetvaQGsGqqLFDHIVdCIvDF        591
SP|P35557|HXK4_H FRVmLVKvgeGeegqwsVktkhqmYsIPedaMtGTaEmLFDyIseCISDF        133
Consensus              frvllvkvr gk g   vemhnkiyaipeevmqgtgeelfdhiv cisdf
```

```
SP|P19367|HXK1_H   LDYMGIKGPRMPLGFTFSFPCQQTSLDAGILITWTKGFKATDCVGHDVVT    635
SP|P52789|HXK2_H   LeYMGmKGvslPLGFTFSFPCQQnSLDesILlkWTKGFKAsgCeGeDVVT    635
SP|P52790|HXK3_H   qqkqGlsGqslPLGFTFSFPCrQlgLDqGILlnWTKGFKAsDCeGqDVVs    641
SP|P35557|HXK4_H   LDkhqmKhkklPLGFTFSFPvrhediDkGILlnWTKGFKAsgaeGnnVVg    183
Consensus             ldkmgmkg slplgftfsfpcqq sld gillnwtkgfkasdceg dvvt

SP|P19367|HXK1_H   LLRDAIKRREEFDLDVVAVVNDTVGTMMTCAYEEPTCEVGLIVGTGSNAC    685
SP|P52789|HXK2_H   LLkeAIhRREEFDLDVVAVVNDTVGTMMTCgfEdPhCEVGLIVGTGSNAC    685
SP|P52790|HXK3_H   LLReAItRRqaveLnVVAiVNDTVGTMMsCgYEdPrCEiGLIVGTGtNAC    691
SP|P35557|HXK4_H   LLRDAIKRRgdFemDVVAmVNDTVaTMisCyYEdhqCEVGmIVGTGcNAC    233
Consensus             llrdaikrreefdldvvavvndtvgtmmscgyedp cevglivgtgsnac

SP|P19367|HXK1_H   YMEEMKNVEMVEGDQGQMCINMEWGAFGDNGCLDDIRTHYDRLVDEYSLN    735
SP|P52789|HXK2_H   YMEEMrNVElVEGeeGrMCvNMEWGAFGDNGCLDDfRTefDvaVDElSLN    735
SP|P52790|HXK3_H   YMEElrNVagVpGDsGrMCINMEWGAFGDdGsLamlsTrfDasVDqaSiN    741
SP|P35557|HXK4_H   YMEEMqNVElVEGDeGrMCvNtEWGAFGDsGeLDeflleYDRLVDEsSaN    283
Consensus             ymeemrnvelvegdegrmcinmewgafgdngclddfrtefdrlvde sln

SP|P19367|HXK1_H   AGKQRYEKMISGMYLGEIVRNILIDFTKKGFLFRGQISETLKTRGIFETK    785
SP|P52789|HXK2_H   pGKQRfEKMISGMYLGEIVRNILIDFTKrGlLFRGrISErLKTRGIFETK    785
SP|P52790|HXK3_H   pGKQRfEKMISGMYLGEIVRhILlhlTslGvLFRGQqiqrLqTRdIFkTK    791
SP|P35557|HXK4_H   pGqQlYEKlIgGkYmGElVRlvLlrlvdenlLFhGeaSEqLrTRGaFETr    333
Consensus             pgkqrfekmisgmylgeivrnilidftk gllfrgqiserlktrgifetk

SP|P19367|HXK1_H   FLSQIESDRLALLQVRAILQQLGLNSTCDDSILVKTVCGVVSRRAAQLCG    835
SP|P52789|HXK2_H   FLSQIESDcLALLQVRAILQhLGLeSTCDDSIiVKeVCtVVaRRAAQLCG    835
SP|P52790|HXK3_H   FLSeIESDsLALrQVRAILedLGLplTsDDalmVleVCqaVSqRAAQLCG    841
SP|P35557|HXK4_H   FvSQvESDtgdrkQiynILstLGLrpsttDcdiVrraCesVStRAAhmCs    383
Consensus             flsqiesd lallqvrailq lgl stcddsiivkevc vvsrraaqlcg

SP|P19367|HXK1_H   AGMAAVVDKIRENRGLDRLNVTVGVDGTLYKLHPHFSRIMHQTVKELSPK    885
SP|P52789|HXK2_H   AGMAAVVDrIRENRGLDaLkVTVGVDGTLYKLHPHFakvMHeTVKdLaPK    885
SP|P52790|HXK3_H   AGvAAVVeKIRENRGLeeLaVsVGVDGTLYKLHPrFSslvaaTVrELaPr    891
SP|P35557|HXK4_H   AGlAgVinrmREsRseDvmriTVGVDGsvYKLHPsFkerfHasVrrLtPs    433
Consensus             agmaavvdkirenrgld l vtvgvdgtlyklhphfs  mhatvkelapk

SP|P19367|HXK1_H   CNVSFLLSEDGSGKGAALITAVGVRLRTEASS    917
SP|P52789|HXK2_H   CdVSFLqSEDGSGKGAALITAVacRiReaqqr    917
SP|P52790|HXK3_H   CvVtFLqSEDGSGKGAALvTAVacRLaqltrv    923
SP|P35557|HXK4_H   CeitFieSEeGSGrGAALvsAVackkacmlgq    465
Consensus             c vsflqsedgsgkgaalitavacrla
```

# FIGURE 6

```
FILE: Multiple_Sequence_Alignment Aspartokinase
PROJECT:
NUMBER: 3
MAXLENGTH: 835
NAMES: ASK1_ECOLI ASK2_ECOLI ASK3_ECOLI
MAXNAMELEN: 10
FEATURES
ORIGIN
ASK1_ECOLI ...........MRVLKFGGTSVANAERFLRVADILESNARQGQVATVLSA      39
ASK2_ECOLI msviaqagakgrqlhKFGGSsSlAdvkcyLRVAgImaeysqp.ddmmVvSA      49
ASK3_ECOLI ........mseivVsKFGGTSVAdfdamnRsADIvlSdAnv..rlvVLSA      40
Consensus          v kfggtsvad   lrvadi s a     vlsa

ASK1_ECOLI PAKITNHLVAMIEKTISGQDALPNISDAER.IFAELLTGLAAAQPGFPLA      88
ASK2_ECOLI agstTNqLinwlklsqtdrlsahqvqqtlRryqcdLisGLlpAeead..s      97
ASK3_ECOLI sAgITNlLVAlaEglepGe..rfeklDAiRnIqfaiLerLrypnvir..e      86
Consensus   a itn lva  e  g      da r iq  ll gl  a

ASK1_ECOLI QLKTFVDQEFAQIKHVLHGISLLGQCPDSINAALICRGEKMSIAIMAGVL     138
ASK2_ECOLI lisaFVs.dlerlaalLd.....sginDavyAevvghGEvwSarlMsaVL     141
ASK3_ECOLI eierlle.nitvlaeaaa.....latspaltdeLvshGElMStllfveiL     130
Consensus   i  fv     la  l        da  aelv hge ms  lm  vl

ASK1_ECOLI EARGHNVTVIDPVEKLLAVGHYLESTVDIAESTRRIAASRIP..ADHMVL     186
ASK2_ECOLI nqqGlpaawlD.arefLraeraaqpqVDeglSypllqqllvqh.pgkrlv     189
ASK3_ECOLI reRdvqaqwfDvrkvmrtndrfgraepDIAalaelaAlqllPrlneglVi     180
Consensus     rg  a w d     l   r     vdia s  la  l p      v

ASK1_ECOLI MAGFTAGNEKGELVVLGRNGSDYSAAVLAACLRADCCEIWTDVDGVYTCD     236
ASK2_ECOLI vtGFisrNnaGEtVlLGRNGSDYSAtqigAlagvsrvtIWsDVaGVYsaD     239
ASK3_ECOLI tqGFigsenKGrtttLGRgGSDYtAAlLAeaLhAsrvdIWTDVpGiYTtD     230
Consensus    gfi nnkgetv lgrngsdysaa laa l asrv iwtdv gvyt d

ASK1_ECOLI PRQVPDARLLKSMSYQEAMELSYFGAKVLHPRTITPIAQFQIPCLIKNTG     286
ASK2_ECOLI PRkVkDAcLLpllrldEAsELarlaAgvPVLHaRTlqPvsgseIdlqlrcsy     289
ASK3_ECOLI PRvVsaAkrideiafaEAaEmatFGAKVLHPaTllPavrsdIPvfvgssk     280
Consensus  pr v da ll      ea ela fgakvlhprtl p   s ip      s

ASK1_ECOLI NPQAPGTLIGASRDEDELPVKGISNLNNMAMFSVSGPGMKGMVGMAARVF     336
ASK2_ECOLI tPdqgsTrIervlasg.tgarivtshddvcliefqvPasqdfklahkeid     338
ASK3_ECOLI dPrAgGTLvcnktenp.plfralalrrNqtlltlhslnMlhsrGflAeVF     329
Consensus    p aggtli           r     n  l    p m   g  aevf

ASK1_ECOLI AAMSRARISVVLITQSSSEYSISFCVPQSDCVRAERAMQEEFYLELKEGL     386
ASK2_ECOLI qilkRAqvrplavgvhndrqllqFCyts.......evadsalkildeaGL     381
ASK3_ECOLI gilaRhnISVdLITtSevsvaltldttg...ststgdtlltqsLlmelsa     376
Consensus    il ra isv lit s     l fc t         ll e gl

ASK1_ECOLI LEPLAVTERLAIISVVGDGMRTLRGISAKFFAALARANINIVAIAQGSSE     436
ASK2_ECOLI pgeLrlrqgLAlvamVGaGvtrnplhchrFwqqLkgqpveftwqsddg..     429
ASK3_ECOLI LcrveVeEgLAlvaliGndlskacGvgkevFgvLepfNIrmicygasS..     424
Consensus  l  l v eglalva vg g    g   ff  l  ni         r

ASK1_ECOLI RSISVVVNNDDATTGVRVTHQMLFNTDQVIEVFVIGVGGVGGALLEQLKR     486
ASK2_ECOLI iSlvaVlrtgptesliqglHQsvFraekrIglvlfGkGniGsrwLElfaR     479
ASK3_ECOLI hnlcflVpgeDAeqvVqklHsnLFe.......................     449
Consensus   sl  vv  dae  vq lhq lf     i     g  g  le   r

ASK1_ECOLI QQSWLKNKH.IDLRVCGVANSKALLTNVHGLNLENWQEELAQAKEPFNLG     535
ASK2_ECOLI eQStLsartgfefvlaGVvdSrrsLlsydGLdasralaffndeaveqdee     529
ASK3_ECOLI ................................................     449
Consensus  qs l         gv  s   l    gl

ASK1_ECOLI RLIRLVKEYHLLNPVIVDCTSSQAVADQYADFLREGFHVVTPNKKANTSS     585
ASK2_ECOLI sLflwmrahpyddlVvlDvTaSQqlADQYlDFashGFHVisaNKlAgaSd     579
ASK3_ECOLI ................................................     449
Consensus   l          v d tsq adqy df  gfhv  nk a  s

ASK1_ECOLI MDYYHQLRYAAEKSRRKFLYDTNVGAGLPVIENLQNLLNAGDELMKFSGI     635
ASK2_ECOLI snkYrQihdAfEKtgRhwLYnatVGAGLPinhtvrdLidsGDtilsiSGI     629
ASK3_ECOLI ................................................     449
Consensus    y q  a ek r ly  vgaglp    l  gd    sgi

ASK1_ECOLI LSGSLSYIFGKLDEGMSFSEATTLAREMGYTEPDPRDDLSGMDVARKLLI     685
ASK2_ECOLI fSGtLSwlFlqfDgsvpFtElvdqAwqqGlTEPDPRDDLSGkDVmRKLvI     679
ASK3_ECOLI ................................................     449
Consensus   sg ls f  d   f e  a  g tepdprddlsg dv rkl i

ASK1_ECOLI LARETGRELELADIEIEPVLPAEFNAEGDVAAFMANLSQLDDLFAARVAK     735
ASK2_ECOLI LAREaGyniEpdqvrvEslvPAhce.gGsidhFfeNgdeLneqmvqRlea     728
ASK3_ECOLI ................................................     449
Consensus  lare g  e    e  pa   g   f  n  l       r
```

```
ASK1_ECOLI  ARDEGKVLRYVGNIDEDGVCRVKIAEVDGNDPLFKVKNGENALAFYSHYY    785
ASK2_ECOLI  ARemGlVLRYVarfDanGkaRVgveaVredhPLasllpcdNvfAieSrwY    778
ASK3_ECOLI  ..................................................    449
Consensus   ar  g vlryv   d  g  rv    v    pl      n  a  s  y

ASK1_ECOLI  QPLPLVLRGYGAGNDVTAAGVFADLLRTLSWKLGV                  820
ASK2_ECOLI  rdnPLViRGpGAGrDVTAgaiqsDinRlaqll...                  810
ASK3_ECOLI  ...................................                  449
Consensus      plv rg gag dvta      d  r
```

# FIGURE 7

```
FILE: Multiple_Sequence_Alignment Glutamine synthetase
PROJECT:
NUMBER: 23
MAXLENGTH: 570
NAMES: SALTY THIFE ECOLI ARCFU SEQUENCE BACCE
      BACSU HALVO LACDE P. GLNA_YEAST GLN1_CHLRE
      GLN1_MAIZE GLN1_ORYSA GLNA_LUPLU GLN1_PEA GLN1_DROME GLNA_SQUAC
      GLNA_XENLA GLNA_CHICK MOUSE HAMPSTR GLNA_HUMAN
MAXNAMELEN: 10
FEATURES
ORIGIN
SALTY       ...........................................SAEHV         5
THIFE       ........................................mgySpsdV         8
ECOLI       ...........................................SAEHV         5
ARCFU       ................................mvrrlrgdcmeevEra        16
SEQUENCE    ........................................msks            4
BACCE       ........................................arytkEdi        8
BACSU       ........................................akytrEdi        8
HALVO       .....................mtednaltdgglsdeAqaV               19
LACDE       ..............................mskviteEei               10
P.          ...............................................         0
GLNA_YEAST  ...................................aeasiektqil         11
GLN1_CHLRE  ....................maagsvgvfatdekigslldqSitrh         26
GLN1_MAIZE  ......................................maSltdl          7
GLN1_ORYSA  ......................................manltdl          7
GLNA_LUPLU  ......................................msvlsdl          7
GLN1_PEA    ......................................msSlsdl          7
GLN1_DROME  malrvaglflkkelvapatqqlrllrtgnttrsqflanspntaldksilq    50
GLNA_SQUAC  ......mricrsflflvkkcgnitptiwrnqhtykmatsasanlskivkk    44
GLNA_XENLA  ...............................msvshssrlnkgvre         15
GLNA_CHICK  ..............................matsasshlskAikh         15
MOUSE       ..............................matsasshvnkgikq         15
HAMPSTR     ..............................matsasshlnkgikq         15
GLNA_HUMAN  ..............................mttsasshlnkgikq         15
Consensus

SALTY       LTMLNEHEVKFVDLRFTDTKGKEQHVTIPA.HQVNA.EFFEEGK...MFD    50
THIFE       vkliqEkdiKFiDfRFTDTKGKEQHVsvPg.Hviee.dtFtEGK...aFD    53
ECOLI       LTMLNEHEVKFVDLRFTDTKGKEQHVTIPA.HQVNA.EFFEEGK...MFD    50
ARCFU       kavLkEnnVrqVlcaFaDvrGylQmfsIPA.refvdgsaFEnGi...gFD    62
SEQUENCE    LqlikEHdVKwiDLRFTDTKGKQHVTmPA.rdVdd.dFFEyGK...MFD    49
BACCE       frlakEenVKyirLqFTDllGviknVeIPv.sQltk..aldnkm...MFD    52
BACSU       eklvkEenVKyirLqFTDilGtiknVeIPv.sQlgk..aldnkv...MFD    52
HALVO       ideieEknVdFlrLqFTDilGtvknVsIPA.sQaek..aFtEGi...yFD    63
LACDE       rkdveEknVrFlrLaFTDinGtlknlevPv.sQldd..vlgnqt...rFD    54
P.          ............mcdikrynGfd.yykcPr.tilkktceFvkne......    30
GLNA_YEAST  qkyLeldqrgriiaeyvwidGtg.nlrskg.rtlkkritsidqlpewnFD    59
GLN1_CHLRE  flstvtdqqgkicaeyvwigGsmhdVrsks.rtlstiptkpEdlphwnyD    75
GLN1_MAIZE  vnldlsdctdriiaeyiwigGtgidlrskA.rtVkgpitdpiqlpkwnyD    56
GLN1_ORYSA  vnlnlsdcsdkiiaeyiwvgGsgidlrskA.rtVkgpitdvsqlpkwnyD    56
GLNA_LUPLU  inlnlsdttekiiaeyiwvgGsgvdlrskA.rtlsgpvndpsklpkwnyD    56
GLN1_PEA    infnlsdstekiiaeyiwvgGsgidirskA.rtlpgpvsdpaklpkwnyD    56
GLN1_DROME  ryrnlEtpanrVqatylwidGtgenirlkd.rvldkvpssvEdlpdwqyD    99
GLNA_SQUAC  nyMelpq.dgkVqamyiwidGtgeaVrckt.rtldnepksiaelpewnFD    92
GLNA_XENLA  qyMklpq.gekVqvtyvwidGtgegVrckt.rtldqepktideipewnFD    63
GLNA_CHICK  myMklpq.gekVqamyiwidGtgeHlrckt.rtldhepkslEdlpewnFD    63
MOUSE       myMslpq.gekiqamyiwvdGtgeplrcktcrtldcepkcvEelpewnFD    64
HAMPSTR     myMslpq.gekVqamyiwvdGtgeglrckt.rtldcepkcvEelpewnFD    63
GLNA_HUMAN  vyMslpq.gekVqamyiwidGtgeglrckt.rtldsepkcvEelpewnFD    63
Consensus              a  y  w  gtg   r  k  rtl         lp wnfd

SALTY       GSSIGGWKGINESDMVLMPDASTAVIDPFF...ADSTLIIRCDILEPG..    95
THIFE       GSSIaGWKGINESDMiLlPDpdsAVlDPFm...detTLllRCDviEPa..    98
ECOLI       GSSIGGWKGINESDMVLMPDASTAVIDPFF...ADSTLIIRCDILEPG..    95
ARCFU       GSSvrGfrtIekSDMVwMPDASslkIiPwiddpiqksaImfgnvyEaw..   110
SEQUENCE    GSSIaGWKGIeaSDMiLMPDdSTAVlDPFt...eepTLIIvCDIiEPs..    94
BACCE       GSSIeGfvrIeESDMyLyPDldTwVIfPwt.aekgkvarliCDIyna...    98
BACSU       GSSIeGfvrIeESDMyLyPDlnTfVIfPwt.aekgkvarfiCDIynP...    98
HALVO       GSSIdGfvrIqESDMrLePDpSTfavlPwrkkensaagrliCDvfnts..   111
LACDE       GSSIdGfvrleESDMVLyPDlaTwlvlawttveegtigrlvCsvhnv...   101
P.          GiadkvciGneleffifdk..vnyslDeyn.........tylkvydr...    66
GLNA_YEAST  GSStnqapGhd.SDiyLkP..vayypDPFrr...gdnivvlaacynn...   100
GLN1_CHLRE  GSStGqapGhd.SevyLiP..rsifkDPFrg...gdnilvmCDcyEPpkv   119
GLN1_MAIZE  GSStGqapGed.SevilyP..qaifkDPFrk...gnhilvmCDcytP...    97
GLN1_ORYSA  GSStGqapGed.SevilyP..qaifkDPFrr...gnhilvmCDcytP...    97
GLNA_LUPLU  GSStGqapGkd.SevilwP..qaifkDPFrr...gnnilvmCDtytP...    97
GLN1_PEA    GSStnqapGkd.SevilyP..qaifkDPFrr...gnnilviCDvytP...    97
GLN1_DROME  GSStyqahGeN.SDttLkP..raiyrDPFkpg..kndvIvlCDtysa...   141
GLNA_SQUAC  GSStyqseGsN.SDMyLvP..SamfrDPFrr...DpnklvlCevLky...   133
GLNA_XENLA  GSSthqaeGsN.SDMyLiP..vqmfrDPFcl...DpnklvmCevLky...   104
GLNA_CHICK  GSStfqaeGsN.SDMyLrP..aamfrDPFrk...DpnklvlCevfky...   104
MOUSE       GSStfqseGsN.SnMyLhP..vamfrDPFr......nklvlCevfky...   102
HAMPSTR     GSStfqsessN.SDMyLsP..vamfrDPFrk...epnklvfCevfky...   104
```

```
GLNA_HUMAN  GSStlqseGsN.SDMyLvP..aamfrDPFrk...DpnklvlCevfky...      104
Consensus   gsst q  g   sdm l p        dpfr         lv cdv

SALTY       .......TLQGYDRDPRSIAKRAEDYLRATGIADTVLFGPEPEFFLFDDI      138
THIFE       .......TgQGYeRDPRSvAKRAEiYLksTGIADTsfFG..PEFFvFDsv      139
ECOLI       .......TLQGYDRDPRSIAKRAEDYLRsTGIADTVLFGPEPEFFLFDDI      138
ARCFU       .......gteiaDcDPRgyvakryedmlksegm.saiFGPEiEFFLFegv      152
SEQUENCE    .......TmQGYDRDPRaIArRAEeYLksTGIgDTafFGPEPEFFiFDev      137
BACCE       .......dgtpfegDPRnnlKRvlkemeAlGfs.dfnlGPEPEFFLFk..      138
BACSU       .......dgtpfegDPRnnlKRilkemedlGfs.dfnlGPEPEFFLFk..      138
HALVO       .......TgepfsgDPRgvlKRAiep.eelGy..dVnvaPEPEFFLFe..      149
LACDE       .......dgtpfegDPRnnlKkviaemeemGfs.dfeiGfEaEFFLFk..      141
P.          .......esfscknDlsSIygnhvvn..kvephkdhfnnPnnEylinD..      105
GLNA_YEAST  .......dgtpnkfnhRheAaklfaa..hkdee..iwFGlEqEytLFDmy      139
GLN1_CHLRE  npdgtlaapkpiptntRfacaevmek..Akkee..pwFGiEqEytLlnaI      165
GLN1_MAIZE  .......qgepiptnkRysAakvfshpdvaaev..pwyGiEqEytLlqkd      138
GLN1_ORYSA  .......qgepiptnkRhsAakifshpdvvaev..pwyGiEqEytLlqkd      138
GLNA_LUPLU  .......agepiptnkRhaAakifshpdvvaee..pwFGiEqEytLlqkd      138
GLN1_PEA    .......ageplptnkRynAakifshpdvaaev..pwyGiEqEytLlqkd      138
GLN1_DROME  .......dgkptasnkRaafqaAiDl..isdqe..pwFGiEqEytLlrr.      179
GLNA_SQUAC  .......nrkpaesnlRhscqkimsm..ianey..pwFGmEqEytLlgt.      171
GLNA_XENLA  .......nrksaetnlRhtcKkimem..vndhr..pwFGmEqEytLlgi.      142
GLNA_CHICK  .......nrQsaDtnlRhtcrRimDm..vsnqh..pwFGmEqEytLlgt.      142
MOUSE       .......nrkpaetnlRhIcKRimDm..vsnqh..pwFGmEqEytLmgt.      140
HAMPSTR     .......nqkpaetnlRhtcKRimDm..vsnqh..pwFGmEqEytLlgt.      142
GLNA_HUMAN  .......nrrpaetnlRhtcKRimDm..vsnqh..pwFGmEqEytLmgt.      142
Consensus                 p    n r            pwfg eqeytl

SALTY       RFG.......ASISGSHVAIDDIEGAWNSSTKYEGGNKGHRPGVKGGYFP      181
THIFE       twn.......idmSGcaykvDaeEaAWNSgkeYEsGNmGHRlGVKGGYFP      182
ECOLI       RFG.......sSISGSHVAIDDIEGAWNSSTqYEGGNKGHRPaVKGGYFP      181
ARCFU       dFtrlswdmwvSpnGgagdswgpprimpiSsel...esGymirpKeGYFr      199
SEQUENCE    kyk.......sdISGSmfkIfseqaAWNtdadfEGGNKGHRPGVKGGYFP      180
BACCE       ..........vdekG.....................NptlelndnGGYFd      157
BACSU       ..........ldekG.....................eptlelndKGGYFd      157
HALVO       ..........ededG.....................rattvtndaGGYFd      168
LACDE       ..........egknG.....................eettkvsdhssYFd      160
P.          ...................................dskkvkkKsGYFt      118
GLNA_YEAST  ...........ddv...................yGwpKGgyPapqGpYyc      159
GLN1_CHLRE  ...........tkwp..................lGwpKGgyPapqGpYyc      186
GLN1_MAIZE  ...........vswp..................lGwpvGgyPGpqGpYyc      159
GLN1_ORYSA  ...........vnwp..................lGwvGgfPGpqGpYyc      159
GLNA_LUPLU  ...........ihwp..................iGwplGgfPGpqGpYyc      159
GLN1_PEA    ...........inwp..................lGwpiGgyPGkqGpYyc      159
GLN1_DROME  ...........grts..................fGwpengfPapqGpYyc      200
GLNA_SQUAC  ...........dghp..................fGwpsncfPGpqGpYyc      192
GLNA_XENLA  ...........nghp..................yGwpengfPGpqGpYyc      163
GLNA_CHICK  ...........dghp..................fGwpsncfPGpqGpYyc      163
MOUSE       ...........dghp..................fGwpfngfPGpqGpYyc      161
HAMPSTR     ...........dghp..................fGwpsdgfPGpqGlYyc      163
GLNA_HUMAN  ...........dghp..................fGwpsngfPGpqGpYyc      163
Consensus                                      gwp   pgpqgpyyc

SALTY       VPPVD..SAQDIRSEMCLVMEQMGLVVEAHHHEVATAGQNEVATRFNTMT      229
THIFE       VPPVD..SAQDlRSaMCLaMEeMGLkVEvHHHEVATAGQhEigvgFNTlT      230
ECOLI       VPPVD..SAQDIRSEMCLVMEQMGLVVEAHHHEVATAGQNEVATRFNTMT      229
ARCFU       pPPeD..ttveyRnElvyylEQlGidiEyHHHEVATAGQvEldfkpkqlv      247
SEQUENCE    VPPVD..hdheIRtaMCnalEeMGLkVEvHHHEVATAGQNEigvsFNTlv      228
BACCE       laPmD..lgencRrdivLelEeMGfeiEAsHHEVA.pGQhEidfkyanai      204
BACSU       laPtD..lgencRrdivLelEeMGfeiEAsHHEVA.pGQhEidfkyagav      204
HALVO       laPkD..lAsDvRrdiiyglEsMGfdiEAsHHEVA.eGQhEinftyddal      215
LACDE       maseD..egakcRrEivetlEklGfrVEAaHHEVg.dGQqEidfRFdnal      207
P.          tdPyD..tsniIklriCralndMninVqryHHEVs.tsQhEislkyfdal      165
GLNA_YEAST  gvgagkvyArDmieahyraclyaGLeisginaEVm.psQwEfqvgpcTgi      208
GLN1_CHLRE  sagagvaigrDvaevhyrlclaaGvnisgvnaEVl.psQwEyqvgpcegi      235
GLN1_MAIZE  aagaDkafgrDvvdahykaclyaGinisgingEVm.pGQwEfqvgpsvgi      208
GLN1_ORYSA  aagaekafgrDIvdahykaciyaGinisgingEVm.pGQwEfqvgpsvgi      208
GLNA_LUPLU  gtgaekafgrDIvdshykaclyaGinisgingEVm.pGQwEfqvgpsvgi      208
GLN1_PEA    gigaDkaygrDIvdahykaclfaGinisgingEVm.pGQwEfqvgpsvgi      208
GLN1_DROME  gvgaDrvyArDlveahvvaclyaGidfagtnaEVm.paQwEfqigp.agi      248
GLNA_SQUAC  gvgaDkaygrDIveahyraclyaGielsgtnaEVm.AaQwEyqvgpcegi      241
GLNA_XENLA  gvgaDkvygrDvveshykaclyaGikicgtnaEVm.psQwEfqvgpcegi      212
GLNA_CHICK  gvgaDkaygrDIveahyraclyaGvkiggtnaEVm.paQwEfqvgpcegi      212
MOUSE       gvgaDkaygrDIveahyraclyaGvkitgtnaEVm.paQwEfqigpcegi      210
HAMPSTR     gvgaDkayrrDImeahyraclyaGvkitgtyaEVk.haQwEfqigpcegi      212
GLNA_HUMAN  gvgaDraygrDIveahyraclyaGvkiagtnaEVm.paQwEfqigpcegi      212
Consensus      gad   grdi  ahy acl ag   i g n ev  pgqwe q gp  gi
```

```
SALTY       KKADEIQIYKYVVHNVAHRFGKTATFMPKPMFGD.NGSGMHCHMSLAK..    276
THIFE       prrmrckIlKYVVHNVAavr.qTATFMPKPvvGD.NGSGMHvHqSLgK..    276
ECOLI       KKADEIQIYKYVVHNVAHRFGKTATFMPKPMFGD.NGSGMHCHMSLsK..    276
ARCFU       dvgDafylYKfaakNiAamhGlyATFMPKPlylD.NaSGMHtHqSLwKge    296
SEQUENCE    aKADEvQtlKYcVHNVAdayGKTvTFMPKPlyGD.NGSGMHvHMSiAK..    275
BACCE       rscDdIQtfKlVVktiArkhGlhATFMPKPlyGv.NGSGMHCnlSLfK..    251
BACSU       rscDdIQtfKlVVktiArkhGlhATFMPKPlFGv.NGSGMHCnlSLfK..    251
HALVO       stADnvrtfrsVVraiAaehdlhATFMPKPipri.NGSGMHtHiSLfK..    262
LACDE       atADklQtfKmVVktiArkyhlhAsFMaKPveGl.aGnGMHtnMSLlK..    254
P.          tnADfllItKqiikttvssFnrTATFMPKPlvnD.NGnGlHCniSLwKn.    213
GLNA_YEAST  dmgDqlwmarYflHrVAeeFGikisFhPKPlkGDwNGaGcHtnvStke.    256
GLN1_CHLRE  tmgDhmwmsrYimyrVcemFnvevsFdPKPipGDwNGSGgHtnyStka..   283
GLN1_MAIZE  sagDEIwvarYileritemaGivlsldPKPikGDwNGaGaHtnyStks..   256
GLN1_ORYSA  aaADqvwvarYilerVtevaGvvlsldPKPipGDwNGaGaHtnfStks..   256
GLNA_LUPLU  sagDElwvarYileriteiaGvvlsldPKPipGDwNGaGaHtnyStks..   256
GLN1_PEA    sagDEIwaarYileriteiaGvvvsFdPKPipGDwNGaGaHanfStks..   256
GLN1_DROME  KacDdlwvsrYilqriAeeyGvvvTFdPKPMeGqwNGaGrHtnfStke..   296
GLNA_SQUAC  qmgDhlwIsrfilHrVcedFGiiAsFdPKPipGnwNGaGcHtnfStka.    289
GLNA_XENLA  dmgDhlwmarfilHrVcedFGvvATldPKPMtGnwNGaGcHtnyStes..   260
GLNA_CHICK  emgDhlwIarfilHrVcedFGvivsFdPKPipGnwNGaGcHtnfStkn..   260
MOUSE       qmgDhlwIacfilHrVcedFGviATFdPKPipGnwdGaGcHtnfStka..   258
HAMPSTR     rmgDhlwvarfilHrVckdFGviATFdsKPipGnwNGaGcHtnfStkt..   260
GLNA_HUMAN  smgDhlwvarfilHrVcedFGviATFdPKPipGnwNGaGcHtnfStka..   260
Consensus      gd  w   r il rv e   g  atfdpkp  gdwngag htn stk

SALTY       ..NGTNLFS.GDKYAGLSEQALYYIGGVIKHAKAINALANP.TTNSYKRL    322
THIFE       ..dGkNiFa.GDlYgGLSEiALYYIGGiIKHAKAvNALtNP.sTNSYKRL    322
ECOLI       ..NGvNLFa.GDKYAGLSEQALYYIGGVIKHAKAINALANP.TTNSYKRL    322
ARCFU       pfsGeavFadpDdeymLSqkArYYIGGlleHAKAltALcaP.TvNSYKRL   345
SEQUENCE    ..dGkNtFa.GegYAGLSdtALYfIGGiIKHgKAlNgftNP.sTNSYKRL    321
BACCE       ..NGeNvFydqngdlqLSddArhfIaGilKHApAftAvANP.TvNSYKRL    298
BACSU       ..NGvNaFfdenadlqLSEtAkhfIaGivKHAtsftAvtNP.TvNSYKRL    298
HALVO       ..dGeNaFhdGDdefdLSdtAksfvaGildHApAItAsltr.rstptrRL    309
LACDE       ..dGkNaFydkDgqynLSttALtflnGileHArAItcvANP.TvNSYKRL    301
P.          ..NknifyhndpstffLSkesfYfmyGivKHAKAlqAfcN.aTmNSYKRL    260
GLNA_YEAST  .......mrqp.ggmkyiEQAiekls..krHAehIkly....gsdndmRL    292
GLN1_CHLRE  .......trtapdgwkviqehcakle..arHAvhIaAy....gegnerRL    320
GLN1_MAIZE  .......mrea.ggyevikaAidklG..krHkehIaAy....gegnerRL    292
GLN1_ORYSA  .......mrep.ggyevikkAidkla..lrHkehIaAy....gegnerRL    292
GLNA_LUPLU  .......mrnd.ggyevikkAieklG..krHnehIaAy....gegnerRL    292
GLN1_PEA    .......mren.ggyevikkAieklG..lrHkehIaAy....gegnerRL    292
GLN1_DROME  .......mrad.ggikaiEepiekls..krHerhIkAyypkegkdnerRL    336
GLNA_SQUAC  .......mrdd.gglkyiEdsieklG..krHqyhIrAydpkgggldnaraL   329
GLNA_XENLA  .......mrve.gglkhiEdsieklG..krHdyhIcvydprggkdnsrRL   300
GLNA_CHICK  .......mred.gglkhiEeAiekls..krHqyhIrAydpkgggldnarRL   300
MOUSE       .......mree.nglkciEeAidkls..krHqyhIhtydpkgggldnsrRL  298
HAMPSTR     .......mree.nglkhikeAiekls..krHryhIrAydpkgggldnarRL  300
GLNA_HUMAN  .......mree.nglkyiEeAiekls..krHqyhIrAydpkgggldnarRL  300
Consensus           mr  gg   i ai kl    rh  hi ay    g   n rrl

SALTY       VPGYEAPVMLAYSA..RNRSASIRIPVVASP..KARRIEVRFPDPAANPY    368
THIFE       VPhfEAPVlLAYSA..kNRSASIRIPyVnSP..KARRIEVRFPDstANPY    368
ECOLI       VPGYEAPVMLAYSA..RNRSASIRIPVVsSP..KARRIEVRFPDPAANPY    368
ARCFU       VPGfEAPiyicwSp..RNRSAlvRvPmyvkkp.sAiRvEyRgvDPscNPY    392
SEQUENCE    VPGfEAPvMLAYSA..RNRSASIRIPyVnSP..KARRIEaRFPDPsANPY    367
BACCE       VPGYEAPcyvAwSA..qNRSplvRIPasrg...istRvEVRsvDPAANPY    343
BACSU       VPGYEAPcyvAwSA..qNRSplIRIPasrg...istRvEVRsvDPAANPY    343
HALVO       VPGYEAPVyiAwSd..RNRSAlIRkPaartP..aAsRIEaRFPDPscNPY    355
LACDE       iPGfEAPVyiswas..RNRSpmvRIPnane...vgtRlEmRstDPtANPY    346
P.          VPGfEtcqkLfYSfg..sRSAvIRlslinysnpsekRIEfRlPDcAnsPh   308
GLNA_YEAST  tgrhEtasMtAfSsgvaNRgsSIRIPrsvake.gygyfEdRrPasnidPY   341
GLN1_CHLRE  tgkhEtssMsdfSwgvaNRgcSIRvgrmvpve.KsgyyEdRrPasnldaY   369
GLN1_MAIZE  tgrhEtadintfkwgvaNRgASIRvgrdtere.gkgyfEdRrPasnmdPY   341
GLN1_ORYSA  tgrhEtadintfkwgvaNRgASIRvgrdteke.gkgyfEdRrPasnmdPY   341
GLNA_LUPLU  tgrhEtadistffwgvaNRgASIRvgrdteke.gkgyfEdRrPasnmdPY   341
GLN1_PEA    tgkhEtadinvfSwgvaNRgsSIRvgrdtekd.gkgyfEdRrPasnmdPY   341
GLN1_DROME  VgrlEtssidkfSwgvaNRavSvRvPrgvata.gkgylEdRrPssncdPY   385
GLNA_SQUAC  tghhEtsninefSAgvaNRgASIRIPrsvgqd.KkgyfEdRrPsancdPY   378
GLNA_XENLA  tqghEtssihefSAgvaNRgASIRIPrqvgqe.gygyfEdRrPaancdPY   349
GLNA_CHICK  tgfhEtssihefSAgvaNRgASIRIPrnvghe.KkgyfEdRgPsancdPY   349
MOUSE       tgfhEtsnindfSAsvaNRSASIRIPwtvgqe.KkgyfEdRrPsancdPY   347
HAMPSTR     tgfhktsnindfSAgvadRSASIRIPrtvgqe.KkgyfEaRcPsancdPf   349
GLNA_HUMAN  tgfhEtsnindfSAgvaNRSASIRIPrtvgqe.KkgyfEdRrPsancdPf   349
Consensus   tg  het        fsagvanrsasiripr       gy edr p  n dpy

SALTY       LCFAALLMAGLDGIKNKIHPGEPMD..KNLYDLPPEE..AKEIPQVAGSL    414
THIFE       LaFsAmLMAGLDGIqNKIHPataMD..KNLYDLPaEE..qanIPgVAaSL    414
ECOLI       LCFAALLMAGLDGIKNKIHPGEaMD..KNLYDLPPEE..AKEIPQVAGSL    414
ARCFU       LaitAqLaAGLDGIKkKIdPGdPll..edvYeLtPaqkrelgvgelpttL   440
SEQUENCE    LaFAALLMAGLDGIqNKIHPGdaaD..KNLYDLPPEE..AKEIPQVcGSL    413
BACCE       LvmAtLLaAGLDGIKNKltPpaavD..rNiYvmtkEEreeagIvdlpatL   391
```

160

```
BACSU      LalsvLLaAGLDGIKNKleapaPiD..rNiYvmskEErmengIvdlpatL    391
HALVO      LaFAALihAGLDGvekgldcpdPvr..eNiYefdeakreeygIetlpkts    403
LACDE      LlllsAcLkAGLtGIKegklPmaPvt..sNLfemtddErkelgIkplpstL   394
P.         LvmAAiilAGyDGIKsKeqPlvPfeskdNhfyissifskyvqhPenfniL    358
GLNA_YEAST LvtgimcetvcgaIdNadmtkEfer..ess...................    369
GLN1_CHLRE vvtrlivettill.................................       382
GLN1_MAIZE vvtgmiaettilwngN..............................       357
GLN1_ORYSA vvtgmiaettLlwkqN..............................       357
GLNA_LUPLU vvtsmiaettLl..................................       353
GLN1_PEA   vvtsmiaettilwkKp..............................       357
GLN1_DROME avcnAivqtcLlne................................       399
GLNA_SQUAC avteALvrtcLldesgdkpieynkn.....................       403
GLNA_XENLA avteALvrttilnetgsetkdykng..agfsraigmasprdaavf.....  392
GLNA_CHICK avteALvrtcLlnetgdepfeykn......................      373
MOUSE      avteAivrtcLlnetgdepfqykn......................      371
HAMPSTR    avteAivrtcLlnetgdqpfqykn......................      373
GLNA_HUMAN svteALirtcLlnetgdepfqykn......................      373
Consensus    vt a    t ll

SALTY      EEALNALDLDREFLKAGGVFTDEAIDAYIALRREEDDRVRMTPHPVEFEL    464
THIFE      EEALrALeaDhdFLmkGGVFseswlegYldvkwaEvqtlRvTtHPVEFqm    464
ECOLI      EEALNeLDLDREFLKAGGVFTDEAIDAYIALRREEDDRVRMTPHPVEFEL    464
ARCFU      rdAidhLas.dElmqe..VlgshifDAfmeLkidEwnqyclyitPwEFmk    487
SEQUENCE   kEALeeLDkgRaFLtkGGVFsDdfIDAYleLksEEeikVRtfvHPlEydL    463
BACCE      aqALvtLqs.nEvisn..algDhllehfIeakefEwDifRtqvHqwErdq    438
BACSU      aEALeefks.nEvmvk..algehlfehfIeakeiEwDmfRtqvHpwErEq    438
HALVO      aarrrprr..dEviqe..algDhvfekfveakRsEfkdylvdvsqwEldr    449
LACDE      hnAikAfke.dEvvKs..aFsehivDsfleLketEwalytqsvsewEvkr    441
P.         thALegyeslhtinespefknffkceepqgisfslvesldalekdhaFlt   408
GLNA_YEAST ...................................................   369
GLN1_CHLRE ...................................................   382
GLN1_MAIZE ...................................................   357
GLN1_ORYSA ...................................................   357
GLNA_LUPLU ...................................................   353
GLN1_PEA   ...................................................   357
GLN1_DROME ...................................................   399
GLNA_SQUAC ...................................................   403
GLNA_XENLA ...................................................   392
GLNA_CHICK ...................................................   373
MOUSE      ...................................................   371
HAMPSTR    ...................................................   373
GLNA_HUMAN ...................................................   373
Consensus

SALTY      YYSV................    468
THIFE      YYS1................    468
ECOLI      YYSV................    468
ARCFU      Yfdi................    491
SEQUENCE   YYSV................    467
BACCE      YmSly...............    443
BACSU      YmSqy...............    443
HALVO      Yletf...............    454
LACDE      Yfny................    445
P.         vnniftevsqriknkhyhgk    428
GLNA_YEAST ....................    369
GLN1_CHLRE ....................    382
GLN1_MAIZE ....................    357
GLN1_ORYSA ....................    357
GLNA_LUPLU ....................    353
GLN1_PEA   ....................    357
GLN1_DROME ....................    399
GLNA_SQUAC ....................    403
GLNA_XENLA ....................    392
GLNA_CHICK ....................    373
MOUSE      ....................    371
HAMPSTR    ....................    373
GLNA_HUMAN ....................    373
Consensus
```

# FIGURE 8

FIGURE 9

# FIGURE 10

# FIGURE 11

FIGURE 12

FIGURE 13

FIGURE 14

FIGURE 15

FIGURE 16

# FIGURE 17

FIGURE 18

FIGURE 19A

FIGURE 19B

FIGURE 20A

FIGURE 20B

FIGURE 21A

FIGURE 21B

FIGURE 22A

FIGURE 22B

FIGURE 23A

FIGURE 23B

FIGURE 24A

FIGURE 24B

FIGURE 25

FIGURE 26

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1396491 A2
- WO 0137835 A1
- WO 200909160 A

**Non-patent literature cited in the description**

- *Bioorganic and Medicinal Chemistry Letters,* vol. 18 (14), 4006-4010
- **LAUFER SA et al.** Design, synthesis, and biological evaluation of novel Tri- and tetrasubstituted imidazoles as highly potent and specific ATP-mimetic inhibitors of p38 MAP kinase: focus on optimized interactions with the enzyme's surface-exposed front region. *Journal of Medicinal Chemistry,* 2008, vol. 51 (14), 4122-4149
- Structural Genomics Consortium. University of Oxford

SEQUENCE LISTING

<110> CSIR

<120> Inhibition of the activity of kinase and sythetase enzymes

<130> P46592ZA00

<150> 2009/09160
<151> 2009-12-22

<160> 59

<170> PatentIn version 3.5

<210> 1
<211> 194
<212> PRT
<213> Homo sapiens

<400> 1

```
Met Glu Glu Lys Leu Lys Lys Thr Lys Ile Ile Phe Val Val Gly Gly
1               5               10              15

Pro Gly Ser Gly Lys Gly Thr Gln Cys Glu Lys Ile Val Gln Lys Tyr
        20              25              30

Gly Tyr Thr His Leu Ser Thr Gly Asp Leu Leu Arg Ser Glu Val Ser
        35              40              45

Ser Gly Ser Ala Arg Gly Lys Lys Leu Ser Glu Ile Met Glu Lys Gly
    50              55              60

Gln Leu Val Pro Leu Glu Thr Val Leu Asp Met Leu Arg Asp Ala Met
65              70              75              80

Val Ala Lys Val Asn Thr Ser Lys Gly Phe Leu Ile Asp Gly Tyr Pro
            85              90              95

Arg Glu Val Gln Gln Gly Glu Glu Phe Glu Arg Arg Ile Gly Gln Pro
        100             105             110

Thr Leu Leu Leu Tyr Val Asp Ala Gly Pro Glu Thr Met Thr Gln Arg
        115             120             125

Leu Leu Lys Arg Gly Glu Thr Ser Gly Arg Val Asp Asp Asn Glu Glu
    130             135             140

Thr Ile Lys Lys Arg Leu Glu Thr Tyr Tyr Lys Ala Thr Glu Pro Val
145             150             155             160

Ile Ala Phe Tyr Glu Lys Arg Gly Ile Val Arg Lys Val Asn Ala Glu
            165             170             175

Gly Ser Val Asp Ser Val Phe Ser Gln Val Cys Thr His Leu Asp Ala
            180             185             190

Leu Lys
```

<210> 2
<211> 239
<212> PRT
<213> Homo sapiens

<400> 2

```
Met Ala Pro Ser Val Pro Ala Ala Glu Pro Glu Tyr Pro Lys Gly Ile
1               5               10              15

Arg Ala Val Leu Leu Gly Pro Pro Gly Ala Gly Lys Gly Thr Gln Ala
        20              25              30

Pro Arg Leu Ala Glu Asn Phe Cys Val Cys His Leu Ala Thr Gly Asp
        35              40              45

Met Leu Arg Ala Met Val Ala Ser Gly Ser Glu Leu Gly Lys Lys Leu
        50              55              60

Lys Ala Thr Met Asp Ala Gly Lys Leu Val Ser Asp Glu Met Val Val
65              70              75              80

Glu Leu Ile Glu Lys Asn Leu Glu Thr Pro Leu Cys Lys Asn Gly Phe
                85              90              95

Leu Leu Asp Gly Phe Pro Arg Thr Val Arg Gln Ala Glu Met Leu Asp
            100             105             110

Asp Leu Met Glu Lys Arg Lys Glu Lys Leu Asp Ser Val Ile Glu Phe
            115             120             125

Ser Ile Pro Asp Ser Leu Leu Ile Arg Arg Ile Thr Gly Arg Leu Ile
    130             135             140

His Pro Lys Ser Gly Arg Ser Tyr His Glu Glu Phe Asn Pro Pro Lys
145             150             155             160

Glu Pro Met Lys Asp Asp Ile Thr Gly Glu Pro Leu Ile Arg Arg Ser
            165             170             175

Asp Asp Asn Glu Lys Ala Leu Lys Ile Arg Leu Gln Ala Tyr His Thr
            180             185             190

Gln Thr Thr Pro Leu Ile Glu Tyr Tyr Arg Lys Arg Gly Ile His Ser
        195             200             205

Ala Ile Asp Ala Ser Gln Thr Pro Asp Val Val Phe Ala Ser Ile Leu
    210             215             220

Ala Ala Phe Ser Lys Ala Thr Cys Lys Asp Leu Val Met Phe Ile
225             230             235
```

<210> 3

<211> 227
<212> PRT
<213> Homo sapiens

<400> 3

```
Met Gly Ala Ser Ala Arg Leu Leu Arg Ala Val Ile Met Gly Ala Pro
1               5                   10                  15

Gly Ser Gly Lys Gly Thr Val Ser Ser Arg Ile Thr Thr His Phe Glu
            20                  25                  30

Leu Lys His Leu Ser Ser Gly Asp Leu Leu Arg Asp Asn Met Leu Arg
            35                  40                  45

Gly Thr Glu Ile Gly Val Leu Ala Lys Ala Phe Ile Asp Gln Gly Lys
        50                  55                  60

Leu Ile Pro Asp Asp Val Met Thr Arg Leu Ala Leu His Glu Leu Lys
65                  70                  75                  80

Asn Leu Thr Gln Tyr Ser Trp Leu Leu Asp Gly Phe Pro Arg Thr Leu
                85                  90                  95

Pro Gln Ala Glu Ala Leu Asp Arg Ala Tyr Gln Ile Asp Thr Val Ile
            100                 105                 110

Asn Leu Asn Val Pro Phe Glu Val Ile Lys Gln Arg Leu Thr Ala Arg
            115                 120                 125

Trp Ile His Pro Ala Ser Gly Arg Val Tyr Asn Ile Glu Phe Asn Pro
        130                 135                 140

Pro Lys Thr Val Gly Ile Asp Asp Leu Thr Gly Glu Pro Leu Ile Gln
145                 150                 155                 160

Arg Glu Asp Asp Lys Pro Glu Thr Val Ile Lys Arg Leu Lys Ala Tyr
                165                 170                 175

Glu Asp Gln Thr Lys Pro Val Leu Glu Tyr Tyr Gln Lys Lys Gly Val
            180                 185                 190
```

```
Leu Glu Thr Phe Ser Gly Thr Glu Thr Asn Lys Ile Trp Pro Tyr Val
        195                 200                 205

Tyr Ala Phe Leu Gln Thr Lys Val Pro Gln Arg Ser Gln Lys Ala Ser
        210                 215                 220

Val Thr Pro
225
```

<210> 4
<211> 223
<212> PRT
<213> Homo sapiens

<400> 4

```
Met Ala Ser Lys Leu Leu Arg Ala Val Ile Leu Gly Pro Pro Gly Ser
1               5               10              15

Gly Lys Gly Thr Val Cys Gln Arg Ile Ala Gln Asn Phe Gly Leu Gln
        20              25              30

His Leu Ser Ser Gly His Phe Leu Arg Glu Asn Ile Lys Ala Ser Thr
        35              40              45

Glu Val Gly Glu Met Ala Lys Gln Tyr Ile Glu Lys Ser Leu Leu Val
    50              55              60

Pro Asp His Val Ile Thr Arg Leu Met Met Ser Glu Leu Glu Asn Arg
65              70              75              80

Arg Gly Gln His Trp Leu Leu Asp Gly Phe Pro Arg Thr Leu Gly Gln
            85              90              95

Ala Glu Ala Leu Asp Lys Ile Cys Glu Val Asp Leu Val Ile Ser Leu
            100             105             110

Asn Ile Pro Phe Glu Thr Leu Lys Asp Arg Leu Ser Arg Arg Trp Ile
            115             120             125

His Pro Pro Ser Gly Arg Val Tyr Asn Leu Asp Phe Asn Pro Pro His
    130             135             140

Val His Gly Ile Asp Asp Val Thr Gly Glu Pro Leu Val Gln Gln Glu
145             150             155             160

Asp Asp Lys Pro Glu Ala Val Ala Ala Arg Leu Arg Gln Tyr Lys Asp
            165             170             175

Val Ala Lys Pro Val Ile Glu Leu Tyr Lys Ser Arg Gly Val Leu His
            180             185             190

Gln Phe Ser Gly Thr Glu Thr Asn Lys Ile Trp Pro Tyr Val Tyr Thr
    195             200             205

Leu Phe Ser Asn Lys Ile Thr Pro Ile Gln Ser Lys Glu Ala Tyr
    210             215             220
```

<210> 5
<211> 198
<212> PRT
<213> Homo sapiens

<400> 5

```
    Met Gly Gly Phe Met Glu Asp Leu Arg Lys Cys Lys Ile Ile Phe Ile
    1               5               10              15

    Ile Gly Gly Pro Gly Ser Gly Lys Gly Thr Gln Cys Glu Lys Leu Val
                20              25              30

    Glu Lys Tyr Gly Phe Thr His Leu Ser Thr Gly Glu Leu Leu Arg Glu
            35              40              45

    Glu Leu Ala Ser Glu Ser Glu Arg Ser Lys Leu Ile Arg Asp Ile Met
        50              55              60

    Glu Arg Gly Asp Leu Val Pro Ser Gly Ile Val Leu Glu Leu Leu Lys
    65              70              75              80

    Glu Ala Met Val Ala Ser Leu Gly Asp Thr Arg Gly Phe Leu Ile Asp
                85              90              95

    Gly Tyr Pro Arg Glu Val Lys Gln Gly Glu Glu Phe Gly Arg Arg Ile
                100             105             110

    Gly Asp Pro Gln Leu Val Ile Cys Met Asp Cys Ser Ala Asp Thr Met
                115             120             125

    Thr Asn Arg Leu Leu Gln Arg Ser Arg Ser Ser Leu Pro Val Asp Asp
        130             135             140

    Thr Thr Lys Thr Ile Ala Lys Arg Leu Glu Ala Tyr Tyr Arg Ala Ser
    145             150             155             160

    Ile Pro Val Ile Ala Tyr Tyr Glu Thr Lys Thr Gln Leu His Lys Ile
                165             170             175

    Asn Ala Glu Gly Thr Pro Glu Asp Val Phe Leu Gln Leu Cys Thr Ala
                180             185             190

    Ile Asp Ser Ile Ile Phe
                195
```

<210> 6
<211> 172
<212> PRT
<213> Homo sapiens

<400> 6

```
Met Leu Leu Pro Asn Ile Leu Leu Thr Gly Thr Pro Gly Val Gly Lys
1               5                   10                  15

Thr Thr Leu Gly Lys Glu Leu Ala Ser Lys Ser Gly Leu Lys Tyr Ile
            20                  25                  30

Asn Val Gly Asp Leu Ala Arg Glu Glu Gln Leu Tyr Asp Gly Tyr Asp
            35                  40                  45

Glu Glu Tyr Asp Cys Pro Ile Leu Asp Glu Asp Arg Val Val Asp Glu
        50                  55                  60

Leu Asp Asn Gln Met Arg Glu Gly Gly Val Ile Val Asp Tyr His Gly
65                  70                  75                  80

Cys Asp Phe Phe Pro Glu Arg Trp Phe His Ile Val Phe Val Leu Arg
                85                  90                  95

Thr Asp Thr Asn Val Leu Tyr Glu Arg Leu Glu Thr Arg Gly Tyr Asn
            100                 105                 110

Glu Lys Lys Leu Thr Asp Asn Ile Gln Cys Glu Ile Phe Gln Val Leu
            115                 120                 125

Tyr Glu Glu Ala Thr Ala Ser Tyr Lys Glu Glu Ile Val His Gln Leu
    130                 135                 140

Pro Ser Asn Lys Pro Glu Glu Leu Glu Asn Asn Val Asp Gln Ile Leu
145                 150                 155                 160

Lys Trp Ile Glu Gln Trp Ile Lys Asp His Asn Ser
                165                 170
```

<210> 7
<211> 173
<212> PRT
<213> Escherichia coli

<400> 7

```
Met Ala Glu Lys Arg Asn Ile Phe Leu Val Gly Pro Met Gly Ala Gly
1               5                   10                  15
```

```
Lys Ser Thr Ile Gly Arg Gln Leu Ala Gln Gln Leu Asn Met Glu Phe
             20                  25                  30

Tyr Asp Ser Asp Gln Glu Ile Glu Lys Arg Thr Gly Ala Asp Val Gly
             35                  40                  45

Trp Val Phe Asp Leu Glu Gly Glu Glu Gly Phe Arg Asp Arg Glu Glu
             50                  55                  60

Lys Val Ile Asn Glu Leu Thr Glu Lys Gln Gly Ile Val Leu Ala Thr
65                  70                  75                  80

Gly Gly Gly Ser Val Lys Ser Arg Glu Thr Arg Asn Arg Leu Ser Ala
                 85                  90                  95

Arg Gly Val Val Val Tyr Leu Glu Thr Thr Ile Glu Lys Gln Leu Ala
             100                 105                 110

Arg Thr Gln Arg Asp Lys Lys Arg Pro Leu Leu His Val Glu Thr Pro
             115                 120                 125

Pro Arg Glu Val Leu Glu Ala Leu Ala Asn Glu Arg Asn Pro Leu Tyr
    130                 135                 140

Glu Glu Ile Ala Asp Val Thr Ile Arg Thr Asp Asp Gln Ser Ala Lys
145                 150                 155                 160

Val Val Ala Asn Gln Ile Ile His Met Leu Glu Ser Asn
                 165                 170
```

<210> 8
<211> 174
<212> PRT
<213> Escherichia coli

<400> 8

```
Met Thr Gln Pro Leu Phe Leu Ile Gly Pro Arg Gly Cys Gly Lys Thr
1                5                   10                  15

Thr Val Gly Met Ala Leu Ala Asp Ser Leu Asn Arg Arg Phe Val Asp
             20                  25                  30

Thr Asp Gln Trp Leu Gln Ser Gln Leu Asn Met Thr Val Ala Glu Ile
             35                  40                  45

Val Glu Arg Glu Glu Trp Ala Gly Phe Arg Ala Arg Glu Thr Ala Ala
             50                  55                  60
```

```
Leu Glu Ala Val Thr Ala Pro Ser Thr Val Ile Ala Thr Gly Gly Gly
65                  70              75                  80


Ile Ile Leu Thr Glu Phe Asn Arg His Phe Met Gln Asn Asn Gly Ile
                85              90                  95


Val Val Tyr Leu Cys Ala Pro Val Ser Val Leu Val Asn Arg Leu Gln
                100             105             110


Ala Ala Pro Glu Glu Asp Leu Arg Pro Thr Leu Thr Gly Lys Pro Leu
            115             120             125


Ser Glu Glu Val Gln Glu Val Leu Glu Glu Arg Asp Ala Leu Tyr Arg
    130             135             140


Glu Val Ala His Ile Ile Ile Asp Ala Thr Asn Glu Pro Ser Gln Val
145             150             155             160


Ile Ser Glu Ile Arg Ser Ala Leu Ala Gln Thr Ile Asn Cys
                165             170
```

<210> 9
<211> 173
<212> PRT
<213> Klebsiella pneumoniae

<400> 9

```
Met Ala Glu Lys Arg Asn Ile Phe Leu Val Gly Pro Met Gly Ala Gly
1               5                   10              15

Lys Ser Thr Ile Gly Arg Gln Leu Ala Gln Gln Leu Asn Met Glu Phe
        20              25              30

Tyr Asp Ser Asp Gln Glu Ile Glu Lys Arg Thr Gly Ala Asp Val Gly
        35              40              45

Trp Val Phe Asp Val Glu Gly Glu Glu Gly Phe Arg Asp Arg Glu Glu
    50              55              60

Lys Ile Ile Asn Glu Leu Thr Glu Lys Gln Gly Ile Val Leu Ala Thr
65              70              75              80

Gly Gly Gly Ser Val Lys Ser Arg Glu Thr Arg Asn Arg Leu Ser Ala
            85              90              95

Arg Gly Val Val Val Tyr Leu Glu Thr Thr Ile Glu Lys Gln Leu Ala
        100             105             110

Arg Thr Gln Arg Asp Lys Lys Arg Pro Leu Leu Gln Val Asp Ala Pro
        115             120             125

Pro Arg Glu Val Leu Glu Ala Leu Ala Asp Glu Arg Asn Pro Leu Tyr
    130             135             140

Glu Glu Ile Ala Asp Val Thr Ile Arg Thr Asp Asp Gln Ser Ala Lys
145             150             155             160

Val Val Ala Asn Gln Ile Ile His Met Leu Glu Ser Asn
            165             170
```

<210> 10
<211> 177
<212> PRT
<213> Klebsiella pneumoniae

<400> 10

```
Met Thr Gln Pro Ile Phe Leu Ile Gly Pro Arg Gly Cys Gly Lys Thr
1               5               10              15

Thr Val Gly His Ala Leu Ala Arg Ala Arg His Phe Gln Phe Ser Asp
            20              25              30

Thr Asp His Arg Leu Gln Ala His Glu Gln Arg Thr Val Ala Glu Ile
        35              40              45

Val Gln Ala Glu Gly Trp Ala Arg Phe Arg Glu Leu Glu Thr Leu Ser
    50              55              60

Leu Lys Ala Val Thr Leu Pro Asn Thr Val Ile Ala Thr Gly Gly Gly
65              70              75              80

Ile Val Leu Ala Glu Gly Asn Arg Gln Phe Met Arg Glu Asn Gly Val
                85              90              95

Val Ile Tyr Leu Gln Ala Ser Val Ser Ala Leu Ile Asp Arg Leu Glu
        100             105             110

Ala Tyr Pro Lys Ala Glu Gln Arg Pro Thr Leu Thr Gly Lys Pro Val
        115             120             125

Arg Glu Glu Val Gly Glu Val Leu Ala Gln Arg Glu Ala Leu Tyr Arg
    130             135             140

Asp Ala Ala His His Ile Val Asp Ala Thr Ala Ser Pro Asp Arg Val
145             150             155             160

Val Glu Gln Ile Met Ser Met Leu Cys Ser Ala Thr Ala Thr Pro Val
                165             170             175

                              Ser
```

<210> 11
<211> 173
<212> PRT
<213> Yersinia pestis

<400> 11

```
Met Ala Glu Lys Arg Asn Ile Phe Leu Val Gly Pro Met Gly Ala Gly
1               5               10              15

Lys Ser Thr Ile Gly Arg Gln Leu Ala Gln Gln Leu Asn Met Glu Phe
            20              25              30

Phe Asp Ser Asp Gln Glu Ile Glu Arg Arg Thr Gly Ala Asp Val Gly
            35              40              45

Trp Val Phe Asp Val Glu Gly Glu Glu Gly Phe Arg Asp Arg Glu Glu
    50              55              60

Lys Val Ile Asn Glu Leu Thr Glu Lys Gln Gly Ile Val Leu Ala Thr
65              70              75              80

Gly Gly Gly Ser Val Lys Ser Arg Glu Thr Arg Asn Arg Leu Ser Ala
            85              90              95

Arg Gly Val Val Val Tyr Leu Glu Thr Thr Ile Glu Lys Gln Leu Ala
            100             105             110

Arg Thr Gln Arg Asp Lys Lys Arg Pro Leu Leu Gln Val Asp Glu Pro
        115             120             125

Pro Arg Glu Val Leu Glu Ala Leu Ala Lys Glu Arg Asn Pro Leu Tyr
    130             135             140

Glu Glu Ile Ala Asp Val Thr Ile Arg Thr Asp Asp Gln Ser Ala Lys
145             150             155             160

Val Val Ala Asn Gln Ile Ile Asn Met Leu Glu Ser Asn
            165             170
```

<210> 12
<211> 174
<212> PRT
<213> Yersinia pestis

<400> 12

```
Met Thr Gln Thr Ile Phe Met Val Gly Ala Arg Gly Ala Gly Lys Thr
1               5               10              15
```

```
Thr Ile Gly Lys Ala Leu Ala Gln Ala Leu Gly Tyr Arg Phe Val Asp
            20                  25                  30

Thr Asp Leu Phe Met Gln Gln Thr Ser Gln Met Thr Val Ala Glu Val
            35                  40                  45

Val Glu Ser Glu Gly Trp Asp Gly Phe Arg Leu Arg Glu Ser Met Ala
            50                  55                  60

Leu Gln Ala Val Thr Ala Pro Lys Thr Val Val Ala Thr Gly Gly Gly
65                  70                  75                  80

Ala Val Leu Ser Ser Glu Asn Arg Ala Phe Met Arg Asp His Gly Arg
                85                  90                  95

Val Ile Tyr Leu Arg Ala Ser Ala Ala Val Leu Ala Lys Arg Leu Ala
            100                 105                 110

Glu Asp Pro Glu Glu Ala Gln Arg Pro Ser Leu Thr Gly Lys Pro Ile
            115                 120                 125

Val Glu Glu Ile Leu Asp Val Leu Ala Ser Arg Glu Ala Leu Tyr Gln
            130                 135                 140

Asp Val Ala His His Val Leu Asp Gly Thr Gln Thr Pro Ser Leu Val
145                 150                 155                 160

Val Glu Gln Ile Leu Gln Met Leu Thr Gly Glu Met Val Lys
                165                 170
```

<210> 13
<211> 173
<212> PRT
<213> Shigella flexneri

<400> 13

29

```
Met Ala Glu Lys Arg Asn Ile Phe Leu Val Gly Pro Met Gly Ala Gly
1               5               10              15

Lys Ser Thr Ile Gly Arg Gln Leu Ala Gln Gln Leu Asn Met Glu Phe
            20              25              30

Tyr Asp Ser Asp Gln Glu Ile Glu Lys Arg Thr Gly Ala Asp Val Gly
        35              40              45

Trp Val Phe Asp Leu Glu Gly Glu Glu Gly Phe Arg Asp Arg Glu Glu
    50              55              60

Lys Val Ile Asn Glu Leu Thr Glu Lys Gln Gly Ile Val Leu Ala Thr

65              70              75              80

Gly Gly Gly Ser Val Lys Ser Arg Glu Thr Arg Asn Arg Leu Ser Ala
            85              90              95

Arg Gly Val Val Val Tyr Leu Glu Thr Thr Ile Glu Lys Gln Leu Ala
        100             105             110

Arg Thr Gln Arg Asp Lys Lys Arg Pro Leu Leu His Val Glu Thr Pro
        115             120             125

Pro Arg Glu Val Leu Glu Ala Leu Ala Asn Glu Arg Asn Pro Leu Tyr
    130             135             140

Glu Glu Ile Ala Asp Val Thr Ile Arg Thr Asp Asp Gln Ser Ala Lys
145             150             155             160

Val Val Ala Asn Gln Ile Ile His Met Leu Glu Ser Asn
            165             170
```

<210> 14
<211> 174
<212> PRT
<213> Shigella flexneri

<400> 14

```
Met Thr Gln Pro Leu Phe Leu Ile Gly Pro Arg Gly Cys Gly Lys Thr
1               5                   10                  15

Thr Val Gly Met Ala Leu Ala Asp Ser Leu Asn Arg Arg Phe Val Asp
            20                  25                  30

Thr Asp Gln Trp Leu Gln Ser Gln Leu Asn Met Thr Val Ala Glu Ile
        35                  40                  45

Val Glu Arg Glu Glu Trp Ala Gly Phe Arg Ala Arg Glu Thr Ala Ala
    50                  55                  60

Leu Glu Ala Val Thr Ala Ala Ser Thr Val Ile Ala Thr Gly Gly Gly
65                  70                  75                  80

Ile Ile Leu Thr Glu Phe Asn Arg His Phe Met Gln Asn Asn Gly Ile
                85                  90                  95

Val Val Tyr Leu Cys Ala Pro Val Ser Val Leu Val Asn Arg Leu Gln
            100                 105                 110

Ala Ala Pro Glu Glu Asp Leu Arg Pro Thr Leu Thr Gly Lys Pro Leu
            115                 120                 125

Ser Glu Glu Val Gln Glu Val Leu Glu Glu Arg Asp Ala Leu Tyr Arg
    130                 135                 140

Glu Val Ala His Ile Ile Ile Asp Ala Thr Asn Glu Pro Ser Gln Val
145                 150                 155                 160

Ile Ser Glu Ile Arg Ser Ala Leu Ala Gln Thr Ile Asn Cys
                165                 170
```

<210> 15
<211> 176
<212> PRT
<213> Mycobacterium tuberculosis

<400> 15

```
Met Ala Pro Lys Ala Val Leu Val Gly Leu Pro Gly Ser Gly Lys Ser
1               5               10              15

Thr Ile Gly Arg Arg Leu Ala Lys Ala Leu Gly Val Gly Leu Leu Asp
        20              25              30

Thr Asp Val Ala Ile Glu Gln Arg Thr Gly Arg Ser Ile Ala Asp Ile
        35              40              45

Phe Ala Thr Asp Gly Glu Gln Glu Phe Arg Arg Ile Glu Glu Asp Val
    50              55              60

Val Arg Ala Ala Leu Ala Asp His Asp Gly Val Leu Ser Leu Gly Gly
65              70              75              80

Gly Ala Val Thr Ser Pro Gly Val Arg Ala Ala Leu Ala Gly His Thr
            85              90              95

Val Val Tyr Leu Glu Ile Ser Ala Ala Glu Gly Val Arg Arg Thr Gly
            100             105             110

Gly Asn Thr Val Arg Pro Leu Leu Ala Gly Pro Asp Arg Ala Glu Lys
        115             120             125

Tyr Arg Ala Leu Met Ala Lys Arg Ala Pro Leu Tyr Arg Arg Val Ala
    130             135             140

Thr Met Arg Val Asp Thr Asn Arg Arg Asn Pro Gly Ala Val Val Arg
145             150             155             160

His Ile Leu Ser Arg Leu Gln Val Pro Ser Pro Ser Glu Ala Ala Thr
                165             170             175
```

<210> 16

<211> 574
<212> PRT
<213> Rattus Norvegicus

<400> 16

32

```
Met Ser Val Gln Glu Asn Thr Leu Pro Gln Gln Leu Trp Pro Trp Ile
1               5                   10                  15

Phe Arg Ser Gln Lys Asp Leu Ala Lys Ser Ala Leu Ser Gly Ala Pro
            20                  25                  30

Gly Gly Pro Ala Gly Tyr Leu Arg Arg Ala Ser Val Ala Gln Leu Thr
        35                  40                  45

Gln Glu Leu Gly Thr Ala Phe Phe Gln Gln Gln Gln Leu Pro Ala Ala
    50                  55                  60

Met Ala Asp Thr Phe Leu Glu His Leu Cys Leu Leu Asp Ile Asp Ser
65                  70                  75                  80

Gln Pro Val Ala Ala Arg Ser Thr Ser Ile Ile Ala Thr Ile Gly Pro
            85                  90                  95

Ala Ser Arg Ser Val Asp Arg Leu Lys Glu Met Ile Lys Ala Gly Met
            100                 105                 110

Asn Ile Ala Arg Leu Asn Phe Ser His Gly Ser His Glu Tyr His Ala
        115                 120                 125

Glu Ser Ile Ala Asn Ile Arg Glu Ala Thr Glu Ser Phe Ala Thr Ser
    130                 135                 140

Pro Leu Ser Tyr Arg Pro Val Ala Ile Ala Leu Asp Thr Lys Gly Pro
145                 150                 155                 160

Glu Ile Arg Thr Gly Val Leu Gln Gly Gly Pro Glu Ser Glu Val Glu
            165                 170                 175

Ile Val Lys Gly Ser Gln Val Leu Val Thr Val Asp Pro Lys Phe Gln
        180                 185                 190

Thr Arg Gly Asp Ala Lys Thr Val Trp Val Asp Tyr His Asn Ile Thr
        195                 200                 205

Arg Val Val Ala Val Gly Gly Arg Ile Tyr Ile Asp Asp Gly Leu Ile
    210                 215                 220

Ser Leu Val Val Gln Lys Ile Gly Pro Glu Gly Leu Val Thr Glu Val
225                 230                 235                 240
```

```
Glu His Gly Gly Ile Leu Gly Ser Arg Lys Gly Val Asn Leu Pro Asn
                    245             250                 255

Thr Glu Val Asp Leu Pro Gly Leu Ser Glu Gln Asp Leu Leu Asp Leu
            260             265                 270

Arg Phe Gly Val Gln His Asn Val Asp Ile Ile Phe Ala Ser Phe Val
            275             280                 285

Arg Lys Ala Ser Asp Val Leu Ala Val Arg Asp Ala Leu Gly Pro Glu
        290             295                 300

Gly Gln Asn Ile Lys Ile Ile Ser Lys Ile Glu Asn His Glu Gly Val
305             310             315                 320

Lys Lys Phe Asp Glu Ile Leu Glu Val Ser Asp Gly Ile Met Val Ala
            325             330                 335

Arg Gly Asp Leu Gly Ile Glu Ile Pro Ala Glu Lys Val Phe Leu Ala
            340             345                 350

Gln Lys Met Met Ile Gly Arg Cys Asn Leu Ala Gly Lys Pro Val Val
        355             360                 365

Cys Ala Thr Gln Met Leu Glu Ser Met Ile Thr Lys Ala Arg Pro Thr
    370             375                 380

Arg Ala Glu Thr Ser Asp Val Ala Asn Ala Val Leu Asp Gly Ala Asp
385             390                 395                 400

Cys Ile Met Leu Ser Gly Glu Thr Ala Lys Gly Ser Phe Pro Val Glu
            405             410                 415

Ala Val Met Met Gln His Ala Ile Ala Arg Glu Ala Glu Ala Ala Val
        420             425                 430

Tyr His Arg Gln Leu Phe Glu Glu Leu Arg Arg Ala Ala Pro Leu Ser
        435             440                 445

Arg Asp Pro Thr Glu Val Thr Ala Ile Gly Ala Val Glu Ala Ser Phe
        450             455                 460

Lys Cys Cys Ala Ala Ala Ile Ile Val Leu Thr Lys Thr Gly Arg Ser
465             470                 475                 480

Ala Gln Leu Leu Ser Gln Tyr Arg Pro Arg Ala Ala Val Ile Ala Val
            485             490                 495
```

34

```
        Thr Arg Ser Ala Gln Ala Ala Arg Gln Val His Leu Ser Arg Gly Val
                500                     505                 510

        Phe Pro Leu Leu Tyr Arg Glu Pro Pro Glu Ala Ile Trp Ala Asp Asp
                515                     520                 525

        Val Asp Arg Arg Val Gln Phe Gly Ile Glu Ser Gly Lys Leu Arg Gly
                530                     535                 540

        Phe Leu Arg Val Gly Asp Leu Val Ile Val Val Thr Gly Trp Arg Pro
        545                     550                 555                 560

        Gly Ser Gly Tyr Thr Asn Ile Met Arg Val Leu Ser Val Ser
                        565                     570
```

<210> 17
<211> 543
<212> PRT
<213> Rattus norvegicus

<400> 17

```
Met Glu Gly Pro Ala Gly Tyr Leu Arg Arg Ala Ser Val Ala Gln Leu
1               5               10              15

Thr Gln Glu Leu Gly Thr Ala Phe Phe Gln Gln Gln Gln Leu Pro Ala
        20              25              30

Ala Met Ala Asp Thr Phe Leu Glu His Leu Cys Leu Leu Asp Ile Asp
        35              40              45

Ser Gln Pro Val Ala Ala Arg Ser Thr Ser Ile Ile Ala Thr Ile Gly
    50              55              60

Pro Ala Ser Arg Ser Val Asp Arg Leu Lys Glu Met Ile Lys Ala Gly
65              70              75              80

Met Asn Ile Ala Arg Leu Asn Phe Ser His Gly Ser His Glu Tyr His
            85              90              95

Ala Glu Ser Ile Ala Asn Ile Arg Glu Ala Thr Glu Ser Phe Ala Thr
            100             105             110

Ser Pro Leu Ser Tyr Arg Pro Val Ala Ile Ala Leu Asp Thr Lys Gly
        115             120             125

Pro Glu Ile Arg Thr Gly Val Leu Gln Gly Gly Pro Glu Ser Glu Val
    130             135             140

Glu Ile Val Lys Gly Ser Gln Val Leu Val Thr Val Asp Pro Lys Phe
145             150             155             160
```

```
Gln Thr Arg Gly Asp Ala Lys Thr Val Trp Val Asp Tyr His Asn Ile
            165                 170                 175

Thr Arg Val Val Ala Val Gly Gly Arg Ile Tyr Ile Asp Asp Gly Leu
            180                 185                 190

Ile Ser Leu Val Val Gln Lys Ile Gly Pro Glu Gly Leu Val Thr Glu
            195                 200                 205

Val Glu His Gly Gly Ile Leu Gly Ser Arg Lys Gly Val Asn Leu Pro
    210                 215                 220

Asn Thr Glu Val Asp Leu Pro Gly Leu Ser Glu Gln Asp Leu Leu Asp
    225                 230                 235                 240

Leu Arg Phe Gly Val Gln His Asn Val Asp Ile Ile Phe Ala Ser Phe
                245                 250                 255

Val Arg Lys Ala Ser Asp Val Leu Ala Val Arg Asp Ala Leu Gly Pro
            260                 265                 270

Glu Gly Gln Asn Ile Lys Ile Ile Ser Lys Ile Glu Asn His Glu Gly
            275                 280                 285

Val Lys Lys Phe Asp Glu Ile Leu Glu Val Ser Asp Gly Ile Met Val
    290                 295                 300

Ala Arg Gly Asp Leu Gly Ile Glu Ile Pro Ala Glu Lys Val Phe Leu
    305                 310                 315                 320

Ala Gln Lys Met Met Ile Gly Arg Cys Asn Leu Ala Gly Lys Pro Val
                325                 330                 335

Val Cys Ala Thr Gln Met Leu Glu Ser Met Ile Thr Lys Ala Arg Pro
            340                 345                 350

Thr Arg Ala Glu Thr Ser Asp Val Ala Asn Ala Val Leu Asp Gly Ala
            355                 360                 365

Asp Cys Ile Met Leu Ser Gly Glu Thr Ala Lys Gly Ser Phe Pro Val
        370                 375                 380

Glu Ala Val Met Met Gln His Ala Ile Ala Arg Glu Ala Glu Ala Ala
    385                 390                 395                 400

Val Tyr His Arg Gln Leu Phe Glu Glu Leu Arg Arg Ala Ala Pro Leu
                405                 410                 415
```

37

```
Ser Arg Asp Pro Thr Glu Val Thr Ala Ile Gly Ala Val Glu Ala Ser
        420             425             430

Phe Lys Cys Cys Ala Ala Ala Ile Ile Val Leu Thr Lys Thr Gly Arg
        435             440             445

Ser Ala Gln Leu Leu Ser Gln Tyr Arg Pro Arg Ala Ala Val Ile Ala
        450             455             460

Val Thr Arg Ser Ala Gln Ala Ala Arg Gln Val His Leu Ser Arg Gly
465             470             475             480

Val Phe Pro Leu Leu Tyr Arg Glu Pro Pro Glu Ala Ile Trp Ala Asp
            485             490             495

Asp Val Asp Arg Arg Val Gln Phe Gly Ile Glu Ser Gly Lys Leu Arg
        500             505             510

Gly Phe Leu Arg Val Gly Asp Leu Val Ile Val Val Thr Gly Trp Arg
        515             520             525

Pro Gly Ser Gly Tyr Thr Asn Ile Met Arg Val Leu Ser Val Ser
        530             535             540
```

<210> 18
<211> 531
<212> PRT
<213> Rattus norvegicus

<400> 18

```
Met Ser Lys Ser His Ser Glu Ala Gly Ser Ala Phe Ile Gln Thr Gln
1               5                   10                  15

Gln Leu His Ala Ala Met Ala Asp Thr Phe Leu Glu His Met Cys Arg
            20                  25                  30

Leu Asp Ile Asp Ser Ala Pro Ile Thr Ala Arg Asn Thr Gly Ile Ile
            35                  40                  45

Cys Thr Ile Gly Pro Ala Ser Arg Ser Val Glu Thr Leu Lys Glu Met
    50                  55                  60

Ile Lys Ser Gly Met Asn Val Ala Arg Met Asn Phe Ser His Gly Thr
65                  70                  75                  80

His Glu Tyr His Ala Glu Thr Ile Lys Asn Val Arg Thr Ala Thr Glu
                85                  90                  95

Ser Phe Ala Ser Asp Pro Ile Leu Tyr Arg Pro Val Ala Val Ala Leu
```

                    100                      105                         110

Asp Thr Lys Gly Pro Glu Ile Arg Thr Gly Leu Ile Lys Gly Ser Gly
        115             120             125

Thr Ala Glu Val Glu Leu Lys Lys Gly Ala Thr Leu Lys Ile Thr Leu
        130             135             140

Asp Asn Ala Tyr Met Glu Lys Cys Asp Glu Asn Ile Leu Trp Leu Asp
145             150             155             160

Tyr Lys Asn Ile Cys Lys Val Val Asp Val Gly Ser Lys Val Tyr Val
            165             170             175

Asp Asp Gly Leu Ile Ser Leu Gln Val Lys Gln Lys Gly Pro Asp Phe
        180             185             190

Leu Val Thr Glu Val Glu Asn Gly Gly Phe Leu Gly Ser Lys Lys Gly
        195             200             205

Val Asn Leu Pro Gly Ala Ala Val Asp Leu Pro Ala Val Ser Glu Lys
    210             215             220

Asp Ile Gln Asp Leu Lys Phe Gly Val Glu Gln Asp Val Asp Met Val
225             230             235             240

Phe Ala Ser Phe Ile Arg Lys Ala Ala Asp Val His Glu Val Arg Lys
            245             250             255

Ile Leu Gly Glu Lys Gly Lys Asn Ile Lys Ile Ser Lys Ile Glu
        260             265             270

Asn His Glu Gly Val Arg Arg Phe Asp Glu Ile Leu Glu Ala Ser Asp
        275             280             285

Gly Ile Met Val Ala Arg Gly Asp Leu Gly Ile Glu Ile Pro Ala Glu
        290             295             300

Lys Val Phe Leu Ala Gln Lys Met Ile Ile Gly Arg Cys Asn Arg Ala
305             310             315             320

Gly Lys Pro Val Ile Cys Ala Thr Gln Met Leu Glu Ser Met Ile Lys
            325             330             335

Lys Pro Arg Pro Thr Arg Ala Glu Gly Ser Asp Val Ala Asn Ala Val
        340             345             350

Leu Asp Gly Ala Asp Cys Ile Met Leu Ser Gly Glu Thr Ala Lys Gly
        355             360             365

```
Asp Tyr Pro Leu Glu Ala Val Arg Met Gln His Leu Ile Ala Arg Glu
    370             375             380

Ala Glu Ala Ala Met Phe His Arg Lys Leu Phe Glu Glu Leu Ala Arg
385             390             395                 400

Ser Ser Ser His Ser Thr Asp Leu Met Glu Ala Met Ala Met Gly Ser
                405             410             415

Val Glu Ala Ser Tyr Lys Cys Leu Ala Ala Ala Leu Ile Val Leu Thr
            420             425             430

Glu Ser Gly Arg Ser Ala His Gln Val Ala Arg Tyr Arg Pro Arg Ala
            435             440             445

Pro Ile Ile Ala Val Thr Arg Asn His Gln Thr Ala Arg Gln Ala His
    450             455             460

Leu Tyr Arg Gly Ile Phe Pro Val Val Cys Lys Asp Pro Val Gln Glu
465             470             475                 480

Ala Trp Ala Glu Asp Val Asp Leu Arg Val Asn Leu Ala Met Asn Val
            485             490             495

Gly Lys Ala Arg Gly Phe Phe Lys Lys Gly Asp Val Val Ile Val Leu
            500             505             510

Thr Gly Trp Arg Pro Gly Ser Gly Phe Thr Asn Thr Met Arg Val Val
            515             520             525

Pro Val Pro
    530
```

<210> 19
<211> 531
<212> PRT
<213> Rattus norvegicus

<400> 19

```
Met Pro Lys Pro Asp Ser Glu Ala Gly Thr Ala Phe Ile Gln Thr Gln
1               5               10              15

Gln Leu His Ala Ala Met Ala Asp Thr Phe Leu Glu His Met Cys Arg
            20              25              30

Leu Asp Ile Asp Ser Ala Pro Ile Thr Ala Arg Asn Thr Gly Ile Ile
            35              40              45
```

```
Cys Thr Ile Gly Pro Ala Ser Arg Ser Val Glu Met Leu Lys Glu Met
    50                  55                  60

Ile Lys Ser Gly Met Asn Val Ala Arg Leu Asn Phe Ser His Gly Thr
65              70                  75                  80

His Glu Tyr His Ala Glu Thr Ile Lys Asn Val Arg Ala Ala Thr Glu
                85                  90                  95

Ser Phe Ala Ser Asp Pro Ile Leu Tyr Arg Pro Val Ala Val Ala Leu
            100                 105                 110

Asp Thr Lys Gly Pro Glu Ile Arg Thr Gly Leu Ile Lys Gly Ser Gly
            115                 120                 125

Thr Ala Glu Val Glu Leu Lys Lys Gly Ala Thr Leu Lys Ile Thr Leu
    130                 135                 140

Asp Asn Ala Tyr Met Glu Lys Cys Asp Glu Asn Ile Leu Trp Leu Asp
145                 150                 155                 160

Tyr Lys Asn Ile Cys Lys Val Val Glu Val Gly Ser Lys Ile Tyr Val
            165                 170                 175

Asp Asp Gly Leu Ile Ser Leu Gln Val Lys Glu Lys Gly Ala Asp Tyr
            180                 185                 190

Leu Val Thr Glu Val Glu Asn Gly Gly Ser Leu Gly Ser Lys Lys Gly
        195                 200                 205

Val Asn Leu Pro Gly Ala Ala Val Asp Leu Pro Ala Val Ser Glu Lys
    210                 215                 220

Asp Ile Gln Asp Leu Lys Phe Gly Val Glu Gln Asp Val Asp Met Val
225                 230                 235                 240

Phe Ala Ser Phe Ile Arg Lys Ala Ala Asp Val His Glu Val Arg Lys
            245                 250                 255

Val Leu Gly Glu Lys Gly Lys Asn Ile Lys Ile Ile Ser Lys Ile Glu
        260                 265                 270

Asn His Glu Gly Val Arg Arg Phe Asp Glu Ile Leu Glu Ala Ser Asp
        275                 280                 285

Gly Ile Met Val Ala Arg Gly Asp Leu Gly Ile Glu Ile Pro Ala Glu
        290                 295                 300

Lys Val Phe Leu Ala Gln Lys Met Met Ile Gly Arg Cys Asn Arg Ala
```

|     |     |     |     |     |     | 305 |     |     |     | 310 |     |     |     | 315 |     |     |     | 320 |
| --- | --- | --- | --- | --- | --- | --- | --- | --- | --- | --- | --- | --- | --- | --- | --- | --- | --- | --- |

Gly Lys Pro Val Ile Cys Ala Thr Gln Met Leu Glu Ser Met Ile Lys
325        330       335

Lys Pro Arg Pro Thr Arg Ala Glu Gly Ser Asp Val Ala Asn Ala Val
340        345       350

Leu Asp Gly Ala Asp Cys Ile Met Leu Ser Gly Glu Thr Ala Lys Gly
355        360       365

Asp Tyr Pro Leu Glu Ala Val Arg Met Gln His Leu Ile Ala Arg Glu
370        375       380

Ala Glu Ala Ala Val Phe His Arg Leu Leu Phe Glu Glu Leu Ala Arg
385        390       395       400

Ala Ser Ser Gln Ser Thr Asp Pro Leu Glu Ala Met Ala Met Gly Ser
405        410       415

Val Glu Ala Ser Tyr Lys Cys Leu Ala Ala Ala Leu Ile Val Leu Thr
420        425       430

Glu Ser Gly Arg Ser Ala His Gln Val Ala Arg Tyr Arg Pro Arg Ala
435        440       445

Pro Ile Ile Ala Val Thr Arg Asn Pro Gln Thr Ala Arg Gln Ala His
450        455       460

Leu Tyr Arg Gly Ile Phe Pro Val Leu Cys Lys Asp Ala Val Leu Asp
465        470       475       480

Ala Trp Ala Glu Asp Val Asp Leu Arg Val Asn Leu Ala Met Asn Val
485        490       495

Gly Lys Ala Arg Gly Phe Phe Lys Lys Gly Asp Val Val Ile Val Leu
500        505       510

Thr Gly Trp Arg Pro Gly Ser Gly Phe Thr Asn Thr Met Arg Val Val
515        520       525

Pro Val Pro
530

<210> 20
<211> 381
<212> PRT

<213> Homo sapiens

<400> 20

```
Met Pro Phe Ser Asn Ser His Asn Ala Leu Lys Leu Arg Phe Pro Ala
1               5                   10                  15

Glu Asp Glu Phe Pro Asp Leu Ser Ala His Asn Asn His Met Ala Lys
            20              25                  30

Val Leu Thr Pro Glu Leu Tyr Ala Glu Leu Arg Ala Lys Ser Thr Pro
            35              40                  45

Ser Gly Phe Thr Leu Asp Asp Val Ile Gln Thr Gly Val Asp Asn Pro
        50              55              60

Gly His Pro Tyr Ile Met Thr Val Gly Cys Val Ala Gly Asp Glu Glu
65              70              75              80

Ser Tyr Glu Val Phe Lys Asp Leu Phe Asp Pro Ile Ile Glu Asp Arg
            85              90                  95

His Gly Gly Tyr Lys Pro Ser Asp Glu His Lys Thr Asp Leu Asn Pro
            100             105                 110

Asp Asn Leu Gln Gly Gly Asp Asp Leu Asp Pro Asn Tyr Val Leu Ser
        115             120                 125

Ser Arg Val Arg Thr Gly Arg Ser Ile Arg Gly Phe Cys Leu Pro Pro
        130             135                 140

His Cys Ser Arg Gly Glu Arg Arg Ala Ile Glu Lys Leu Ala Val Glu
145             150                 155                 160

Ala Leu Ser Ser Leu Asp Gly Asp Leu Ala Gly Arg Tyr Tyr Ala Leu
            165                 170                 175

Lys Ser Met Thr Glu Ala Glu Gln Gln Gln Leu Ile Asp Asp His Phe
            180                 185                 190

Leu Phe Asp Lys Pro Val Ser Pro Leu Leu Leu Ala Ser Gly Met Ala
        195                 200                 205

Arg Asp Trp Pro Asp Ala Arg Gly Ile Trp His Asn Asp Asn Lys Thr
        210                 215                 220

Phe Leu Val Trp Val Asn Glu Glu Asp His Leu Arg Val Ile Ser Met
225                 230                 235                 240

Gln Lys Gly Gly Asn Met Lys Glu Val Phe Thr Arg Phe Cys Thr Gly
            245                 250                 255
```

```
Leu Thr Gln Ile Glu Thr Leu Phe Lys Ser Lys Asp Tyr Glu Phe Met
        260                 265             270

Trp Asn Pro His Leu Gly Tyr Ile Leu Thr Cys Pro Ser Asn Leu Gly
        275                 280             285

Thr Gly Leu Arg Ala Gly Val His Ile Lys Leu Pro Asn Leu Gly Lys
        290                 295             300

His Glu Lys Phe Ser Glu Val Leu Lys Arg Leu Arg Leu Gln Lys Arg
305                 310                 315                 320

Gly Thr Gly Gly Val Asp Thr Ala Ala Val Gly Gly Val Phe Asp Val
                325                 330                 335

Ser Asn Ala Asp Arg Leu Gly Phe Ser Glu Val Glu Leu Val Gln Met
        340                 345             350

Val Val Asp Gly Val Lys Leu Leu Ile Glu Met Glu Gln Arg Leu Glu
        355                 360                 365

Gln Gly Gln Ala Ile Asp Asp Leu Met Pro Ala Gln Lys
        370                 375             380
```

<210> 21
<211> 381
<212> PRT
<213> Homo sapiens

<400> 21

```
Met Pro Phe Gly Asn Thr His Asn Lys Phe Lys Leu Asn Tyr Lys Pro
1                   5                   10                  15


Glu Glu Glu Tyr Pro Asp Leu Ser Lys His Asn Asn His Met Ala Lys
              20                  25                  30


Val Leu Thr Leu Glu Leu Tyr Lys Lys Leu Arg Asp Lys Glu Thr Pro
          35                  40                  45


Ser Gly Phe Thr Val Asp Asp Val Ile Gln Thr Gly Val Asp Asn Pro
      50                  55                  60


Gly His Pro Phe Ile Met Thr Val Gly Cys Val Ala Gly Asp Glu Glu
65                  70                  75                  80


Ser Tyr Glu Val Phe Lys Glu Leu Phe Asp Pro Ile Ile Ser Asp Arg
                  85                  90                  95


His Gly Gly Tyr Lys Pro Thr Asp Lys His Lys Thr Asp Leu Asn His
              100                 105                 110
```

Glu Asn Leu Lys Gly Gly Asp Asp Leu Asp Pro Asn Tyr Val Leu Ser
115 120 125

Ser Arg Val Arg Thr Gly Arg Ser Ile Lys Gly Tyr Thr Leu Pro Pro
130 135 140

His Cys Ser Arg Gly Glu Arg Arg Ala Val Glu Lys Leu Ser Val Glu
145 150 155 160

Ala Leu Asn Ser Leu Thr Gly Glu Phe Lys Gly Lys Tyr Tyr Pro Leu
165 170 175

Lys Ser Met Thr Glu Lys Glu Gln Gln Gln Leu Ile Asp Asp His Phe
180 185 190

Leu Phe Asp Lys Pro Val Ser Pro Leu Leu Leu Ala Ser Gly Met Ala
195 200 205

Arg Asp Trp Pro Asp Ala Arg Gly Ile Trp His Asn Asp Asn Lys Ser
210 215 220

Phe Leu Val Trp Val Asn Glu Glu Asp His Leu Arg Val Ile Ser Met
225 230 235 240

Glu Lys Gly Gly Asn Met Lys Glu Val Phe Arg Arg Phe Cys Val Gly
245 250 255

Leu Gln Lys Ile Glu Glu Ile Phe Lys Lys Ala Gly His Pro Phe Met
260 265 270

Trp Asn Gln His Leu Gly Tyr Val Leu Thr Cys Pro Ser Asn Leu Gly
275 280 285

Thr Gly Leu Arg Gly Gly Val His Val Lys Leu Ala His Leu Ser Lys
290 295 300

His Pro Lys Phe Glu Glu Ile Leu Thr Arg Leu Arg Leu Gln Lys Arg
305 310 315 320

Gly Thr Gly Gly Val Asp Thr Ala Ala Val Gly Ser Val Phe Asp Val
325 330 335

Ser Asn Ala Asp Arg Leu Gly Ser Ser Glu Val Glu Gln Val Gln Leu
340 345 350

Val Val Asp Gly Val Lys Leu Met Val Glu Met Glu Lys Lys Leu Glu
355 360 365

```
Lys Gly Gln Ser Ile Asp Asp Met Ile Pro Ala Gln Lys
    370                 375                 380
```

<210> 22
<211> 417
<212> PRT
<213> Homo sapiens

<400> 22

```
Lys Gly Gln Ser Ile Asp Asp Met Ile Pro Ala Gln Lys
```

```
Met Ala Gly Pro Phe Ser Arg Leu Leu Ser Ala Arg Pro Gly Leu Arg
1               5                   10                  15

Leu Leu Ala Leu Ala Gly Ala Gly Ser Leu Ala Ala Gly Phe Leu Leu
            20                  25                  30

Arg Pro Glu Pro Val Arg Ala Ala Ser Glu Arg Arg Arg Leu Tyr Pro
        35                  40                  45

Pro Ser Ala Glu Tyr Pro Asp Leu Arg Lys His Asn Asn Cys Met Ala
    50                  55                  60

Ser His Leu Thr Pro Ala Val Tyr Ala Arg Leu Cys Asp Lys Thr Thr
65                  70                  75                  80

Pro Thr Gly Trp Thr Leu Asp Gln Cys Ile Gln Thr Gly Val Asp Asn
            85                  90                  95

Pro Gly His Pro Phe Ile Lys Thr Val Gly Met Val Ala Gly Asp Glu
            100                 105                 110

Glu Thr Tyr Glu Val Phe Ala Asp Leu Phe Asp Pro Val Ile Gln Glu
        115                 120                 125

Arg His Asn Gly Tyr Asp Pro Arg Thr Met Lys His Thr Thr Asp Leu
    130                 135                 140

Asp Ala Ser Lys Ile Arg Ser Gly Tyr Phe Asp Glu Arg Tyr Val Leu
145                 150                 155                 160

Ser Ser Arg Val Arg Thr Gly Arg Ser Ile Arg Gly Leu Ser Leu Pro
            165                 170                 175

Pro Ala Cys Thr Arg Ala Glu Arg Arg Glu Val Glu Arg Val Val Val
            180                 185                 190

Asp Ala Leu Ser Gly Leu Lys Gly Asp Leu Ala Gly Arg Tyr Tyr Arg
            195                 200                 205

Leu Ser Glu Met Thr Glu Ala Glu Gln Gln Gln Leu Ile Asp Asp His
```

EP 2 516 668 B1

210                215                220

Phe Leu Phe Asp Lys Pro Val Ser Pro Leu Leu Thr Ala Ala Gly Met
225                230                235                240

Ala Arg Asp Trp Pro Asp Ala Arg Gly Ile Trp His Asn Asn Glu Lys
245                250                255

Ser Phe Leu Ile Trp Val Asn Glu Glu Asp His Thr Arg Val Ile Ser
260                265                270

Met Glu Lys Gly Gly Asn Met Lys Arg Val Phe Glu Arg Phe Cys Arg
275                280                285

Gly Leu Lys Glu Val Glu Arg Leu Ile Gln Glu Arg Gly Trp Glu Phe
290                295                300

Met Trp Asn Glu Arg Leu Gly Tyr Ile Leu Thr Cys Pro Ser Asn Leu
305                310                315                320

Gly Thr Gly Leu Arg Ala Gly Val His Ile Lys Leu Pro Leu Leu Ser
325                330                335

Lys Asp Ser Arg Phe Pro Lys Ile Leu Glu Asn Leu Arg Leu Gln Lys
340                345                350

Arg Gly Thr Gly Gly Val Asp Thr Ala Ala Thr Gly Gly Val Phe Asp
355                360                365

Ile Ser Asn Leu Asp Arg Leu Gly Lys Ser Glu Val Glu Leu Val Gln
370                375                380

Leu Val Ile Asp Gly Val Asn Tyr Leu Ile Asp Cys Glu Arg Arg Leu
385                390                395                400

Glu Arg Gly Gln Asp Ile Arg Ile Pro Thr Pro Val Ile His Thr Lys
405                410                415

His

<210> 23
<211> 419
<212> PRT
<213> Homo sapiens

<400> 23

51

Met Ala Ser Ile Phe Ser Lys Leu Leu Thr Gly Arg Asn Ala Ser Leu
1                   5                   10                  15

Met Ala Ser Ile Phe Ser Lys Leu Leu Thr Gly Arg Asn Ala Ser Leu
1                   5                   10                  15

```
Leu Phe Ala Thr Met Gly Thr Ser Val Leu Thr Thr Gly Tyr Leu Leu
            20                  25                  30

Asn Arg Gln Lys Val Cys Ala Glu Val Arg Glu Gln Pro Arg Leu Phe
            35                  40                  45

Pro Pro Ser Ala Asp Tyr Pro Asp Leu Arg Lys His Asn Asn Cys Met
            50                  55                  60

Ala Glu Cys Leu Thr Pro Ala Ile Tyr Ala Lys Leu Arg Asn Lys Val
65                  70                  75                  80

Thr Pro Asn Gly Tyr Thr Leu Asp Gln Cys Ile Gln Thr Gly Val Asp
                85                  90                  95

Asn Pro Gly His Pro Phe Ile Lys Thr Val Gly Met Val Ala Gly Asp
                100                 105                 110

Glu Glu Ser Tyr Glu Val Phe Ala Asp Leu Phe Asp Pro Val Ile Lys
            115                 120                 125

Leu Arg His Asn Gly Tyr Asp Pro Arg Val Met Lys His Thr Thr Asp
            130                 135                 140

Leu Asp Ala Ser Lys Ile Thr Gln Gly Gln Phe Asp Glu His Tyr Val
145                 150                 155                 160

Leu Ser Ser Arg Val Arg Thr Gly Arg Ser Ile Arg Gly Leu Ser Leu
                165                 170                 175

Pro Pro Ala Cys Thr Arg Ala Glu Arg Arg Glu Val Glu Asn Val Ala
                180                 185                 190

Ile Thr Ala Leu Glu Gly Leu Lys Gly Asp Leu Ala Gly Arg Tyr Tyr
                195                 200                 205

Lys Leu Ser Glu Met Thr Glu Gln Asp Gln Gln Arg Leu Ile Asp Asp
            210                 215                 220

His Phe Leu Phe Asp Lys Pro Val Ser Pro Leu Leu Thr Cys Ala Gly
225                 230                 235                 240

Met Ala Arg Asp Trp Pro Asp Ala Arg Gly Ile Trp His Asn Tyr Asp
                245                 250                 255

Lys Thr Phe Leu Ile Trp Ile Asn Glu Glu Asp His Thr Arg Val Ile
                260                 265                 270
```

```
Ser Met Glu Lys Gly Gly Asn Met Lys Arg Val Phe Glu Arg Phe Cys
        275             280             285

Arg Gly Leu Lys Glu Val Glu Arg Leu Ile Gln Glu Arg Gly Trp Glu
        290             295             300

Phe Met Trp Asn Glu Arg Leu Gly Tyr Ile Leu Thr Cys Pro Ser Asn
305             310             315             320

Leu Gly Thr Gly Leu Arg Ala Gly Val His Val Arg Ile Pro Lys Leu
            325             330             335

Ser Lys Asp Pro Arg Phe Ser Lys Ile Leu Glu Asn Leu Arg Leu Gln
            340             345             350

Lys Arg Gly Thr Gly Gly Val Asp Thr Ala Ala Val Ala Asp Val Tyr
        355             360             365

Asp Ile Ser Asn Ile Asp Arg Ile Gly Arg Ser Glu Val Glu Leu Val
        370             375             380

Gln Ile Val Ile Asp Gly Val Asn Tyr Leu Val Asp Cys Glu Lys Lys
385             390             395             400

Leu Glu Arg Gly Gln Asp Ile Lys Val Pro Pro Pro Leu Pro Gln Phe
            405             410             415

Gly Lys Lys
```

<210> 24
<211> 523
<212> PRT
<213> Mus musculus

<400> 24

```
Met Ala Ala Ala Leu Gln Val Leu Pro Cys Leu Leu Arg Ala Pro Ser
1               5                   10                  15

Arg Pro Leu Leu Trp Gly Pro Pro Val Ala Arg Met Thr Ser Gly Met
        20                  25                  30

Ala Leu Ala Glu Gln Ala Arg Gln Leu Phe Asp Ser Ala Val Gly Ala
        35                  40                  45

Val Gln Pro Gly Pro Met Leu Gln Arg Thr Leu Ser Leu Asp Pro Ser
    50                  55                  60

Gly Arg Gln Leu Lys Val Arg Asp Arg Thr Phe Gln Leu Arg Glu Asn
65                  70                  75                  80
```

Leu Tyr Leu Val Gly Phe Gly Lys Ala Val Leu Gly Met Ala Ala Ala
                    85                  90                  95

Ala Glu Glu Leu Leu Ala Gln His Leu Val Gln Gly Val Ile Ser Val
                   100                 105                 110

Pro Lys Gly Ile Arg Ala Ala Met Glu His Ala Gly Lys Lys Glu Met
                   115                 120                 125

Leu Leu Lys Pro His Ser Arg Val Gln Val Phe Glu Gly Ala Glu Asp
        130                 135                 140

Asn Leu Pro Asp Arg Asp Ala Leu Arg Ala Ala Leu Thr Ile Gln Gln
145                 150                 155                 160

Leu Ala Glu Gly Leu Thr Ala Asp Asp Leu Leu Leu Val Leu Ile Ser
                   165                 170                 175

Gly Gly Gly Ser Ala Leu Leu Pro Ala Pro Ile Pro Pro Val Thr Leu
                   180                 185                 190

Glu Glu Lys Gln Met Leu Thr Lys Leu Leu Ala Ala Arg Gly Ala Thr
                   195                 200                 205

Ile Gln Glu Leu Asn Thr Ile Arg Lys Ala Leu Ser Gln Leu Lys Gly
        210                 215                 220

Gly Gly Leu Ala Gln Ala Ala Tyr Pro Ala Gln Val Ile Ser Leu Ile
225                 230                 235                 240

Leu Ser Asp Val Ile Gly Asp Pro Leu Glu Val Ile Ala Ser Gly Pro
                   245                 250                 255

Thr Val Ala Ser Ala His Ser Val Gln Asp Cys Leu His Ile Leu Asn
                   260                 265                 270

His Tyr Gly Leu Arg Ala Ala Leu Pro Arg Ser Val Lys Thr Val Leu
                   275                 280                 285

Ser Arg Ala Asp Ser Asp Pro His Gly Pro His Thr Cys Gly His Val
        290                 295                 300

Leu Asn Val Ile Ile Gly Ser Asn Ser Leu Ala Leu Ala Glu Ala Gln
305                 310                 315                 320

Arg Gln Ala Glu Val Leu Gly Tyr His Ala Met Val Leu Ser Thr Ala
                   325                 330                 335

```
Met Gln Gly Asp Val Lys Arg Val Ala Arg Phe Tyr Gly Leu Leu Ala
        340                 345             350

Arg Val Ala Ala Ala His Leu Thr Pro Ser Leu Ala Glu Arg Pro Leu
        355                 360             365

Glu Glu Glu Ala Glu Leu His Gln Leu Ala Ala Glu Leu Gln Leu Pro
    370                 375             380

Asp Leu Gln Leu Glu Glu Ala Leu Glu Ala Val Ala Lys Ala Lys Gly
385                 390             395                 400

Pro Val Cys Leu Leu Ala Gly Gly Glu Pro Thr Val Gln Leu Gln Gly
            405                 410                 415

Ser Gly Lys Gly Gly Arg Asn Gln Glu Leu Ala Leu His Val Gly Val
        420                 425                 430

Glu Leu Gly Arg Gln Pro Leu Gly Pro Ile Asp Val Leu Phe Leu Ser
        435                 440                 445

Gly Gly Thr Asp Gly Gln Asp Gly Pro Thr Lys Val Ala Gly Ala Trp
    450                 455                 460

Val Met Ser Asp Leu Ile Ser Gln Ala Ser Ala Glu Ser Leu Asp Ile
465                 470                 475                 480

Ala Thr Ser Leu Thr Asn Asn Asp Ser Tyr Thr Phe Phe Cys Arg Phe
            485                 490                 495

Arg Gly Gly Thr His Leu Leu His Thr Gly Leu Thr Gly Thr Asn Val
        500                 505                 510

Met Asp Val His Leu Leu Ile Leu His Pro Gln
        515                 520
```

<210> 25
<211> 396
<212> PRT
<213> Mus musculus

<400> 25

Met Leu Leu Lys Pro His Ser Arg Val Gln Val Phe Glu Gly Ala Glu

Asp Asn Leu Pro Asp Arg Asp Ala Leu Arg Ala Ala Leu Thr Ile Gln

Gln Leu Ala Glu Gly Leu Thr Ala Asp Asp Leu Leu Leu Val Leu Ile

```
                  35                      40                      45

         Ser Gly Gly Gly Ser Ala Leu Leu Pro Ala Pro Ile Pro Pro Val Thr
             50              55              60

         Leu Glu Glu Lys Gln Met Leu Thr Lys Leu Leu Ala Ala Arg Gly Ala
         65              70              75              80

         Thr Ile Gln Glu Leu Asn Thr Ile Arg Lys Ala Leu Ser Gln Leu Lys
                         85              90              95

         Gly Gly Gly Leu Ala Gln Ala Ala Tyr Pro Ala Gln Val Ile Ser Leu
                     100             105             110

         Ile Leu Ser Asp Val Ile Gly Asp Pro Leu Glu Val Ile Ala Ser Gly
                 115             120             125

         Pro Thr Val Ala Ser Ala His Ser Val Gln Asp Cys Leu His Ile Leu
             130             135             140

         Asn His Tyr Gly Leu Arg Ala Ala Leu Pro Arg Ser Val Lys Thr Val
         145             150             155             160

         Leu Ser Arg Ala Asp Ser Asp Pro His Gly Pro His Thr Cys Gly His
                     165             170             175

         Val Leu Asn Val Ile Ile Gly Ser Asn Ser Leu Ala Leu Ala Glu Ala
                 180             185             190

         Gln Arg Gln Ala Glu Val Leu Gly Tyr His Ala Met Val Leu Ser Thr
                 195             200             205

         Ala Met Gln Gly Asp Val Lys Arg Val Ala Arg Phe Tyr Gly Leu Leu
             210             215             220

         Ala Arg Val Ala Ala Ala His Leu Thr Pro Ser Leu Ala Glu Arg Pro
         225             230             235             240

         Leu Glu Glu Glu Ala Glu Leu His Gln Leu Ala Ala Glu Leu Gln Leu
                     245             250             255

         Pro Asp Leu Gln Leu Glu Glu Ala Leu Glu Ala Val Ala Lys Ala Lys
                     260             265             270

         Gly Pro Val Cys Leu Leu Ala Gly Gly Glu Pro Thr Val Gln Leu Gln
                     275             280             285

         Gly Ser Gly Lys Gly Gly Arg Asn Gln Glu Leu Ala Leu His Val Gly
             290             295             300
```

EP 2 516 668 B1

```
Val Glu Leu Gly Arg Gln Pro Leu Gly Pro Ile Asp Val Leu Phe Leu
305             310             315             320


Ser Gly Gly Thr Asp Gly Gln Asp Gly Pro Thr Lys Val Ala Gly Ala
            325             330             335


Trp Val Met Ser Asp Leu Ile Ser Gln Ala Ser Ala Glu Ser Leu Asp
            340             345             350


Ile Ala Thr Ser Leu Thr Asn Asn Asp Ser Tyr Thr Phe Phe Cys Arg
            355             360             365


Phe Arg Gly Gly Thr His Leu Leu His Thr Gly Leu Thr Gly Thr Asn
    370             375             380


Val Met Asp Val His Leu Leu Ile Leu His Pro Gln
385             390             395
```

<210> 26
<211> 917
<212> PRT
<213> Homo sapiens

<400> 26

```
Met Ile Ala Ala Gln Leu Leu Ala Tyr Tyr Phe Thr Glu Leu Lys Asp
1               5               10              15


Asp Gln Val Lys Lys Ile Asp Lys Tyr Leu Tyr Ala Met Arg Leu Ser
            20              25              30


Asp Glu Thr Leu Ile Asp Ile Met Thr Arg Phe Arg Lys Glu Met Lys
            35              40              45


Asn Gly Leu Ser Arg Asp Phe Asn Pro Thr Ala Thr Val Lys Met Leu
    50              55              60


Pro Thr Phe Val Arg Ser Ile Pro Asp Gly Ser Glu Lys Gly Asp Phe
65              70              75              80


Ile Ala Leu Asp Leu Gly Gly Ser Ser Phe Arg Ile Leu Arg Val Gln
            85              90              95


Val Asn His Glu Lys Asn Gln Asn Val His Met Glu Ser Glu Val Tyr
            100             105             110


Asp Thr Pro Glu Asn Ile Val His Gly Ser Gly Ser Gln Leu Phe Asp
            115             120             125
```

His Val Ala Glu Cys Leu Gly Asp Phe Met Glu Lys Arg Lys Ile Lys
130                     135                 140

Asp Lys Lys Leu Pro Val Gly Phe Thr Phe Ser Phe Pro Cys Gln Gln
145                 150                 155                 160

Ser Lys Ile Asp Glu Ala Ile Leu Ile Thr Trp Thr Lys Arg Phe Lys
                165                 170                 175

Ala Ser Gly Val Glu Gly Ala Asp Val Val Lys Leu Leu Asn Lys Ala
            180                 185                 190

Ile Lys Lys Arg Gly Asp Tyr Asp Ala Asn Ile Val Ala Val Val Asn
            195                 200                 205

Asp Thr Val Gly Thr Met Met Thr Cys Gly Tyr Asp Asp Gln His Cys
210                 215                 220

Glu Val Gly Leu Ile Ile Gly Thr Gly Thr Asn Ala Cys Tyr Met Glu
225                 230                 235                 240

Glu Leu Arg His Ile Asp Leu Val Glu Gly Asp Glu Gly Arg Met Cys
            245                 250                 255

Ile Asn Thr Glu Trp Gly Ala Phe Gly Asp Asp Gly Ser Leu Glu Asp
            260                 265                 270

Ile Arg Thr Glu Phe Asp Arg Glu Ile Asp Arg Gly Ser Leu Asn Pro
            275                 280                 285

Gly Lys Gln Leu Phe Glu Lys Met Val Ser Gly Met Tyr Leu Gly Glu
290                 295                 300

Leu Val Arg Leu Ile Leu Val Lys Met Ala Lys Glu Gly Leu Leu Phe
305                 310                 315                 320

Glu Gly Arg Ile Thr Pro Glu Leu Leu Thr Arg Gly Lys Phe Asn Thr
            325                 330                 335

Ser Asp Val Ser Ala Ile Glu Lys Asn Lys Glu Gly Leu His Asn Ala
            340                 345                 350

Lys Glu Ile Leu Thr Arg Leu Gly Val Glu Pro Ser Asp Asp Asp Cys
            355                 360                 365

Val Ser Val Gln His Val Cys Thr Ile Val Ser Phe Arg Ser Ala Asn
370                 375                 380

Leu Val Ala Ala Thr Leu Gly Ala Ile Leu Asn Arg Leu Arg Asp Asn

61

385 390 395 400

Lys Gly Thr Pro Arg Leu Arg Thr Thr Val Gly Val Asp Gly Ser Leu
405 410 415

Tyr Lys Thr His Pro Gln Tyr Ser Arg Arg Phe His Lys Thr Leu Arg
420 425 430

Arg Leu Val Pro Asp Ser Asp Val Arg Phe Leu Leu Ser Glu Ser Gly
435 440 445

Ser Gly Lys Gly Ala Ala Met Val Thr Ala Val Ala Tyr Arg Leu Ala
450 455 460

Glu Gln His Arg Gln Ile Glu Glu Thr Leu Ala His Phe His Leu Thr
465 470 475 480

Lys Asp Met Leu Leu Glu Val Lys Lys Arg Met Arg Ala Glu Met Glu
485 490 495

Leu Gly Leu Arg Lys Gln Thr His Asn Asn Ala Val Val Lys Met Leu
500 505 510

Pro Ser Phe Val Arg Arg Thr Pro Asp Gly Thr Glu Asn Gly Asp Phe
515 520 525

Leu Ala Leu Asp Leu Gly Gly Thr Asn Phe Arg Val Leu Leu Val Lys
530 535 540

Ile Arg Ser Gly Lys Lys Arg Thr Val Glu Met His Asn Lys Ile Tyr
545 550 555 560

Ala Ile Pro Ile Glu Ile Met Gln Gly Thr Gly Glu Glu Leu Phe Asp
565 570 575

His Ile Val Ser Cys Ile Ser Asp Phe Leu Asp Tyr Met Gly Ile Lys
580 585 590

Gly Pro Arg Met Pro Leu Gly Phe Thr Phe Ser Phe Pro Cys Gln Gln
595 600 605

Thr Ser Leu Asp Ala Gly Ile Leu Ile Thr Trp Thr Lys Gly Phe Lys
610 615 620

Ala Thr Asp Cys Val Gly His Asp Val Val Thr Leu Leu Arg Asp Ala
625 630 635 640

Ile Lys Arg Arg Glu Glu Phe Asp Leu Asp Val Val Ala Val Val Asn
645 650 655

```
Asp Thr Val Gly Thr Met Met Thr Cys Ala Tyr Glu Glu Pro Thr Cys
            660                 665                 670

Glu Val Gly Leu Ile Val Gly Thr Gly Ser Asn Ala Cys Tyr Met Glu
            675                 680                 685

Glu Met Lys Asn Val Glu Met Val Glu Gly Asp Gln Gly Gln Met Cys
            690                 695                 700

Ile Asn Met Glu Trp Gly Ala Phe Gly Asp Asn Gly Cys Leu Asp Asp
705                 710                 715                 720

Ile Arg Thr His Tyr Asp Arg Leu Val Asp Glu Tyr Ser Leu Asn Ala
            725                 730                 735

Gly Lys Gln Arg Tyr Glu Lys Met Ile Ser Gly Met Tyr Leu Gly Glu
            740                 745                 750

Ile Val Arg Asn Ile Leu Ile Asp Phe Thr Lys Lys Gly Phe Leu Phe
            755                 760                 765

Arg Gly Gln Ile Ser Glu Thr Leu Lys Thr Arg Gly Ile Phe Glu Thr
            770                 775                 780

Lys Phe Leu Ser Gln Ile Glu Ser Asp Arg Leu Ala Leu Leu Gln Val
785                 790                 795                 800

Arg Ala Ile Leu Gln Gln Leu Gly Leu Asn Ser Thr Cys Asp Asp Ser
            805                 810                 815

Ile Leu Val Lys Thr Val Cys Gly Val Val Ser Arg Arg Ala Ala Gln
            820                 825                 830

Leu Cys Gly Ala Gly Met Ala Ala Val Val Asp Lys Ile Arg Glu Asn
            835                 840                 845

Arg Gly Leu Asp Arg Leu Asn Val Thr Val Gly Val Asp Gly Thr Leu
            850                 855                 860

Tyr Lys Leu His Pro His Phe Ser Arg Ile Met His Gln Thr Val Lys
865                 870                 875                 880

Glu Leu Ser Pro Lys Cys Asn Val Ser Phe Leu Leu Ser Glu Asp Gly
            885                 890                 895

Ser Gly Lys Gly Ala Ala Leu Ile Thr Ala Val Gly Val Arg Leu Arg
            900                 905                 910
```

```
Thr Glu Ala Ser Ser
        915
```

<210> 27
<211> 917
<212> PRT
<213> Homo sapiens

<400> 27

```
Thr Glu Ala Ser Ser
```

```
Met Ile Ala Ser His Leu Leu Ala Tyr Phe Phe Thr Glu Leu Asn His
1               5               10              15

Asp Gln Val Gln Lys Val Asp Gln Tyr Leu Tyr His Met Arg Leu Ser
            20              25              30

Asp Glu Thr Leu Leu Glu Ile Ser Lys Arg Phe Arg Lys Glu Met Glu
        35              40              45

Lys Gly Leu Gly Ala Thr Thr His Pro Thr Ala Ala Val Lys Met Leu
    50              55              60

Pro Thr Phe Val Arg Ser Thr Pro Asp Gly Thr Glu His Gly Glu Phe
65              70              75              80

Leu Ala Leu Asp Leu Gly Gly Thr Asn Phe Arg Val Leu Trp Val Lys
            85              90              95

Val Thr Asp Asn Gly Leu Gln Lys Val Glu Met Glu Asn Gln Ile Tyr
        100             105             110

Ala Ile Pro Glu Asp Ile Met Arg Gly Ser Gly Thr Gln Leu Phe Asp
        115             120             125

His Ile Ala Glu Cys Leu Ala Asn Phe Met Asp Lys Leu Gln Ile Lys
    130             135             140

Asp Lys Lys Leu Pro Leu Gly Phe Thr Phe Ser Phe Pro Cys His Gln
145             150             155             160

Thr Lys Leu Asp Glu Ser Phe Leu Val Ser Trp Thr Lys Gly Phe Lys
            165             170             175

Ser Ser Gly Val Glu Gly Arg Asp Val Val Ala Leu Ile Arg Lys Ala
            180             185             190

Ile Gln Arg Arg Gly Asp Phe Asp Ile Asp Ile Val Ala Val Val Asn
        195             200             205

Asp Thr Val Gly Thr Met Met Thr Cys Gly Tyr Asp Asp His Asn Cys
```

210       215       220

Glu Ile Gly Leu Ile Val Gly Thr Gly Ser Asn Ala Cys Tyr Met Glu
225       230       235       240

Glu Met Arg His Ile Asp Met Val Glu Gly Asp Glu Gly Arg Met Cys
245       250       255

Ile Asn Met Glu Trp Gly Ala Phe Gly Asp Asp Gly Ser Leu Asn Asp
260       265       270

Ile Arg Thr Glu Phe Asp Gln Glu Ile Asp Met Gly Ser Leu Asn Pro
275       280       285

Gly Lys Gln Leu Phe Glu Lys Met Ile Ser Gly Met Tyr Met Gly Glu
290       295       300

Leu Val Arg Leu Ile Leu Val Lys Met Ala Lys Glu Glu Leu Leu Phe
305       310       315       320

Gly Gly Lys Leu Ser Pro Glu Leu Leu Asn Thr Gly Arg Phe Glu Thr
325       330       335

Lys Asp Ile Ser Asp Ile Glu Gly Glu Lys Asp Gly Ile Arg Lys Ala
340       345       350

Arg Glu Val Leu Met Arg Leu Gly Leu Asp Pro Thr Gln Glu Asp Cys
355       360       365

Val Ala Thr His Arg Ile Cys Gln Ile Val Ser Thr Arg Ser Ala Ser
370       375       380

Leu Cys Ala Ala Thr Leu Ala Ala Val Leu Gln Arg Ile Lys Glu Asn
385       390       395       400

Lys Gly Glu Glu Arg Leu Arg Ser Thr Ile Gly Val Asp Gly Ser Val
405       410       415

Tyr Lys Lys His Pro His Phe Ala Lys Arg Leu His Lys Thr Val Arg
420       425       430

Arg Leu Val Pro Gly Cys Asp Val Arg Phe Leu Arg Ser Glu Asp Gly
435       440       445

Ser Gly Lys Gly Ala Ala Met Val Thr Ala Val Ala Tyr Arg Leu Ala
450       455       460

Asp Gln His Arg Ala Arg Gln Lys Thr Leu Glu His Leu Gln Leu Ser
465       470       475       480

```
His Asp Gln Leu Leu Glu Val Lys Arg Arg Met Lys Val Glu Met Glu
              485             490             495

Arg Gly Leu Ser Lys Glu Thr His Ala Ser Ala Pro Val Lys Met Leu
              500             505             510

Pro Thr Tyr Val Cys Ala Thr Pro Asp Gly Thr Glu Lys Gly Asp Phe
              515             520             525

Leu Ala Leu Asp Leu Gly Gly Thr Asn Phe Arg Val Leu Leu Val Arg
              530             535             540

Val Arg Asn Gly Lys Trp Gly Gly Val Glu Met His Asn Lys Ile Tyr
545             550             555             560

Ala Ile Pro Gln Glu Val Met His Gly Thr Gly Asp Glu Leu Phe Asp
              565             570             575

His Ile Val Gln Cys Ile Ala Asp Phe Leu Glu Tyr Met Gly Met Lys
              580             585             590

Gly Val Ser Leu Pro Leu Gly Phe Thr Phe Ser Phe Pro Cys Gln Gln
              595             600             605

Asn Ser Leu Asp Glu Ser Ile Leu Leu Lys Trp Thr Lys Gly Phe Lys
              610             615             620

Ala Ser Gly Cys Glu Gly Glu Asp Val Val Thr Leu Leu Lys Glu Ala
625             630             635             640

Ile His Arg Arg Glu Glu Phe Asp Leu Asp Val Val Ala Val Val Asn
              645             650             655

Asp Thr Val Gly Thr Met Met Thr Cys Gly Phe Glu Asp Pro His Cys
              660             665             670

Glu Val Gly Leu Ile Val Gly Thr Gly Ser Asn Ala Cys Tyr Met Glu
              675             680             685

Glu Met Arg Asn Val Glu Leu Val Glu Gly Glu Glu Gly Arg Met Cys
              690             695             700

Val Asn Met Glu Trp Gly Ala Phe Gly Asp Asn Gly Cys Leu Asp Asp
705             710             715             720

Phe Arg Thr Glu Phe Asp Val Ala Val Asp Glu Leu Ser Leu Asn Pro
              725             730             735
```

67

```
Gly Lys Gln Arg Phe Glu Lys Met Ile Ser Gly Met Tyr Leu Gly Glu
            740                 745                 750

Ile Val Arg Asn Ile Leu Ile Asp Phe Thr Lys Arg Gly Leu Leu Phe
            755                 760                 765

Arg Gly Arg Ile Ser Glu Arg Leu Lys Thr Arg Gly Ile Phe Glu Thr
            770                 775                 780

Lys Phe Leu Ser Gln Ile Glu Ser Asp Cys Leu Ala Leu Leu Gln Val
785                 790                 795                 800

Arg Ala Ile Leu Gln His Leu Gly Leu Glu Ser Thr Cys Asp Asp Ser
                805                 810                 815

Ile Ile Val Lys Glu Val Cys Thr Val Val Ala Arg Arg Ala Ala Gln
            820                 825                 830

Leu Cys Gly Ala Gly Met Ala Ala Val Val Asp Arg Ile Arg Glu Asn
            835                 840                 845

Arg Gly Leu Asp Ala Leu Lys Val Thr Val Gly Val Asp Gly Thr Leu
    850                 855                 860

Tyr Lys Leu His Pro His Phe Ala Lys Val Met His Glu Thr Val Lys
865                 870                 875                 880

Asp Leu Ala Pro Lys Cys Asp Val Ser Phe Leu Gln Ser Glu Asp Gly
                885                 890                 895

Ser Gly Lys Gly Ala Ala Leu Ile Thr Ala Val Ala Cys Arg Ile Arg
            900                 905                 910

Glu Ala Gly Gln Arg
            915
```

<210> 28
<211> 923
<212> PRT
<213> Homo sapiens

<400> 28

```
Met Asp Ser Ile Gly Ser Ser Gly Leu Arg Gln Gly Glu Glu Thr Leu
1               5                   10                  15


Ser Cys Ser Glu Glu Gly Leu Pro Gly Pro Ser Asp Ser Ser Glu Leu
              20                  25                  30


Val Gln Glu Cys Leu Gln Gln Phe Lys Val Thr Arg Ala Gln Leu Gln
```

                    35                      40                         45


        Gln Ile Gln Ala Ser Leu Leu Gly Ser Met Glu Gln Ala Leu Arg Gly
            50              55              60

        Gln Ala Ser Pro Ala Pro Ala Val Arg Met Leu Pro Thr Tyr Val Gly
        65              70              75              80

        Ser Thr Pro His Gly Thr Glu Gln Gly Asp Phe Val Val Leu Glu Leu
                        85              90              95

        Gly Ala Thr Gly Ala Ser Leu Arg Val Leu Trp Val Thr Leu Thr Gly
                        100             105             110

        Ile Glu Gly His Arg Val Glu Pro Arg Ser Gln Glu Phe Val Ile Pro
                        115             120             125

        Gln Glu Val Met Leu Gly Ala Gly Gln Gln Leu Phe Asp Phe Ala Ala
            130             135             140

        His Cys Leu Ser Glu Phe Leu Asp Ala Gln Pro Val Asn Lys Gln Gly
        145             150             155             160

        Leu Gln Leu Gly Phe Ser Phe Ser Phe Pro Cys His Gln Thr Gly Leu
                        165             170             175

        Asp Arg Ser Thr Leu Ile Ser Trp Thr Lys Gly Phe Arg Cys Ser Gly
                        180             185             190

        Val Glu Gly Gln Asp Val Val Gln Leu Leu Arg Asp Ala Ile Arg Arg
                        195             200             205

        Gln Gly Ala Tyr Asn Ile Asp Val Val Ala Val Val Asn Asp Thr Val
            210             215             220

        Gly Thr Met Met Gly Cys Glu Pro Gly Val Arg Pro Cys Glu Val Gly
        225             230             235             240

        Leu Val Val Asp Thr Gly Thr Asn Ala Cys Tyr Met Glu Glu Ala Arg
                        245             250             255

        His Val Ala Val Leu Asp Glu Asp Arg Gly Arg Val Cys Val Ser Val
                        260             265             270

        Glu Trp Gly Ser Phe Ser Asp Asp Gly Ala Leu Gly Pro Val Leu Thr
                        275             280             285

        Thr Phe Asp His Thr Leu Asp His Glu Ser Leu Asn Pro Gly Ala Gln
            290             295             300


                                        70

Arg Phe Glu Lys Met Ile Gly Gly Leu Tyr Leu Gly Glu Leu Val Arg
305                 310             315             320

Leu Val Leu Ala His Leu Ala Arg Cys Gly Val Leu Phe Gly Gly Cys
                325             330             335

Thr Ser Pro Ala Leu Leu Ser Gln Gly Ser Ile Leu Leu Glu His Val
                340             345             350

Ala Glu Met Glu Asp Pro Ser Thr Gly Ala Ala Arg Val His Ala Ile
                355             360             365

Leu Gln Asp Leu Gly Leu Ser Pro Gly Ala Ser Asp Val Glu Leu Val
                370             375             380

Gln His Val Cys Ala Ala Val Cys Thr Arg Ala Ala Gln Leu Cys Ala
385             390             395             400

Ala Ala Leu Ala Ala Val Leu Ser Cys Leu Gln His Ser Arg Glu Gln
                405             410             415

Gln Thr Leu Gln Val Ala Val Ala Thr Gly Gly Arg Val Cys Glu Arg
                420             425             430

His Pro Arg Phe Cys Ser Val Leu Gln Gly Thr Val Met Leu Leu Ala
                435             440             445

Pro Glu Cys Asp Val Ser Leu Ile Pro Ser Val Asp Gly Gly Gly Arg
                450             455             460

Gly Val Ala Met Val Thr Ala Val Ala Ala Arg Leu Ala Ala His Arg
465             470             475             480

Arg Leu Leu Glu Glu Thr Leu Ala Pro Phe Arg Leu Asn His Asp Gln
                485             490             495

Leu Ala Ala Val Gln Ala Gln Met Arg Lys Ala Met Ala Lys Gly Leu
                500             505             510

Arg Gly Glu Ala Ser Ser Leu Arg Met Leu Pro Thr Phe Val Arg Ala
                515             520             525

Thr Pro Asp Gly Ser Glu Arg Gly Asp Phe Leu Ala Leu Asp Leu Gly
                530             535             540

Gly Thr Asn Phe Arg Val Leu Leu Val Arg Val Thr Thr Gly Val Gln
545             550             555             560

71

```
Ile Thr Ser Glu Ile Tyr Ser Ile Pro Glu Thr Val Ala Gln Gly Ser
            565             570             575

Gly Gln Gln Leu Phe Asp His Ile Val Asp Cys Ile Val Asp Phe Gln
            580             585             590

Gln Lys Gln Gly Leu Ser Gly Gln Ser Leu Pro Leu Gly Phe Thr Phe
            595             600             605

Ser Phe Pro Cys Arg Gln Leu Gly Leu Asp Gln Gly Ile Leu Leu Asn
        610             615             620

Trp Thr Lys Gly Phe Lys Ala Ser Asp Cys Glu Gly Gln Asp Val Val
625             630             635             640

Ser Leu Leu Arg Glu Ala Ile Thr Arg Arg Gln Ala Val Glu Leu Asn
            645             650             655

Val Val Ala Ile Val Asn Asp Thr Val Gly Thr Met Met Ser Cys Gly
            660             665             670

Tyr Glu Asp Pro Arg Cys Glu Ile Gly Leu Ile Val Gly Thr Gly Thr
            675             680             685

Asn Ala Cys Tyr Met Glu Glu Leu Arg Asn Val Ala Gly Val Pro Gly
        690             695             700

Asp Ser Gly Arg Met Cys Ile Asn Met Glu Trp Gly Ala Phe Gly Asp
705             710             715             720

Asp Gly Ser Leu Ala Met Leu Ser Thr Arg Phe Asp Ala Ser Val Asp
            725             730             735

Gln Ala Ser Ile Asn Pro Gly Lys Gln Arg Phe Glu Lys Met Ile Ser
            740             745             750

Gly Met Tyr Leu Gly Glu Ile Val Arg His Ile Leu Leu His Leu Thr
            755             760             765

Ser Leu Gly Val Leu Phe Arg Gly Gln Gln Ile Gln Arg Leu Gln Thr
        770             775             780

Arg Asp Ile Phe Lys Thr Lys Phe Leu Ser Glu Ile Glu Ser Asp Ser
785             790             795             800

Leu Ala Leu Arg Gln Val Arg Ala Ile Leu Glu Asp Leu Gly Leu Pro
            805             810             815
```

```
Leu Thr Ser Asp Asp Ala Leu Met Val Leu Glu Val Cys Gln Ala Val
        820                 825             830

Ser Gln Arg Ala Ala Gln Leu Cys Gly Ala Gly Val Ala Ala Val Val
        835                 840             845

Glu Lys Ile Arg Glu Asn Arg Gly Leu Glu Glu Leu Ala Val Ser Val
        850                 855             860

Gly Val Asp Gly Thr Leu Tyr Lys Leu His Pro Arg Phe Ser Ser Leu
865                 870                 875                 880

Val Ala Ala Thr Val Arg Glu Leu Ala Pro Arg Cys Val Val Thr Phe
                885                 890                 895

Leu Gln Ser Glu Asp Gly Ser Gly Lys Gly Ala Ala Leu Val Thr Ala
        900                 905                 910

Val Ala Cys Arg Leu Ala Gln Leu Thr Arg Val
        915                 920
```

<210> 29
<211> 465
<212> PRT
<213> Homo sapiens

<400> 29

```
Met Leu Asp Asp Arg Ala Arg Met Glu Ala Ala Lys Lys Glu Lys Val
1               5               10              15

Glu Gln Ile Leu Ala Glu Phe Gln Leu Gln Glu Glu Asp Leu Lys Lys
            20              25              30

Val Met Arg Arg Met Gln Lys Glu Met Asp Arg Gly Leu Arg Leu Glu
        35              40              45

Thr His Glu Glu Ala Ser Val Lys Met Leu Pro Thr Tyr Val Arg Ser
    50              55              60

Thr Pro Glu Gly Ser Glu Val Gly Asp Phe Leu Ser Leu Asp Leu Gly
65              70              75              80

Gly Thr Asn Phe Arg Val Met Leu Val Lys Val Gly Glu Gly Glu Glu
            85              90              95

Gly Gln Trp Ser Val Lys Thr Lys His Gln Met Tyr Ser Ile Pro Glu
            100             105             110

Asp Ala Met Thr Gly Thr Ala Glu Met Leu Phe Asp Tyr Ile Ser Glu
            115             120             125
```

74

Cys Ile Ser Asp Phe Leu Asp Lys His Gln Met Lys His Lys Lys Leu
    130             135             140

Pro Leu Gly Phe Thr Phe Ser Phe Pro Val Arg His Glu Asp Ile Asp
    145             150             155             160

Lys Gly Ile Leu Leu Asn Trp Thr Lys Gly Phe Lys Ala Ser Gly Ala
                165             170             175

Glu Gly Asn Asn Val Val Gly Leu Leu Arg Asp Ala Ile Lys Arg Arg
                180             185             190

Gly Asp Phe Glu Met Asp Val Val Ala Met Val Asn Asp Thr Val Ala
    195             200             205

Thr Met Ile Ser Cys Tyr Tyr Glu Asp His Gln Cys Glu Val Gly Met
    210             215             220

Ile Val Gly Thr Gly Cys Asn Ala Cys Tyr Met Glu Glu Met Gln Asn
225             230             235             240

Val Glu Leu Val Glu Gly Asp Glu Gly Arg Met Cys Val Asn Thr Glu
                245             250             255

Trp Gly Ala Phe Gly Asp Ser Gly Glu Leu Asp Glu Phe Leu Leu Glu
                260             265             270

Tyr Asp Arg Leu Val Asp Glu Ser Ser Ala Asn Pro Gly Gln Gln Leu
    275             280             285

Tyr Glu Lys Leu Ile Gly Gly Lys Tyr Met Gly Glu Leu Val Arg Leu
    290             295             300

Val Leu Leu Arg Leu Val Asp Glu Asn Leu Leu Phe His Gly Glu Ala
305             310             315             320

Ser Glu Gln Leu Arg Thr Arg Gly Ala Phe Glu Thr Arg Phe Val Ser
                325             330             335

Gln Val Glu Ser Asp Thr Gly Asp Arg Lys Gln Ile Tyr Asn Ile Leu
    340             345             350

Ser Thr Leu Gly Leu Arg Pro Ser Thr Thr Asp Cys Asp Ile Val Arg
    355             360             365

Arg Ala Cys Glu Ser Val Ser Thr Arg Ala Ala His Met Cys Ser Ala
    370             375             380

```
Gly Leu Ala Gly Val Ile Asn Arg Met Arg Glu Ser Arg Ser Glu Asp
385                 390             395             400

Val Met Arg Ile Thr Val Gly Val Asp Gly Ser Val Tyr Lys Leu His
            405             410             415

Pro Ser Phe Lys Glu Arg Phe His Ala Ser Val Arg Arg Leu Thr Pro
            420             425             430

Ser Cys Glu Ile Thr Phe Ile Glu Ser Glu Glu Gly Ser Gly Arg Gly
        435             440             445

Ala Ala Leu Val Ser Ala Val Ala Cys Lys Lys Ala Cys Met Leu Gly
    450             455             460

Gln
465
```

<210> 30
<211> 820
<212> PRT
<213> Escherichia coli

<400> 30

```
Met Arg Val Leu Lys Phe Gly Gly Thr Ser Val Ala Asn Ala Glu Arg
1               5               10              15

Phe Leu Arg Val Ala Asp Ile Leu Glu Ser Asn Ala Arg Gln Gly Gln
            20              25              30

Val Ala Thr Val Leu Ser Ala Pro Ala Lys Ile Thr Asn His Leu Val
        35              40              45

Ala Met Ile Glu Lys Thr Ile Ser Gly Gln Asp Ala Leu Pro Asn Ile
    50              55              60

Ser Asp Ala Glu Arg Ile Phe Ala Glu Leu Leu Thr Gly Leu Ala Ala
65              70              75              80

Ala Gln Pro Gly Phe Pro Leu Ala Gln Leu Lys Thr Phe Val Asp Gln
            85              90              95

Glu Phe Ala Gln Ile Lys His Val Leu His Gly Ile Ser Leu Leu Gly
            100             105             110

Gln Cys Pro Asp Ser Ile Asn Ala Ala Leu Ile Cys Arg Gly Glu Lys
        115             120             125

Met Ser Ile Ala Ile Met Ala Gly Val Leu Glu Ala Arg Gly His Asn
```

```
            130                    135                        140


        Val Thr Val Ile Asp Pro Val Glu Lys Leu Leu Ala Val Gly His Tyr
        145             150             155                 160


        Leu Glu Ser Thr Val Asp Ile Ala Glu Ser Thr Arg Arg Ile Ala Ala
                        165             170                 175


        Ser Arg Ile Pro Ala Asp His Met Val Leu Met Ala Gly Phe Thr Ala
                    180             185             190


        Gly Asn Glu Lys Gly Glu Leu Val Val Leu Gly Arg Asn Gly Ser Asp
                    195             200             205


        Tyr Ser Ala Ala Val Leu Ala Ala Cys Leu Arg Ala Asp Cys Cys Glu
            210             215             220


        Ile Trp Thr Asp Val Asp Gly Val Tyr Thr Cys Asp Pro Arg Gln Val
        225             230             235                 240


        Pro Asp Ala Arg Leu Leu Lys Ser Met Ser Tyr Gln Glu Ala Met Glu
                    245             250             255


        Leu Ser Tyr Phe Gly Ala Lys Val Leu His Pro Arg Thr Ile Thr Pro
                    260             265             270


        Ile Ala Gln Phe Gln Ile Pro Cys Leu Ile Lys Asn Thr Gly Asn Pro
                    275             280             285


        Gln Ala Pro Gly Thr Leu Ile Gly Ala Ser Arg Asp Glu Asp Glu Leu
            290             295             300


        Pro Val Lys Gly Ile Ser Asn Leu Asn Asn Met Ala Met Phe Ser Val
        305             310             315                 320


        Ser Gly Pro Gly Met Lys Gly Met Val Gly Met Ala Ala Arg Val Phe
                    325             330             335


        Ala Ala Met Ser Arg Ala Arg Ile Ser Val Val Leu Ile Thr Gln Ser
                    340             345             350


        Ser Ser Glu Tyr Ser Ile Ser Phe Cys Val Pro Gln Ser Asp Cys Val
            355             360             365


        Arg Ala Glu Arg Ala Met Gln Glu Glu Phe Tyr Leu Glu Leu Lys Glu
            370             375             380


        Gly Leu Leu Glu Pro Leu Ala Val Thr Glu Arg Leu Ala Ile Ile Ser
        385             390             395                 400
```

```
Val Val Gly Asp Gly Met Arg Thr Leu Arg Gly Ile Ser Ala Lys Phe
            405                 410                 415

Phe Ala Ala Leu Ala Arg Ala Asn Ile Asn Ile Val Ala Ile Ala Gln
            420                 425                 430

Gly Ser Ser Glu Arg Ser Ile Ser Val Val Val Asn Asn Asp Asp Ala
            435                 440                 445

Thr Thr Gly Val Arg Val Thr His Gln Met Leu Phe Asn Thr Asp Gln
    450                 455                 460

Val Ile Glu Val Phe Val Ile Gly Val Gly Gly Val Gly Gly Ala Leu
465                 470                 475                 480

Leu Glu Gln Leu Lys Arg Gln Gln Ser Trp Leu Lys Asn Lys His Ile
                485                 490                 495

Asp Leu Arg Val Cys Gly Val Ala Asn Ser Lys Ala Leu Leu Thr Asn
            500                 505                 510

Val His Gly Leu Asn Leu Glu Asn Trp Gln Glu Glu Leu Ala Gln Ala
        515                 520                 525

Lys Glu Pro Phe Asn Leu Gly Arg Leu Ile Arg Leu Val Lys Glu Tyr
        530                 535                 540

His Leu Leu Asn Pro Val Ile Val Asp Cys Thr Ser Ser Gln Ala Val
545                 550                 555                 560

Ala Asp Gln Tyr Ala Asp Phe Leu Arg Glu Gly Phe His Val Val Thr
                565                 570                 575

Pro Asn Lys Lys Ala Asn Thr Ser Ser Met Asp Tyr Tyr His Gln Leu
            580                 585                 590

Arg Tyr Ala Ala Glu Lys Ser Arg Arg Lys Phe Leu Tyr Asp Thr Asn
            595                 600                 605

Val Gly Ala Gly Leu Pro Val Ile Glu Asn Leu Gln Asn Leu Leu Asn
    610                 615                 620

Ala Gly Asp Glu Leu Met Lys Phe Ser Gly Ile Leu Ser Gly Ser Leu
625                 630                 635                 640

Ser Tyr Ile Phe Gly Lys Leu Asp Glu Gly Met Ser Phe Ser Glu Ala
                645                 650                 655
```

```
Thr Thr Leu Ala Arg Glu Met Gly Tyr Thr Glu Pro Asp Pro Arg Asp
        660             665             670

Asp Leu Ser Gly Met Asp Val Ala Arg Lys Leu Leu Ile Leu Ala Arg
        675             680             685

Glu Thr Gly Arg Glu Leu Glu Leu Ala Asp Ile Glu Ile Glu Pro Val
    690             695             700

Leu Pro Ala Glu Phe Asn Ala Glu Gly Asp Val Ala Ala Phe Met Ala
705             710             715             720

Asn Leu Ser Gln Leu Asp Asp Leu Phe Ala Ala Arg Val Ala Lys Ala
        725             730             735

Arg Asp Glu Gly Lys Val Leu Arg Tyr Val Gly Asn Ile Asp Glu Asp
        740             745             750

Gly Val Cys Arg Val Lys Ile Ala Glu Val Asp Gly Asn Asp Pro Leu
        755             760             765

Phe Lys Val Lys Asn Gly Glu Asn Ala Leu Ala Phe Tyr Ser His Tyr
    770             775             780

Tyr Gln Pro Leu Pro Leu Val Leu Arg Gly Tyr Gly Ala Gly Asn Asp
785             790             795             800

Val Thr Ala Ala Gly Val Phe Ala Asp Leu Leu Arg Thr Leu Ser Trp
        805             810             815

Lys Leu Gly Val
        820
```

\<210> 31
\<211> 810
\<212> PRT
\<213> Escherichia coli

\<400> 31

```
Met Ser Val Ile Ala Gln Ala Gly Ala Lys Gly Arg Gln Leu His Lys
1               5                   10                  15


Phe Gly Gly Ser Ser Leu Ala Asp Val Lys Cys Tyr Leu Arg Val Ala
          20                  25                  30


Gly Ile Met Ala Glu Tyr Ser Gln Pro Asp Asp Met Met Val Val Ser
          35                  40                  45


Ala Ala Gly Ser Thr Thr Asn Gln Leu Ile Asn Trp Leu Lys Leu Ser
```

50                          55                          60

Gln Thr Asp Arg Leu Ser Ala His Gln Val Gln Gln Thr Leu Arg Arg
65                  70                  75                  80

Tyr Gln Cys Asp Leu Ile Ser Gly Leu Leu Pro Ala Glu Glu Ala Asp
                85                  90                  95

Ser Leu Ile Ser Ala Phe Val Ser Asp Leu Glu Arg Leu Ala Ala Leu
                100                 105                 110

Leu Asp Ser Gly Ile Asn Asp Ala Val Tyr Ala Glu Val Val Gly His
                115                 120                 125

Gly Glu Val Trp Ser Ala Arg Leu Met Ser Ala Val Leu Asn Gln Gln
        130                 135                 140

Gly Leu Pro Ala Ala Trp Leu Asp Ala Arg Glu Phe Leu Arg Ala Glu
145                 150                 155                 160

Arg Ala Ala Gln Pro Gln Val Asp Glu Gly Leu Ser Tyr Pro Leu Leu
                165                 170                 175

Gln Gln Leu Leu Val Gln His Pro Gly Lys Arg Leu Val Val Thr Gly
                180                 185                 190

Phe Ile Ser Arg Asn Asn Ala Gly Glu Thr Val Leu Leu Gly Arg Asn
                195                 200                 205

Gly Ser Asp Tyr Ser Ala Thr Gln Ile Gly Ala Leu Ala Gly Val Ser
        210                 215                 220

Arg Val Thr Ile Trp Ser Asp Val Ala Gly Val Tyr Ser Ala Asp Pro
225                 230                 235                 240

Arg Lys Val Lys Asp Ala Cys Leu Leu Pro Leu Leu Arg Leu Asp Glu
                245                 250                 255

Ala Ser Glu Leu Ala Arg Leu Ala Ala Pro Val Leu His Ala Arg Thr
                260                 265                 270

Leu Gln Pro Val Ser Gly Ser Glu Ile Asp Leu Gln Leu Arg Cys Ser
                275                 280                 285

Tyr Thr Pro Asp Gln Gly Ser Thr Arg Ile Glu Arg Val Leu Ala Ser
        290                 295                 300

Gly Thr Gly Ala Arg Ile Val Thr Ser His Asp Asp Val Cys Leu Ile
305                 310                 315                 320

```
Glu Phe Gln Val Pro Ala Ser Gln Asp Phe Lys Leu Ala His Lys Glu
            325             330             335

Ile Asp Gln Ile Leu Lys Arg Ala Gln Val Arg Pro Leu Ala Val Gly
            340             345             350

Val His Asn Asp Arg Gln Leu Leu Gln Phe Cys Tyr Thr Ser Glu Val
            355             360             365

Ala Asp Ser Ala Leu Lys Ile Leu Asp Glu Ala Gly Leu Pro Gly Glu
    370             375             380

Leu Arg Leu Arg Gln Gly Leu Ala Leu Val Ala Met Val Gly Ala Gly
385             390             395             400

Val Thr Arg Asn Pro Leu His Cys His Arg Phe Trp Gln Gln Leu Lys
            405             410             415

Gly Gln Pro Val Glu Phe Thr Trp Gln Ser Asp Asp Gly Ile Ser Leu
            420             425             430

Val Ala Val Leu Arg Thr Gly Pro Thr Glu Ser Leu Ile Gln Gly Leu
            435             440             445

His Gln Ser Val Phe Arg Ala Glu Lys Arg Ile Gly Leu Val Leu Phe
    450             455             460

Gly Lys Gly Asn Ile Gly Ser Arg Trp Leu Glu Leu Phe Ala Arg Glu
465             470             475             480

Gln Ser Thr Leu Ser Ala Arg Thr Gly Phe Glu Phe Val Leu Ala Gly
            485             490             495

Val Val Asp Ser Arg Arg Ser Leu Leu Ser Tyr Asp Gly Leu Asp Ala
            500             505             510

Ser Arg Ala Leu Ala Phe Phe Asn Asp Glu Ala Val Glu Gln Asp Glu
            515             520             525

Glu Ser Leu Phe Leu Trp Met Arg Ala His Pro Tyr Asp Asp Leu Val
            530             535             540

Val Leu Asp Val Thr Ala Ser Gln Gln Leu Ala Asp Gln Tyr Leu Asp
545             550             555             560

Phe Ala Ser His Gly Phe His Val Ile Ser Ala Asn Lys Leu Ala Gly
            565             570             575
```

```
Ala Ser Asp Ser Asn Lys Tyr Arg Gln Ile His Asp Ala Phe Glu Lys
        580                 585             590

Thr Gly Arg His Trp Leu Tyr Asn Ala Thr Val Gly Ala Gly Leu Pro
        595                 600             605

Ile Asn His Thr Val Arg Asp Leu Ile Asp Ser Gly Asp Thr Ile Leu
    610                 615             620

Ser Ile Ser Gly Ile Phe Ser Gly Thr Leu Ser Trp Leu Phe Leu Gln
625                 630             635                 640

Phe Asp Gly Ser Val Pro Phe Thr Glu Leu Val Asp Gln Ala Trp Gln
                645             650                 655

Gln Gly Leu Thr Glu Pro Asp Pro Arg Asp Asp Leu Ser Gly Lys Asp
        660                 665             670

Val Met Arg Lys Leu Val Ile Leu Ala Arg Glu Ala Gly Tyr Asn Ile
        675                 680             685

Glu Pro Asp Gln Val Arg Val Glu Ser Leu Val Pro Ala His Cys Glu
    690                 695             700

Gly Gly Ser Ile Asp His Phe Phe Glu Asn Gly Asp Glu Leu Asn Glu
705                 710             715                 720

Gln Met Val Gln Arg Leu Glu Ala Ala Arg Glu Met Gly Leu Val Leu
                725             730                 735

Arg Tyr Val Ala Arg Phe Asp Ala Asn Gly Lys Ala Arg Val Gly Val
            740             745                 750

Glu Ala Val Arg Glu Asp His Pro Leu Ala Ser Leu Leu Pro Cys Asp
        755                 760             765

Asn Val Phe Ala Ile Glu Ser Arg Trp Tyr Arg Asp Asn Pro Leu Val
        770                 775             780

Ile Arg Gly Pro Gly Ala Gly Arg Asp Val Thr Ala Gly Ala Ile Gln
785                 790             795                 800

Ser Asp Ile Asn Arg Leu Ala Gln Leu Leu
                805             810
```

<210> 32
<211> 449

<212> PRT
<213> Escherichia coli

<400> 32

Met Ser Glu Ile Val Val Ser Lys Phe Gly Gly Thr Ser Val Ala Asp
1           5               10              15

Phe Asp Ala Met Asn Arg Ser Ala Asp Ile Val Leu Ser Asp Ala Asn
            20              25              30

Val Arg Leu Val Val Leu Ser Ala Ser Ala Gly Ile Thr Asn Leu Leu
            35              40              45

Val Ala Leu Ala Glu Gly Leu Glu Pro Gly Glu Arg Phe Glu Lys Leu
        50              55              60

Asp Ala Ile Arg Asn Ile Gln Phe Ala Ile Leu Glu Arg Leu Arg Tyr
65              70              75              80

Pro Asn Val Ile Arg Glu Glu Ile Glu Arg Leu Leu Glu Asn Ile Thr
            85              90              95

Val Leu Ala Glu Ala Ala Ala Leu Ala Thr Ser Pro Ala Leu Thr Asp
            100             105             110

Glu Leu Val Ser His Gly Glu Leu Met Ser Thr Leu Leu Phe Val Glu
        115             120             125

Ile Leu Arg Glu Arg Asp Val Gln Ala Gln Trp Phe Asp Val Arg Lys
    130             135             140

Val Met Arg Thr Asn Asp Arg Phe Gly Arg Ala Glu Pro Asp Ile Ala
145             150             155             160

Ala Leu Ala Glu Leu Ala Ala Leu Gln Leu Leu Pro Arg Leu Asn Glu
            165             170             175

Gly Leu Val Ile Thr Gln Gly Phe Ile Gly Ser Glu Asn Lys Gly Arg
            180             185             190

Thr Thr Thr Leu Gly Arg Gly Gly Ser Asp Tyr Thr Ala Ala Leu Leu
        195             200             205

Ala Glu Ala Leu His Ala Ser Arg Val Asp Ile Trp Thr Asp Val Pro
    210             215             220

Gly Ile Tyr Thr Thr Asp Pro Arg Val Val Ser Ala Ala Lys Arg Ile
225             230             235             240

Asp Glu Ile Ala Phe Ala Glu Ala Ala Glu Met Ala Thr Phe Gly Ala
            245             250             255

Lys Val Leu His Pro Ala Thr Leu Leu Pro Ala Val Arg Ser Asp Ile
            260                 265             270

Pro Val Phe Val Gly Ser Ser Lys Asp Pro Arg Ala Gly Gly Thr Leu
            275                 280             285

Val Cys Asn Lys Thr Glu Asn Pro Pro Leu Phe Arg Ala Leu Ala Leu
            290                 295             300

Arg Arg Asn Gln Thr Leu Leu Thr Leu His Ser Leu Asn Met Leu His
305             310                 315                 320

Ser Arg Gly Phe Leu Ala Glu Val Phe Gly Ile Leu Ala Arg His Asn
            325                 330                 335

Ile Ser Val Asp Leu Ile Thr Thr Ser Glu Val Ser Val Ala Leu Thr
            340                 345                 350

Leu Asp Thr Thr Gly Ser Thr Ser Thr Gly Asp Thr Leu Leu Thr Gln
            355                 360                 365

Ser Leu Leu Met Glu Leu Ser Ala Leu Cys Arg Val Glu Val Glu Glu
            370                 375                 380

Gly Leu Ala Leu Val Ala Leu Ile Gly Asn Asp Leu Ser Lys Ala Cys
385             390                 395                 400

Gly Val Gly Lys Glu Val Phe Gly Val Leu Glu Pro Phe Asn Ile Arg
            405                 410                 415

Met Ile Cys Tyr Gly Ala Ser Ser His Asn Leu Cys Phe Leu Val Pro
            420                 425                 430

Gly Glu Asp Ala Glu Gln Val Val Gln Lys Leu His Ser Asn Leu Phe
            435                 440                 445

Glu

<210> 33
<211> 468
<212> PRT
<213> Salmonella typhimurium

<400> 33

Ser Ala Glu His Val Leu Thr Met Leu Asn Glu His Glu Val Lys Phe
1               5                   10                  15

Val Asp Leu Arg Phe Thr Asp Thr Lys Gly Lys Glu Gln His Val Thr
20 25 30

Ile Pro Ala His Gln Val Asn Ala Glu Phe Phe Glu Glu Gly Lys Met
35 40 45

Phe Asp Gly Ser Ser Ile Gly Gly Trp Lys Gly Ile Asn Glu Ser Asp
50 55 60

Met Val Leu Met Pro Asp Ala Ser Thr Ala Val Ile Asp Pro Phe Phe
65 70 75 80

Ala Asp Ser Thr Leu Ile Ile Arg Cys Asp Ile Leu Glu Pro Gly Thr
85 90 95

Leu Gln Gly Tyr Asp Arg Asp Pro Arg Ser Ile Ala Lys Arg Ala Glu
100 105 110

Asp Tyr Leu Arg Ala Thr Gly Ile Ala Asp Thr Val Leu Phe Gly Pro
115 120 125

Glu Pro Glu Phe Phe Leu Phe Asp Asp Ile Arg Phe Gly Ala Ser Ile
130 135 140

Ser Gly Ser His Val Ala Ile Asp Asp Ile Glu Gly Ala Trp Asn Ser
145 150 155 160

Ser Thr Lys Tyr Glu Gly Gly Asn Lys Gly His Arg Pro Gly Val Lys
165 170 175

Gly Gly Tyr Phe Pro Val Pro Pro Val Asp Ser Ala Gln Asp Ile Arg
180 185 190

Ser Glu Met Cys Leu Val Met Glu Gln Met Gly Leu Val Val Glu Ala
195 200 205

His His His Glu Val Ala Thr Ala Gly Gln Asn Glu Val Ala Thr Arg
210 215 220

Phe Asn Thr Met Thr Lys Lys Ala Asp Glu Ile Gln Ile Tyr Lys Tyr
225 230 235 240

Val Val His Asn Val Ala His Arg Phe Gly Lys Thr Ala Thr Phe Met
245 250 255

Pro Lys Pro Met Phe Gly Asp Asn Gly Ser Gly Met His Cys His Met
260 265 270

Ser Leu Ala Lys Asn Gly Thr Asn Leu Phe Ser Gly Asp Lys Tyr Ala

```
                        275                     280                     285


        Gly Leu Ser Glu Gln Ala Leu Tyr Tyr Ile Gly Gly Val Ile Lys His
            290             295             300


        Ala Lys Ala Ile Asn Ala Leu Ala Asn Pro Thr Thr Asn Ser Tyr Lys
        305             310             315                     320


        Arg Leu Val Pro Gly Tyr Glu Ala Pro Val Met Leu Ala Tyr Ser Ala
                        325             330                     335


        Arg Asn Arg Ser Ala Ser Ile Arg Ile Pro Val Val Ala Ser Pro Lys
                        340             345             350


        Ala Arg Arg Ile Glu Val Arg Phe Pro Asp Pro Ala Ala Asn Pro Tyr
                        355             360             365


        Leu Cys Phe Ala Ala Leu Leu Met Ala Gly Leu Asp Gly Ile Lys Asn
            370             375             380


        Lys Ile His Pro Gly Glu Pro Met Asp Lys Asn Leu Tyr Asp Leu Pro
        385             390             395                     400


        Pro Glu Glu Ala Lys Glu Ile Pro Gln Val Ala Gly Ser Leu Glu Glu
                        405             410             415


        Ala Leu Asn Ala Leu Asp Leu Asp Arg Glu Phe Leu Lys Ala Gly Gly
                        420             425             430


        Val Phe Thr Asp Glu Ala Ile Asp Ala Tyr Ile Ala Leu Arg Arg Glu
                        435             440             445


        Glu Asp Asp Arg Val Arg Met Thr Pro His Pro Val Glu Phe Glu Leu
            450             455             460


        Tyr Tyr Ser Val
            465
```

<210> 34
<211> 468
<212> PRT
<213> Acidimicrobium ferrooxidans

<400> 34

Met Gly Tyr Ser Pro Ser Asp Val Val Lys Leu Ile Gln Glu Lys Asp
1                   5                   10                  15

Ile Lys Phe Ile Asp Phe Arg Phe Thr Asp Thr Lys Gly Lys Glu Gln
                20                  25                  30

His Val Ser Val Pro Gly His Val Ile Glu Glu Asp Thr Phe Thr Glu
        35              40                  45

Gly Lys Ala Phe Asp Gly Ser Ser Ile Ala Gly Trp Lys Gly Ile Asn
        50              55                  60

Glu Ser Asp Met Ile Leu Leu Pro Asp Pro Asp Ser Ala Val Leu Asp
65              70                  75                      80

Pro Phe Met Asp Glu Thr Thr Leu Leu Leu Arg Cys Asp Val Ile Glu
                85              90                      95

Pro Ala Thr Gly Gln Gly Tyr Glu Arg Asp Pro Arg Ser Val Ala Lys
            100             105             110

Arg Ala Glu Ile Tyr Leu Lys Ser Thr Gly Ile Ala Asp Thr Ser Phe
            115             120             125

Phe Gly Pro Glu Phe Phe Val Phe Asp Ser Val Thr Trp Asn Ile Asp
        130             135             140

Met Ser Gly Cys Ala Tyr Lys Val Asp Ala Glu Glu Ala Ala Trp Asn
145             150             155             160

Ser Gly Lys Glu Tyr Glu Ser Gly Asn Met Gly His Arg Leu Gly Val
                165             170             175

Lys Gly Gly Tyr Phe Pro Val Pro Val Asp Ser Ala Gln Asp Leu
                180             185             190

Arg Ser Ala Met Cys Leu Ala Met Glu Glu Met Gly Leu Lys Val Glu
        195             200             205

Val His His His Glu Val Ala Thr Ala Gly Gln His Glu Ile Gly Val
        210             215             220

Gly Phe Asn Thr Leu Thr Pro Arg Arg Met Arg Cys Lys Ile Leu Lys
225             230             235             240

Tyr Val Val His Asn Val Ala Ala Val Arg Gln Thr Ala Thr Phe Met
                245             250             255

Pro Lys Pro Val Val Gly Asp Asn Gly Ser Gly Met His Val His Gln
            260             265             270

Ser Leu Gly Lys Asp Gly Lys Asn Ile Phe Ala Gly Asp Leu Tyr Gly
            275             280             285

```
Gly Leu Ser Glu Ile Ala Leu Tyr Tyr Ile Gly Gly Ile Ile Lys His
    290                 295             300

Ala Lys Ala Val Asn Ala Leu Thr Asn Pro Ser Thr Asn Ser Tyr Lys
    305             310             315                 320

Arg Leu Val Pro His Phe Glu Ala Pro Val Leu Leu Ala Tyr Ser Ala
                325             330                 335

Lys Asn Arg Ser Ala Ser Ile Arg Ile Pro Tyr Val Asn Ser Pro Lys
            340             345             350

Ala Arg Arg Ile Glu Val Arg Phe Pro Asp Ser Thr Ala Asn Pro Tyr
            355             360             365

Leu Ala Phe Ser Ala Met Leu Met Ala Gly Leu Asp Gly Ile Gln Asn
    370             375             380

Lys Ile His Pro Ala Thr Ala Met Asp Lys Asn Leu Tyr Asp Leu Pro
385             390             395                 400

Ala Glu Glu Gln Ala Asn Ile Pro Gly Val Ala Ala Ser Leu Glu Glu
            405             410                 415

Ala Leu Arg Ala Leu Glu Ala Asp His Asp Phe Leu Met Lys Gly Gly
            420             425             430

Val Phe Ser Glu Ser Trp Leu Glu Gly Tyr Leu Asp Val Lys Trp Ala
    435             440                 445

Glu Val Gln Thr Leu Arg Val Thr Thr His Pro Val Glu Phe Gln Met
    450             455             460

Tyr Tyr Ser Leu
465
```

<210> 35
<211> 468
<212> PRT
<213> Escherichia coli

<400> 35

```
Ser Ala Glu His Val Leu Thr Met Leu Asn Glu His Glu Val Lys Phe
1                   5                   10                  15

Val Asp Leu Arg Phe Thr Asp Thr Lys Gly Lys Glu Gln His Val Thr
                20                  25                  30

Ile Pro Ala His Gln Val Asn Ala Glu Phe Phe Glu Glu Gly Lys Met
                35                  40                  45
```

```
Phe Asp Gly Ser Ser Ile Gly Gly Trp Lys Gly Ile Asn Glu Ser Asp
    50                  55                  60

Met Val Leu Met Pro Asp Ala Ser Thr Ala Val Ile Asp Pro Phe Phe
    65              70                  75                  80

Ala Asp Ser Thr Leu Ile Ile Arg Cys Asp Ile Leu Glu Pro Gly Thr
                85              90                  95

Leu Gln Gly Tyr Asp Arg Asp Pro Arg Ser Ile Ala Lys Arg Ala Glu
            100             105             110

Asp Tyr Leu Arg Ser Thr Gly Ile Ala Asp Thr Val Leu Phe Gly Pro
        115             120             125

Glu Pro Glu Phe Phe Leu Phe Asp Asp Ile Arg Phe Gly Ser Ser Ile
        130             135             140

Ser Gly Ser His Val Ala Ile Asp Asp Ile Glu Gly Ala Trp Asn Ser
145             150             155             160

Ser Thr Gln Tyr Glu Gly Gly Asn Lys Gly His Arg Pro Ala Val Lys
            165             170             175

Gly Gly Tyr Phe Pro Val Pro Pro Val Asp Ser Ala Gln Asp Ile Arg
            180             185             190

Ser Glu Met Cys Leu Val Met Glu Gln Met Gly Leu Val Val Glu Ala
        195             200             205

His His His Glu Val Ala Thr Ala Gly Gln Asn Glu Val Ala Thr Arg
    210             215             220

Phe Asn Thr Met Thr Lys Lys Ala Asp Glu Ile Gln Ile Tyr Lys Tyr
225             230             235             240

Val Val His Asn Val Ala His Arg Phe Gly Lys Thr Ala Thr Phe Met
            245             250             255

Pro Lys Pro Met Phe Gly Asp Asn Gly Ser Gly Met His Cys His Met
        260             265             270

Ser Leu Ser Lys Asn Gly Val Asn Leu Phe Ala Gly Asp Lys Tyr Ala
        275             280             285

Gly Leu Ser Glu Gln Ala Leu Tyr Tyr Ile Gly Gly Val Ile Lys His
    290             295             300
```

94

```
Ala Lys Ala Ile Asn Ala Leu Ala Asn Pro Thr Thr Asn Ser Tyr Lys
305             310             315                 320


Arg Leu Val Pro Gly Tyr Glu Ala Pro Val Met Leu Ala Tyr Ser Ala
            325             330                 335


Arg Asn Arg Ser Ala Ser Ile Arg Ile Pro Val Val Ser Ser Pro Lys
            340             345                 350


Ala Arg Arg Ile Glu Val Arg Phe Pro Asp Pro Ala Ala Asn Pro Tyr
            355             360             365


Leu Cys Phe Ala Ala Leu Leu Met Ala Gly Leu Asp Gly Ile Lys Asn
    370             375             380


Lys Ile His Pro Gly Glu Ala Met Asp Lys Asn Leu Tyr Asp Leu Pro
385             390             395                 400


Pro Glu Glu Ala Lys Glu Ile Pro Gln Val Ala Gly Ser Leu Glu Glu
                405             410             415


Ala Leu Asn Glu Leu Asp Leu Asp Arg Glu Phe Leu Lys Ala Gly Gly
            420             425             430


Val Phe Thr Asp Glu Ala Ile Asp Ala Tyr Ile Ala Leu Arg Arg Glu
        435             440             445


Glu Asp Asp Arg Val Arg Met Thr Pro His Pro Val Glu Phe Glu Leu
    450             455             460


Tyr Tyr Ser Val
465
```

<210> 36
<211> 491
<212> PRT
<213> Archaeoglobus fulgidus

<400> 36

```
Met Val Arg Arg Leu Arg Gly Asp Cys Met Glu Glu Val Glu Arg Ala
1               5                   10                  15

Lys Ala Val Leu Lys Glu Asn Asn Val Arg Gln Val Leu Cys Ala Phe
            20                  25                  30

Ala Asp Val Arg Gly Tyr Leu Gln Met Phe Ser Ile Pro Ala Arg Glu
            35                  40                  45

Phe Val Asp Gly Ser Ala Phe Glu Asn Gly Ile Gly Phe Asp Gly Ser
```

```
                    50                          55                          60


        Ser Val Arg Gly Phe Arg Thr Ile Glu Lys Ser Asp Met Val Trp Met
        65                  70                  75                  80


        Pro Asp Ala Ser Ser Leu Lys Ile Ile Pro Trp Ile Asp Asp Pro Ile
                        85                  90                  95


        Gln Lys Ser Ala Ile Met Phe Gly Asn Val Tyr Glu Ala Trp Gly Thr
                        100                 105                 110


        Glu Ile Ala Asp Cys Asp Pro Arg Gly Tyr Val Ala Lys Arg Tyr Glu
                    115                 120                 125


        Asp Met Leu Lys Ser Glu Gly Met Ser Ala Ile Phe Gly Pro Glu Ile
                    130                 135                 140


        Glu Phe Phe Leu Phe Glu Gly Val Asp Phe Thr Arg Leu Ser Trp Asp
        145                 150                 155                 160


        Met Trp Val Ser Pro Asn Gly Gly Ala Gly Asp Ser Trp Gly Pro Pro
                        165                 170                 175


        Arg Ile Met Pro Ile Ser Ser Glu Leu Glu Ser Gly Tyr Met Ile Arg
                    180                 185                 190


        Pro Lys Glu Gly Tyr Phe Arg Pro Pro Pro Glu Asp Thr Thr Val Glu
                    195                 200                 205


        Tyr Arg Asn Glu Leu Val Tyr Tyr Leu Glu Gln Leu Gly Ile Asp Ile
                    210                 215                 220


        Glu Tyr His His His Glu Val Ala Thr Ala Gly Gln Val Glu Leu Asp
        225                 230                 235                 240


        Phe Lys Pro Lys Gln Leu Val Asp Val Gly Asp Ala Phe Tyr Leu Tyr
                        245                 250                 255


        Lys Phe Ala Ala Lys Asn Ile Ala Ala Met His Gly Leu Tyr Ala Thr
                        260                 265                 270


        Phe Met Pro Lys Pro Leu Tyr Leu Asp Asn Ala Ser Gly Met His Thr
                    275                 280                 285


        His Gln Ser Leu Trp Lys Gly Glu Pro Phe Ser Gly Glu Ala Val Phe
                    290                 295                 300


        Ala Asp Pro Asp Asp Glu Tyr Met Leu Ser Gln Lys Ala Arg Tyr Tyr
        305                 310                 315                 320
```

97

```
Ile Gly Gly Leu Leu Glu His Ala Lys Ala Leu Thr Ala Leu Cys Ala
            325             330             335

Pro Thr Val Asn Ser Tyr Lys Arg Leu Val Pro Gly Phe Glu Ala Pro
            340             345             350

Ile Tyr Ile Cys Trp Ser Pro Arg Asn Arg Ser Ala Leu Val Arg Val
            355             360             365

Pro Met Tyr Val Lys Lys Pro Ser Ala Ile Arg Val Glu Tyr Arg Gly
    370             375             380

Val Asp Pro Ser Cys Asn Pro Tyr Leu Ala Ile Thr Ala Gln Leu Ala
385             390             395             400

Ala Gly Leu Asp Gly Ile Lys Lys Lys Ile Asp Pro Gly Asp Pro Leu
            405             410             415

Leu Glu Asp Val Tyr Glu Leu Thr Pro Ala Gln Lys Arg Glu Leu Gly
            420             425             430

Val Gly Glu Leu Pro Thr Thr Leu Arg Asp Ala Ile Asp His Leu Ala
            435             440             445

Ser Asp Glu Leu Met Gln Glu Val Leu Gly Ser His Ile Phe Asp Ala
    450             455             460

Phe Met Glu Leu Lys Ile Asp Glu Trp Asn Gln Tyr Cys Leu Tyr Ile
465             470             475             480

Thr Pro Trp Glu Phe Met Lys Tyr Phe Asp Ile
            485             490
```

<210> 37
<211> 467
<212> PRT
<213> Azotobacter vinelandii

<400> 37

```
Met Ser Lys Ser Leu Gln Leu Ile Lys Glu His Asp Val Lys Trp Ile
1           5           10              15

Asp Leu Arg Phe Thr Asp Thr Lys Gly Lys Gln Gln His Val Thr Met
            20              25              30

Pro Ala Arg Asp Val Asp Asp Phe Phe Glu Tyr Gly Lys Met Phe
            35              40              45
```

```
Asp Gly Ser Ser Ile Ala Gly Trp Lys Gly Ile Glu Ala Ser Asp Met
    50              55              60

Ile Leu Met Pro Asp Asp Ser Thr Ala Val Leu Asp Pro Phe Thr Glu
65              70              75              80

Glu Pro Thr Leu Ile Ile Val Cys Asp Ile Ile Glu Pro Ser Thr Met
            85              90              95

Gln Gly Tyr Asp Arg Asp Pro Arg Ala Ile Ala Arg Arg Ala Glu Glu
        100             105             110

Tyr Leu Lys Ser Thr Gly Ile Gly Asp Thr Ala Phe Phe Gly Pro Glu
        115             120             125

Pro Glu Phe Phe Ile Phe Asp Glu Val Lys Tyr Lys Ser Asp Ile Ser
    130             135             140

Gly Ser Met Phe Lys Ile Phe Ser Glu Gln Ala Ala Trp Asn Thr Asp
145             150             155             160

Ala Asp Phe Glu Gly Gly Asn Lys Gly His Arg Pro Gly Val Lys Gly
            165             170             175

Gly Tyr Phe Pro Val Pro Pro Val Asp His Asp His Glu Ile Arg Thr
        180             185             190

Ala Met Cys Asn Ala Leu Glu Glu Met Gly Leu Lys Val Glu Val His
        195             200             205

His His Glu Val Ala Thr Ala Gly Gln Asn Glu Ile Gly Val Ser Phe
    210             215             220

Asn Thr Leu Val Ala Lys Ala Asp Glu Val Gln Thr Leu Lys Tyr Cys
225             230             235             240

Val His Asn Val Ala Asp Ala Tyr Gly Lys Thr Val Thr Phe Met Pro
            245             250             255

Lys Pro Leu Tyr Gly Asp Asn Gly Ser Gly Met His Val His Met Ser
        260             265             270

Ile Ala Lys Asp Gly Lys Asn Thr Phe Ala Gly Glu Gly Tyr Ala Gly
        275             280             285

Leu Ser Asp Thr Ala Leu Tyr Phe Ile Gly Gly Ile Ile Lys His Gly
        290             295             300

Lys Ala Leu Asn Gly Phe Thr Asn Pro Ser Thr Asn Ser Tyr Lys Arg
```

                305                    310                    315                    320

Leu Val Pro Gly Phe Glu Ala Pro Val Met Leu Ala Tyr Ser Ala Arg
              325                    330                    335

Asn Arg Ser Ala Ser Ile Arg Ile Pro Tyr Val Asn Ser Pro Lys Ala
          340                    345                    350

Arg Arg Ile Glu Ala Arg Phe Pro Asp Pro Ser Ala Asn Pro Tyr Leu
          355                    360                    365

Ala Phe Ala Ala Leu Leu Met Ala Gly Leu Asp Gly Ile Gln Asn Lys
      370                    375                    380

Ile His Pro Gly Asp Ala Ala Asp Lys Asn Leu Tyr Asp Leu Pro Pro
385                    390                    395                    400

Glu Glu Ala Lys Glu Ile Pro Gln Val Cys Gly Ser Leu Lys Glu Ala
              405                    410                    415

Leu Glu Glu Leu Asp Lys Gly Arg Ala Phe Leu Thr Lys Gly Gly Val
          420                    425                    430

Phe Ser Asp Asp Phe Ile Asp Ala Tyr Leu Glu Leu Lys Ser Glu Glu
          435                    440                    445

Glu Ile Lys Val Arg Thr Phe Val His Pro Leu Glu Tyr Asp Leu Tyr
      450                    455                    460

Tyr Ser Val
465

<210> 38
<211> 443
<212> PRT
<213> Bacillus cereus

<400> 38

```
Ala Arg Tyr Thr Lys Glu Asp Ile Phe Arg Leu Ala Lys Glu Glu Asn
1               5                   10              15

Val Lys Tyr Ile Arg Leu Gln Phe Thr Asp Leu Leu Gly Val Ile Lys
            20                  25                  30

Asn Val Glu Ile Pro Val Ser Gln Leu Thr Lys Ala Leu Asp Asn Lys
            35                  40                  45

Met Met Phe Asp Gly Ser Ser Ile Glu Gly Phe Val Arg Ile Glu Glu
        50                  55                  60
```

```
Ser Asp Met Tyr Leu Tyr Pro Asp Leu Asp Thr Trp Val Ile Phe Pro
65                  70              75                  80

Trp Thr Ala Glu Lys Gly Lys Val Ala Arg Leu Ile Cys Asp Ile Tyr
                85              90                  95

Asn Ala Asp Gly Thr Pro Phe Glu Gly Asp Pro Arg Asn Asn Leu Lys
            100             105             110

Arg Val Leu Lys Glu Met Glu Ala Leu Gly Phe Ser Asp Phe Asn Leu
        115             120             125

Gly Pro Glu Pro Glu Phe Phe Leu Phe Lys Val Asp Glu Lys Gly Asn
    130             135             140

Pro Thr Leu Glu Leu Asn Asp Asn Gly Gly Tyr Phe Asp Leu Ala Pro
145             150             155             160

Met Asp Leu Gly Glu Asn Cys Arg Arg Asp Ile Val Leu Glu Leu Glu
            165             170             175

Glu Met Gly Phe Glu Ile Glu Ala Ser His His Glu Val Ala Pro Gly
            180             185             190

Gln His Glu Ile Asp Phe Lys Tyr Ala Asn Ala Ile Arg Ser Cys Asp
    195             200             205

Asp Ile Gln Thr Phe Lys Leu Val Val Lys Thr Ile Ala Arg Lys His
    210             215             220

Gly Leu His Ala Thr Phe Met Pro Lys Pro Leu Tyr Gly Val Asn Gly
225             230             235             240

Ser Gly Met His Cys Asn Leu Ser Leu Phe Lys Asn Gly Glu Asn Val
            245             250             255

Phe Tyr Asp Gln Asn Gly Asp Leu Gln Leu Ser Asp Asp Ala Arg His
            260             265             270

Phe Ile Ala Gly Ile Leu Lys His Ala Pro Ala Phe Thr Ala Val Ala
    275             280             285

Asn Pro Thr Val Asn Ser Tyr Lys Arg Leu Val Pro Gly Tyr Glu Ala
    290             295             300

Pro Cys Tyr Val Ala Trp Ser Ala Gln Asn Arg Ser Pro Leu Val Arg
305             310             315             320
```

102

Ile Pro Ala Ser Arg Gly Ile Ser Thr Arg Val Glu Val Arg Ser Val
                325                 330                 335

Asp Pro Ala Ala Asn Pro Tyr Leu Val Met Ala Thr Leu Leu Ala Ala
        340                 345                 350

Gly Leu Asp Gly Ile Lys Asn Lys Leu Thr Pro Pro Ala Ala Val Asp
        355                 360                 365

Arg Asn Ile Tyr Val Met Thr Lys Glu Glu Arg Glu Glu Ala Gly Ile
    370                 375                 380

Val Asp Leu Pro Ala Thr Leu Ala Gln Ala Leu Val Thr Leu Gln Ser
385                 390                 395                 400

Asn Glu Val Ile Ser Asn Ala Leu Gly Asp His Leu Leu Glu His Phe
                405                 410                 415

Ile Glu Ala Lys Glu Phe Glu Trp Asp Ile Phe Arg Thr Gln Val His
                420                 425                 430

Gln Trp Glu Arg Asp Gln Tyr Met Ser Leu Tyr
        435                 440

<210> 39
<211> 443
<212> PRT
<213> Bacillus subtilis

<400> 39

Ala Lys Tyr Thr Arg Glu Asp Ile Glu Lys Leu Val Lys Glu Glu Asn
1                   5                   10                  15

Val Lys Tyr Ile Arg Leu Gln Phe Thr Asp Ile Leu Gly Thr Ile Lys
            20                  25                  30

Asn Val Glu Ile Pro Val Ser Gln Leu Gly Lys Ala Leu Asp Asn Lys
            35                  40                  45

Val Met Phe Asp Gly Ser Ser Ile Glu Gly Phe Val Arg Ile Glu Glu
        50                  55                  60

Ser Asp Met Tyr Leu Tyr Pro Asp Leu Asn Thr Phe Val Ile Phe Pro
65                  70                  75                  80

Trp Thr Ala Glu Lys Gly Lys Val Ala Arg Phe Ile Cys Asp Ile Tyr
                85                  90                  95

Asn Pro Asp Gly Thr Pro Phe Glu Gly Asp Pro Arg Asn Asn Leu Lys
            100                 105                 110

Arg Ile Leu Lys Glu Met Glu Asp Leu Gly Phe Ser Asp Phe Asn Leu
        115                 120                 125

Gly Pro Glu Pro Glu Phe Phe Leu Phe Lys Leu Asp Glu Lys Gly Glu
        130                 135                 140

Pro Thr Leu Glu Leu Asn Asp Lys Gly Gly Tyr Phe Asp Leu Ala Pro
145                 150                 155                 160

Thr Asp Leu Gly Glu Asn Cys Arg Arg Asp Ile Val Leu Glu Leu Glu
                165                 170                 175

Glu Met Gly Phe Glu Ile Glu Ala Ser His His Glu Val Ala Pro Gly
            180                 185                 190

Gln His Glu Ile Asp Phe Lys Tyr Ala Gly Ala Val Arg Ser Cys Asp
        195                 200                 205

Asp Ile Gln Thr Phe Lys Leu Val Val Lys Thr Ile Ala Arg Lys His
        210                 215                 220

Gly Leu His Ala Thr Phe Met Pro Lys Pro Leu Phe Gly Val Asn Gly
225                 230                 235                 240

Ser Gly Met His Cys Asn Leu Ser Leu Phe Lys Asn Gly Val Asn Ala
                245                 250                 255

Phe Phe Asp Glu Asn Ala Asp Leu Gln Leu Ser Glu Thr Ala Lys His
            260                 265                 270

Phe Ile Ala Gly Ile Val Lys His Ala Thr Ser Phe Thr Ala Val Thr
        275                 280                 285

Asn Pro Thr Val Asn Ser Tyr Lys Arg Leu Val Pro Gly Tyr Glu Ala
        290                 295                 300

Pro Cys Tyr Val Ala Trp Ser Ala Gln Asn Arg Ser Pro Leu Ile Arg
305                 310                 315                 320

Ile Pro Ala Ser Arg Gly Ile Ser Thr Arg Val Glu Val Arg Ser Val
                325                 330                 335

Asp Pro Ala Ala Asn Pro Tyr Leu Ala Leu Ser Val Leu Leu Ala Ala
        340                 345                 350

Gly Leu Asp Gly Ile Lys Asn Lys Leu Glu Ala Pro Ala Pro Ile Asp
        355                 360                 365

```
Arg Asn Ile Tyr Val Met Ser Lys Glu Glu Arg Met Glu Asn Gly Ile
    370             375             380

Val Asp Leu Pro Ala Thr Leu Ala Glu Ala Leu Glu Glu Phe Lys Ser
385             390             395             400

Asn Glu Val Met Val Lys Ala Leu Gly Glu His Leu Phe Glu His Phe
                405             410             415

Ile Glu Ala Lys Glu Ile Glu Trp Asp Met Phe Arg Thr Gln Val His
            420             425             430

Pro Trp Glu Arg Glu Gln Tyr Met Ser Gln Tyr
            435             440
```

<210> 40
<211> 454
<212> PRT
<213> Halobacterium volcanii

<400> 40

Met Thr Glu Asp Asn Ala Leu Thr Asp Gly Gly Leu Ser Asp Glu Ala
1               5                   10                  15

Gln Ala Val Ile Asp Glu Ile Glu Glu Lys Asn Val Asp Phe Leu Arg
              20                  25                  30

Leu Gln Phe Thr Asp Ile Leu Gly Thr Val Lys Asn Val Ser Ile Pro
          35                  40                  45

Ala Ser Gln Ala Glu Lys Ala Phe Thr Glu Gly Ile Tyr Phe Asp Gly
      50                  55                  60

Ser Ser Ile Asp Gly Phe Val Arg Ile Gln Glu Ser Asp Met Arg Leu
65                  70                  75                  80

Glu Pro Asp Pro Ser Thr Phe Ala Val Leu Pro Trp Arg Lys Lys Glu
              85                  90                  95

Asn Ser Ala Ala Gly Arg Leu Ile Cys Asp Val Phe Asn Thr Ser Thr
              100                 105                 110

Gly Glu Pro Phe Ser Gly Asp Pro Arg Gly Val Leu Lys Arg Ala Ile
          115                 120                 125

Glu Pro Glu Glu Leu Gly Tyr Asp Val Asn Val Ala Pro Glu Pro Glu
      130                 135                 140

Phe Phe Leu Phe Glu Glu Asp Glu Asp Gly Arg Ala Thr Thr Val Thr

107

145                    150                    155                    160

Asn Asp Ala Gly Gly Tyr Phe Asp Leu Ala Pro Lys Asp Leu Ala Ser
            165              170              175

Asp Val Arg Arg Asp Ile Ile Tyr Gly Leu Glu Ser Met Gly Phe Asp
            180              185              190

Ile Glu Ala Ser His His Glu Val Ala Glu Gly Gln His Glu Ile Asn
            195              200              205

Phe Thr Tyr Asp Asp Ala Leu Ser Thr Ala Asp Asn Val Arg Thr Phe
    210              215              220

Arg Ser Val Val Arg Ala Ile Ala Ala Glu His Asp Leu His Ala Thr
225              230              235              240

Phe Met Pro Lys Pro Ile Pro Arg Ile Asn Gly Ser Gly Met His Thr
            245              250              255

His Ile Ser Leu Phe Lys Asp Gly Glu Asn Ala Phe His Asp Gly Asp
            260              265              270

Asp Glu Phe Asp Leu Ser Asp Thr Ala Lys Ser Phe Val Ala Gly Ile
            275              280              285

Leu Asp His Ala Pro Ala Ile Thr Ala Ser Leu Thr Arg Arg Ser Thr
    290              295              300

Pro Thr Arg Arg Leu Val Pro Gly Tyr Glu Ala Pro Val Tyr Ile Ala
305              310              315              320

Trp Ser Asp Arg Asn Arg Ser Ala Leu Ile Arg Lys Pro Ala Ala Arg
            325              330              335

Thr Pro Ala Ala Ser Arg Ile Glu Ala Arg Phe Pro Asp Pro Ser Cys
            340              345              350

Asn Pro Tyr Leu Ala Phe Ala Ala Leu Ile His Ala Gly Leu Asp Gly
            355              360              365

Val Glu Lys Gly Leu Asp Cys Pro Asp Pro Val Arg Glu Asn Ile Tyr
    370              375              380

Glu Phe Asp Glu Ala Lys Arg Glu Glu Tyr Gly Ile Glu Thr Leu Pro
385              390              395              400

Lys Thr Ser Ala Ala Arg Arg Arg Pro Arg Arg Asp Glu Val Ile Gln
            405              410              415

```
        Glu Ala Leu Gly Asp His Val Phe Glu Lys Phe Val Glu Ala Lys Arg
                    420             425             430

        Ser Glu Phe Lys Asp Tyr Leu Val Asp Val Ser Gln Trp Glu Leu Asp
                    435             440             445

        Arg Tyr Leu Glu Thr Phe
                    450
```

<210> 41
<211> 445
<212> PRT
<213> Lactobacillus delbrueckii

<400> 41

```
        Met Ser Lys Val Ile Thr Glu Glu Glu Ile Arg Lys Asp Val Glu Glu
        1               5               10              15

        Lys Asn Val Arg Phe Leu Arg Leu Ala Phe Thr Asp Ile Asn Gly Thr
                    20              25              30

        Leu Lys Asn Leu Glu Val Pro Val Ser Gln Leu Asp Asp Val Leu Gly
                    35              40              45

        Asn Gln Thr Arg Phe Asp Gly Ser Ser Ile Asp Gly Phe Val Arg Leu
                50              55              60

        Glu Glu Ser Asp Met Val Leu Tyr Pro Asp Leu Ala Thr Trp Leu Val
        65              70              75              80

        Leu Ala Trp Thr Thr Val Glu Glu Gly Thr Ile Gly Arg Leu Val Cys
                        85              90              95

        Ser Val His Asn Val Asp Gly Thr Pro Phe Glu Gly Asp Pro Arg Asn
                    100             105             110

        Asn Leu Lys Lys Val Ile Ala Glu Met Glu Glu Met Gly Phe Ser Asp
                    115             120             125

        Phe Glu Ile Gly Phe Glu Ala Glu Phe Phe Leu Phe Lys Glu Gly Lys
                130             135             140

        Asn Gly Glu Glu Thr Thr Lys Val Ser Asp His Ser Ser Tyr Phe Asp
        145             150             155             160

        Met Ala Ser Glu Asp Glu Gly Ala Lys Cys Arg Arg Glu Ile Val Glu
                        165             170             175
```

```
Thr Leu Glu Lys Leu Gly Phe Arg Val Glu Ala Ala His His Glu Val
          180                 185                 190

Gly Asp Gly Gln Gln Glu Ile Asp Phe Arg Phe Asp Asn Ala Leu Ala
          195                 200                 205

Thr Ala Asp Lys Leu Gln Thr Phe Lys Met Val Val Lys Thr Ile Ala
    210                 215                 220

Arg Lys Tyr His Leu His Ala Ser Phe Met Ala Lys Pro Val Glu Gly
225                 230                 235                 240

Leu Ala Gly Asn Gly Met His Thr Asn Met Ser Leu Leu Lys Asp Gly
              245                 250                 255

Lys Asn Ala Phe Tyr Asp Lys Asp Gly Gln Tyr Asn Leu Ser Thr Thr
          260                 265                 270

Ala Leu Thr Phe Leu Asn Gly Ile Leu Glu His Ala Arg Ala Ile Thr
          275                 280                 285

Cys Val Ala Asn Pro Thr Val Asn Ser Tyr Lys Arg Leu Ile Pro Gly
    290                 295                 300

Phe Glu Ala Pro Val Tyr Ile Ser Trp Ala Ser Arg Asn Arg Ser Pro
305                 310                 315                 320

Met Val Arg Ile Pro Asn Ala Asn Glu Val Gly Thr Arg Leu Glu Met
              325                 330                 335

Arg Ser Thr Asp Pro Thr Ala Asn Pro Tyr Leu Leu Leu Ser Ala Cys
          340                 345                 350

Leu Lys Ala Gly Leu Thr Gly Ile Lys Glu Gly Lys Leu Pro Met Ala
          355                 360                 365

Pro Val Thr Ser Asn Leu Phe Glu Met Thr Asp Asp Glu Arg Lys Glu
    370                 375                 380

Leu Gly Ile Lys Pro Leu Pro Ser Thr Leu His Asn Ala Ile Lys Ala
385                 390                 395                 400

Phe Lys Glu Asp Glu Val Val Lys Ser Ala Phe Ser Glu His Ile Val
              405                 410                 415

Asp Ser Phe Leu Glu Leu Lys Glu Thr Glu Trp Ala Leu Tyr Thr Gln
          420                 425                 430

Ser Val Ser Glu Trp Glu Val Lys Arg Tyr Phe Asn Tyr
```

435 440 445

<210> 42
<211> 428
<212> PRT
<213> Plasmodium falciparum

<400> 42

```
Met Cys Asp Ile Lys Arg Tyr Asn Gly Phe Asp Tyr Tyr Lys Cys Pro
1               5                   10                  15

Arg Thr Ile Leu Lys Lys Thr Cys Glu Phe Val Lys Asn Glu Gly Ile
            20                  25                  30

Ala Asp Lys Val Cys Ile Gly Asn Glu Leu Glu Phe Phe Ile Phe Asp
        35                  40                  45

Lys Val Asn Tyr Ser Leu Asp Glu Tyr Asn Thr Tyr Leu Lys Val Tyr
    50                  55                  60

Asp Arg Glu Ser Phe Ser Cys Lys Asn Asp Leu Ser Ser Ile Tyr Gly
65                  70                  75                  80

Asn His Val Val Asn Lys Val Glu Pro His Lys Asp His Phe Asn Asn
                85                  90                  95

Pro Asn Asn Glu Tyr Leu Ile Asn Asp Asp Ser Lys Lys Val Lys Lys
            100                 105                 110

Lys Ser Gly Tyr Phe Thr Thr Asp Pro Tyr Asp Thr Ser Asn Ile Ile
            115                 120                 125

Lys Leu Arg Ile Cys Arg Ala Leu Asn Asp Met Asn Ile Asn Val Gln
    130                 135                 140

Arg Tyr His His Glu Val Ser Thr Ser Gln His Glu Ile Ser Leu Lys
145                 150                 155                 160

Tyr Phe Asp Ala Leu Thr Asn Ala Asp Phe Leu Leu Ile Thr Lys Gln
            165                 170                 175

Ile Ile Lys Thr Thr Val Ser Ser Phe Asn Arg Thr Ala Thr Phe Met
            180                 185                 190

Pro Lys Pro Leu Val Asn Asp Asn Gly Asn Gly Leu His Cys Asn Ile
            195                 200                 205

Ser Leu Trp Lys Asn Asn Lys Asn Ile Phe Tyr His Asn Asp Pro Ser
    210                 215                 220
```

```
Thr Phe Phe Leu Ser Lys Glu Ser Phe Tyr Phe Met Tyr Gly Ile Val
225                 230             235                 240

Lys His Ala Lys Ala Leu Gln Ala Phe Cys Asn Ala Thr Met Asn Ser
                245             250                 255

Tyr Lys Arg Leu Val Pro Gly Phe Glu Thr Cys Gln Lys Leu Phe Tyr
                260             265                 270

Ser Phe Gly Ser Arg Ser Ala Val Ile Arg Leu Ser Leu Ile Asn Tyr
                275             280             285

Ser Asn Pro Ser Glu Lys Arg Ile Glu Phe Arg Leu Pro Asp Cys Ala
        290             295             300

Asn Ser Pro His Leu Val Met Ala Ala Ile Ile Leu Ala Gly Tyr Asp
305             310             315                 320

Gly Ile Lys Ser Lys Glu Gln Pro Leu Val Pro Phe Glu Ser Lys Asp
                325             330             335

Asn His Phe Tyr Ile Ser Ser Ile Phe Ser Lys Tyr Val Gln His Pro
                340             345             350

Glu Asn Phe Asn Ile Leu Thr His Ala Leu Glu Gly Tyr Glu Ser Leu
        355             360             365

His Thr Ile Asn Glu Ser Pro Glu Phe Lys Asn Phe Phe Lys Cys Glu
370             375             380

Glu Pro Gln Gly Ile Ser Phe Ser Leu Val Glu Ser Leu Asp Ala Leu
385             390             395                 400

Glu Lys Asp His Ala Phe Leu Thr Val Asn Asn Ile Phe Thr Glu Val
                405             410             415

Ser Gln Arg Ile Lys Asn Lys His Tyr His Gly Lys
                420             425
```

<210> 43
<211> 369
<212> PRT
<213> Saccharomyces cerevisiae

<400> 43

```
Ala Glu Ala Ser Ile Glu Lys Thr Gln Ile Leu Gln Lys Tyr Leu Glu
1               5                   10                  15
```

```
Leu Asp Gln Arg Gly Arg Ile Ile Ala Glu Tyr Val Trp Ile Asp Gly
```

|        |     |     | 20  |     |     |     | 25  |     |     |     | 30  |     |     |     |     |
|--------|-----|-----|-----|-----|-----|-----|-----|-----|-----|-----|-----|-----|-----|-----|-----|

Thr Gly Asn Leu Arg Ser Lys Gly Arg Thr Leu Lys Lys Arg Ile Thr
    35              40              45

Ser Ile Asp Gln Leu Pro Glu Trp Asn Phe Asp Gly Ser Ser Thr Asn
    50              55              60

Gln Ala Pro Gly His Asp Ser Asp Ile Tyr Leu Lys Pro Val Ala Tyr
65              70              75              80

Tyr Pro Asp Pro Phe Arg Arg Gly Asp Asn Ile Val Val Leu Ala Ala
            85              90              95

Cys Tyr Asn Asn Asp Gly Thr Pro Asn Lys Phe Asn His Arg His Glu
        100             105             110

Ala Ala Lys Leu Phe Ala Ala His Lys Asp Glu Glu Ile Trp Phe Gly
        115             120             125

Leu Glu Gln Glu Tyr Thr Leu Phe Asp Met Tyr Asp Asp Val Tyr Gly
    130             135             140

Trp Pro Lys Gly Gly Tyr Pro Ala Pro Gln Gly Pro Tyr Tyr Cys Gly
145             150             155             160

Val Gly Ala Gly Lys Val Tyr Ala Arg Asp Met Ile Glu Ala His Tyr
            165             170             175

Arg Ala Cys Leu Tyr Ala Gly Leu Glu Ile Ser Gly Ile Asn Ala Glu
        180             185             190

Val Met Pro Ser Gln Trp Glu Phe Gln Val Gly Pro Cys Thr Gly Ile
        195             200             205

Asp Met Gly Asp Gln Leu Trp Met Ala Arg Tyr Phe Leu His Arg Val
    210             215             220

Ala Glu Glu Phe Gly Ile Lys Ile Ser Phe His Pro Lys Pro Leu Lys
225             230             235             240

Gly Asp Trp Asn Gly Ala Gly Cys His Thr Asn Val Ser Thr Lys Glu
            245             250             255

Met Arg Gln Pro Gly Gly Met Lys Tyr Ile Glu Gln Ala Ile Glu Lys
        260             265             270

Leu Ser Lys Arg His Ala Glu His Ile Lys Leu Tyr Gly Ser Asp Asn
        275             280             285

Asp Met Arg Leu Thr Gly Arg His Glu Thr Ala Ser Met Thr Ala Phe
290                     295                 300

Ser Ser Gly Val Ala Asn Arg Gly Ser Ser Ile Arg Ile Pro Arg Ser
305                     310                 315                 320

Val Ala Lys Glu Gly Tyr Gly Tyr Phe Glu Asp Arg Arg Pro Ala Ser
                        325                 330                 335

Asn Ile Asp Pro Tyr Leu Val Thr Gly Ile Met Cys Glu Thr Val Cys
                340                 345                 350

Gly Ala Ile Asp Asn Ala Asp Met Thr Lys Glu Phe Glu Arg Glu Ser
                355                 360                 365

Ser

<210> 44
<211> 382
<212> PRT
<213> Chlamydomonas reinhardtii

<400> 44

Met Ala Ala Gly Ser Val Gly Val Phe Ala Thr Asp Glu Lys Ile Gly
1                   5                   10                  15

Ser Leu Leu Asp Gln Ser Ile Thr Arg His Phe Leu Ser Thr Val Thr
                20                  25                  30

Asp Gln Gln Gly Lys Ile Cys Ala Glu Tyr Val Trp Ile Gly Gly Ser
                35                  40                  45

Met His Asp Val Arg Ser Lys Ser Arg Thr Leu Ser Thr Ile Pro Thr
        50                  55                  60

Lys Pro Glu Asp Leu Pro His Trp Asn Tyr Asp Gly Ser Ser Thr Gly
65                  70                  75                  80

Gln Ala Pro Gly His Asp Ser Glu Val Tyr Leu Ile Pro Arg Ser Ile
                85                  90                  95

Phe Lys Asp Pro Phe Arg Gly Gly Asp Asn Ile Leu Val Met Cys Asp
                100                 105                 110

Cys Tyr Glu Pro Pro Lys Val Asn Pro Asp Gly Thr Leu Ala Ala Pro
                115                 120                 125

```
Lys Pro Ile Pro Thr Asn Thr Arg Phe Ala Cys Ala Glu Val Met Glu
    130             135             140

Lys Ala Lys Lys Glu Glu Pro Trp Phe Gly Ile Glu Gln Glu Tyr Thr
145             150             155             160

Leu Leu Asn Ala Ile Thr Lys Trp Pro Leu Gly Trp Pro Lys Gly Gly
                165             170             175

Tyr Pro Ala Pro Gln Gly Pro Tyr Tyr Cys Ser Ala Gly Ala Gly Val
            180             185             190

Ala Ile Gly Arg Asp Val Ala Glu Val His Tyr Arg Leu Cys Leu Ala
        195             200             205

Ala Gly Val Asn Ile Ser Gly Val Asn Ala Glu Val Leu Pro Ser Gln
    210             215             220

Trp Glu Tyr Gln Val Gly Pro Cys Glu Gly Ile Thr Met Gly Asp His
225             230             235             240

Met Trp Met Ser Arg Tyr Ile Met Tyr Arg Val Cys Glu Met Phe Asn
            245             250             255

Val Glu Val Ser Phe Asp Pro Lys Pro Ile Pro Gly Asp Trp Asn Gly
            260             265             270

Ser Gly Gly His Thr Asn Tyr Ser Thr Lys Ala Thr Arg Thr Ala Pro
            275             280             285

Asp Gly Trp Lys Val Ile Gln Glu His Cys Ala Lys Leu Glu Ala Arg
    290             295             300

His Ala Val His Ile Ala Ala Tyr Gly Glu Gly Asn Glu Arg Arg Leu
305             310             315             320

Thr Gly Lys His Glu Thr Ser Ser Met Ser Asp Phe Ser Trp Gly Val
            325             330             335

Ala Asn Arg Gly Cys Ser Ile Arg Val Gly Arg Met Val Pro Val Glu
            340             345             350

Lys Ser Gly Tyr Tyr Glu Asp Arg Arg Pro Ala Ser Asn Leu Asp Ala
            355             360             365

Tyr Val Val Thr Arg Leu Ile Val Glu Thr Thr Ile Leu Leu
    370             375             380
```

<210>  45

117

<211> 357
<212> PRT
<213> Zea mays

<400> 45

```
Met Ala Ser Leu Thr Asp Leu Val Asn Leu Asp Leu Ser Asp Cys Thr
1               5               10              15

Asp Arg Ile Ile Ala Glu Tyr Ile Trp Ile Gly Gly Thr Gly Ile Asp
            20              25              30

Leu Arg Ser Lys Ala Arg Thr Val Lys Gly Pro Ile Thr Asp Pro Ile
            35              40              45

Gln Leu Pro Lys Trp Asn Tyr Asp Gly Ser Ser Thr Gly Gln Ala Pro
    50              55              60

Gly Glu Asp Ser Glu Val Ile Leu Tyr Pro Gln Ala Ile Phe Lys Asp
65              70              75              80

Pro Phe Arg Lys Gly Asn His Ile Leu Val Met Cys Asp Cys Tyr Thr
            85              90              95

Pro Gln Gly Glu Pro Ile Pro Thr Asn Lys Arg Tyr Ser Ala Ala Lys
            100             105             110

Val Phe Ser His Pro Asp Val Ala Ala Glu Val Pro Trp Tyr Gly Ile
    115             120             125

Glu Gln Glu Tyr Thr Leu Leu Gln Lys Asp Val Ser Trp Pro Leu Gly
    130             135             140

Trp Pro Val Gly Gly Tyr Pro Gly Pro Gln Gly Pro Tyr Tyr Cys Ala
145             150             155             160

Ala Gly Ala Asp Lys Ala Phe Gly Arg Asp Val Val Asp Ala His Tyr
            165             170             175

Lys Ala Cys Leu Tyr Ala Gly Ile Asn Ile Ser Gly Ile Asn Gly Glu
    180             185             190

Val Met Pro Gly Gln Trp Glu Phe Gln Val Gly Pro Ser Val Gly Ile
    195             200             205

Ser Ala Gly Asp Glu Ile Trp Val Ala Arg Tyr Ile Leu Glu Arg Ile
    210             215             220

Thr Glu Met Ala Gly Ile Val Leu Ser Leu Asp Pro Lys Pro Ile Lys
225             230             235             240
```

119

```
Gly Asp Trp Asn Gly Ala Gly Ala His Thr Asn Tyr Ser Thr Lys Ser
                245                 250                 255

Met Arg Glu Ala Gly Gly Tyr Glu Val Ile Lys Ala Ala Ile Asp Lys
            260                 265                 270

Leu Gly Lys Arg His Lys Glu His Ile Ala Ala Tyr Gly Glu Gly Asn
            275                 280                 285

Glu Arg Arg Leu Thr Gly Arg His Glu Thr Ala Asp Ile Asn Thr Phe
    290                 295                 300

Lys Trp Gly Val Ala Asn Arg Gly Ala Ser Ile Arg Val Gly Arg Asp
305                 310                 315                 320

Thr Glu Arg Glu Gly Lys Gly Tyr Phe Glu Asp Arg Arg Pro Ala Ser
                325                 330                 335

Asn Met Asp Pro Tyr Val Val Thr Gly Met Ile Ala Glu Thr Thr Ile
            340                 345                 350

Leu Trp Asn Gly Asn
            355
```

<210> 46
<211> 357
<212> PRT
<213> Orysa sativa

<400> 46

Met Ala Asn Leu Thr Asp Leu Val Asn Leu Asn Leu Ser Asp Cys Ser
1              5                   10                  15

Asp Lys Ile Ile Ala Glu Tyr Ile Trp Val Gly Gly Ser Gly Ile Asp
           20                  25                  30

Leu Arg Ser Lys Ala Arg Thr Val Lys Gly Pro Ile Thr Asp Val Ser
           35                  40                  45

Gln Leu Pro Lys Trp Asn Tyr Asp Gly Ser Ser Thr Gly Gln Ala Pro
       50                  55                  60

Gly Glu Asp Ser Glu Val Ile Leu Tyr Pro Gln Ala Ile Phe Lys Asp
65              70                  75                  80

Pro Phe Arg Arg Gly Asp Asn Ile Leu Val Met Cys Asp Cys Tyr Thr
               85                  90                  95

Pro Gln Gly Glu Pro Ile Pro Thr Asn Lys Arg His Ser Ala Ala Lys

100                          105                          110

Ile Phe Ser His Pro Asp Val Val Ala Glu Val Pro Trp Tyr Gly Ile
        115             120             125

Glu Gln Glu Tyr Thr Leu Leu Gln Lys Asp Val Asn Trp Pro Leu Gly
    130             135             140

Trp Pro Val Gly Gly Phe Pro Gly Pro Gln Gly Pro Tyr Tyr Cys Ala
145             150             155             160

Ala Gly Ala Glu Lys Ala Phe Gly Arg Asp Ile Val Asp Ala His Tyr
            165             170             175

Lys Ala Cys Ile Tyr Ala Gly Ile Asn Ile Ser Gly Ile Asn Gly Glu
        180             185             190

Val Met Pro Gly Gln Trp Glu Phe Gln Val Gly Pro Ser Val Gly Ile
        195             200             205

Ala Ala Ala Asp Gln Val Trp Val Ala Arg Tyr Ile Leu Glu Arg Val
    210             215             220

Thr Glu Val Ala Gly Val Val Leu Ser Leu Asp Pro Lys Pro Ile Pro
225             230             235             240

Gly Asp Trp Asn Gly Ala Gly Ala His Thr Asn Phe Ser Thr Lys Ser
            245             250             255

Met Arg Glu Pro Gly Gly Tyr Glu Val Ile Lys Lys Ala Ile Asp Lys
        260             265             270

Leu Ala Leu Arg His Lys Glu His Ile Ala Ala Tyr Gly Glu Gly Asn
    275             280             285

Glu Arg Arg Leu Thr Gly Arg His Glu Thr Ala Asp Ile Asn Thr Phe
    290             295             300

Lys Trp Gly Val Ala Asn Arg Gly Ala Ser Ile Arg Val Gly Arg Asp
305             310             315             320

Thr Glu Lys Glu Gly Lys Gly Tyr Phe Glu Asp Arg Arg Pro Ala Ser
            325             330             335

Asn Met Asp Pro Tyr Val Val Thr Gly Met Ile Ala Glu Thr Thr Leu
            340             345             350

Leu Trp Lys Gln Asn
        355

122

<210> 47
<211> 353
<212> PRT
<213> Lupinus luteus

<400> 47

```
Met Ser Val Leu Ser Asp Leu Ile Asn Leu Asn Leu Ser Asp Thr Thr
1               5                   10                  15

Glu Lys Ile Ile Ala Glu Tyr Ile Trp Val Gly Gly Ser Gly Val Asp
            20                  25                  30

Leu Arg Ser Lys Ala Arg Thr Leu Ser Gly Pro Val Asn Asp Pro Ser
            35                  40                  45

Lys Leu Pro Lys Trp Asn Tyr Asp Gly Ser Ser Thr Gly Gln Ala Pro
        50                  55                  60

Gly Lys Asp Ser Glu Val Ile Leu Trp Pro Gln Ala Ile Phe Lys Asp
65                  70                  75                  80

Pro Phe Arg Arg Gly Asn Asn Ile Leu Val Met Cys Asp Thr Tyr Thr
                85                  90                  95

Pro Ala Gly Glu Pro Ile Pro Thr Asn Lys Arg His Ala Ala Ala Lys
                100                 105                 110

Ile Phe Ser His Pro Asp Val Val Ala Glu Glu Pro Trp Phe Gly Ile
            115                 120                 125

Glu Gln Glu Tyr Thr Leu Leu Gln Lys Asp Ile His Trp Pro Ile Gly
            130                 135                 140

Trp Pro Leu Gly Gly Phe Pro Gly Pro Gln Gly Pro Tyr Tyr Cys Gly
145                 150                 155                 160

Thr Gly Ala Glu Lys Ala Phe Gly Arg Asp Ile Val Asp Ser His Tyr
                165                 170                 175

Lys Ala Cys Leu Tyr Ala Gly Ile Asn Ile Ser Gly Ile Asn Ala Glu
            180                 185                 190

Val Met Pro Gly Gln Trp Glu Phe Gln Val Gly Pro Ser Val Gly Ile
            195                 200                 205

Ser Ala Gly Asp Glu Leu Trp Val Ala Arg Tyr Ile Leu Glu Arg Ile
            210                 215                 220
```

123

```
Thr Glu Ile Ala Gly Val Val Leu Ser Leu Asp Pro Lys Pro Ile Pro
225                 230             235                 240


Gly Asp Trp Asn Gly Ala Gly Ala His Thr Asn Tyr Ser Thr Lys Ser
                245                 250                 255


Met Arg Asn Asp Gly Gly Tyr Glu Val Ile Lys Lys Ala Ile Glu Lys
                260                 265                 270


Leu Gly Lys Arg His Asn Glu His Ile Ala Ala Tyr Gly Glu Gly Asn
                275                 280                 285


Glu Arg Arg Leu Thr Gly Arg His Glu Thr Ala Asp Ile Ser Thr Phe
    290             295                 300


Phe Trp Gly Val Ala Asn Arg Gly Ala Ser Ile Arg Val Gly Arg Asp
305                 310                 315                 320


Thr Glu Lys Glu Gly Lys Gly Tyr Phe Glu Asp Arg Arg Pro Ala Ser
                325                 330                 335


Asn Met Asp Pro Tyr Val Val Thr Ser Met Ile Ala Glu Thr Thr Leu
                340                 345                 350


Leu
```

<210> 48
<211> 357
<212> PRT
<213> Pisum sativum

<400> 48

```
Met Ser Ser Leu Ser Asp Leu Ile Asn Phe Asn Leu Ser Asp Ser Thr
1               5               10              15

Glu Lys Ile Ile Ala Glu Tyr Ile Trp Val Gly Gly Ser Gly Ile Asp
        20              25              30

Ile Arg Ser Lys Ala Arg Thr Leu Pro Gly Pro Val Ser Asp Pro Ala
        35              40              45

Lys Leu Pro Lys Trp Asn Tyr Asp Gly Ser Ser Thr Asn Gln Ala Pro
    50              55              60

Gly Lys Asp Ser Glu Val Ile Leu Tyr Pro Gln Ala Ile Phe Lys Asp
65              70              75              80

Pro Phe Arg Arg Gly Asn Asn Ile Leu Val Ile Cys Asp Val Tyr Thr
            85              90              95
```

```
Pro Ala Gly Glu Pro Leu Pro Thr Asn Lys Arg Tyr Asn Ala Ala Lys
        100                 105                 110

Ile Phe Ser His Pro Asp Val Ala Ala Glu Val Pro Trp Tyr Gly Ile
        115                 120                 125

Glu Gln Glu Tyr Thr Leu Leu Gln Lys Asp Ile Asn Trp Pro Leu Gly
        130                 135                 140

Trp Pro Ile Gly Gly Tyr Pro Gly Lys Gln Gly Pro Tyr Tyr Cys Gly
145                 150                 155                 160

Ile Gly Ala Asp Lys Ala Tyr Gly Arg Asp Ile Val Asp Ala His Tyr
                165                 170                 175

Lys Ala Cys Leu Phe Ala Gly Ile Asn Ile Ser Gly Ile Asn Gly Glu
        180                 185                 190

Val Met Pro Gly Gln Trp Glu Phe Gln Val Gly Pro Ser Val Gly Ile
        195                 200                 205

Ser Ala Gly Asp Glu Ile Trp Ala Ala Arg Tyr Ile Leu Glu Arg Ile
        210                 215                 220

Thr Glu Ile Ala Gly Val Val Val Ser Phe Asp Pro Lys Pro Ile Pro
225                 230                 235                 240

Gly Asp Trp Asn Gly Ala Gly Ala His Ala Asn Phe Ser Thr Lys Ser
                245                 250                 255

Met Arg Glu Asn Gly Gly Tyr Glu Val Ile Lys Lys Ala Ile Glu Lys
                260                 265                 270

Leu Gly Leu Arg His Lys Glu His Ile Ala Ala Tyr Gly Glu Gly Asn
                275                 280                 285

Glu Arg Arg Leu Thr Gly Lys His Glu Thr Ala Asp Ile Asn Val Phe
        290                 295                 300

Ser Trp Gly Val Ala Asn Arg Gly Ser Ser Ile Arg Val Gly Arg Asp
305                 310                 315                 320

Thr Glu Lys Asp Gly Lys Gly Tyr Phe Glu Asp Arg Arg Pro Ala Ser
                325                 330                 335

Asn Met Asp Pro Tyr Val Val Thr Ser Met Ile Ala Glu Thr Thr Ile
                340                 345                 350
```

```
Leu Trp Lys Lys Pro
        355
```

<210> 49
<211> 399
<212> PRT
<213> Drosophila melanogaster

<400> 49

```
Leu Trp Lys Lys Pro
```

Met Ala Leu Arg Val Ala Gly Leu Phe Leu Lys Lys Glu Leu Val Ala
1               5               10                15

Pro Ala Thr Gln Gln Leu Arg Leu Leu Arg Thr Gly Asn Thr Thr Arg
            20              25              30

Ser Gln Phe Leu Ala Asn Ser Pro Asn Thr Ala Leu Asp Lys Ser Ile
        35              40              45

Leu Gln Arg Tyr Arg Asn Leu Glu Thr Pro Ala Asn Arg Val Gln Ala
    50              55              60

Thr Tyr Leu Trp Ile Asp Gly Thr Gly Glu Asn Ile Arg Leu Lys Asp
65              70              75              80

Arg Val Leu Asp Lys Val Pro Ser Ser Val Glu Asp Leu Pro Asp Trp
            85              90              95

Gln Tyr Asp Gly Ser Ser Thr Tyr Gln Ala His Gly Glu Asn Ser Asp
            100             105             110

Thr Thr Leu Lys Pro Arg Ala Ile Tyr Arg Asp Pro Phe Lys Pro Gly
        115             120             125

Lys Asn Asp Val Ile Val Leu Cys Asp Thr Tyr Ser Ala Asp Gly Lys
    130             135             140

Pro Thr Ala Ser Asn Lys Arg Ala Ala Phe Gln Ala Ala Ile Asp Leu
145             150             155             160

Ile Ser Asp Gln Glu Pro Trp Phe Gly Ile Glu Gln Glu Tyr Thr Leu
            165             170             175

Leu Arg Arg Gly Arg Thr Ser Phe Gly Trp Pro Glu Asn Gly Phe Pro
            180             185             190

Ala Pro Gln Gly Pro Tyr Tyr Cys Gly Val Gly Ala Asp Arg Val Tyr
            195             200             205

Ala Arg Asp Leu Val Glu Ala His Val Val Ala Cys Leu Tyr Ala Gly

```
              210                    215                     220


    Ile Asp Phe Ala Gly Thr Asn Ala Glu Val Met Pro Ala Gln Trp Glu
    225                 230                 235                 240


    Phe Gln Ile Gly Pro Ala Gly Ile Lys Ala Cys Asp Asp Leu Trp Val
                    245                 250                 255


    Ser Arg Tyr Ile Leu Gln Arg Ile Ala Glu Glu Tyr Gly Val Val Val
                    260                 265                 270


    Thr Phe Asp Pro Lys Pro Met Glu Gly Gln Trp Asn Gly Ala Gly Arg
                    275                 280                 285


    His Thr Asn Phe Ser Thr Lys Glu Met Arg Ala Asp Gly Gly Ile Lys
        290                 295                 300


    Ala Ile Glu Glu Pro Ile Glu Lys Leu Ser Lys Arg His Glu Arg His
    305                 310                 315                 320


    Ile Lys Ala Tyr Tyr Pro Lys Glu Gly Lys Asp Asn Glu Arg Arg Leu
                    325                 330                 335


    Val Gly Arg Leu Glu Thr Ser Ser Ile Asp Lys Phe Ser Trp Gly Val
                    340                 345                 350


    Ala Asn Arg Ala Val Ser Val Arg Val Pro Arg Gly Val Ala Thr Ala
                355                 360                 365


    Gly Lys Gly Tyr Leu Glu Asp Arg Arg Pro Ser Ser Asn Cys Asp Pro
            370                 375                 380


    Tyr Ala Val Cys Asn Ala Ile Val Gln Thr Cys Leu Leu Asn Glu
    385                 390                 395
```

<210> 50
<211> 403
<212> PRT
<213> Squalus acanthia

<400> 50

Met Arg Ile Cys Arg Ser Phe Leu Phe Leu Val Lys Lys Cys Gly Asn
1                   5                   10                  15

Ile Thr Pro Thr Ile Trp Arg Asn Gln His Thr Tyr Lys Met Ala Thr
            20                  25                  30

Ser Ala Ser Ala Asn Leu Ser Lys Ile Val Lys Lys Asn Tyr Met Glu
            35                  40                  45

Leu Pro Gln Asp Gly Lys Val Gln Ala Met Tyr Ile Trp Ile Asp Gly
    50              55              60

Thr Gly Glu Ala Val Arg Cys Lys Thr Arg Thr Leu Asp Asn Glu Pro
65              70              75              80

Lys Ser Ile Ala Glu Leu Pro Glu Trp Asn Phe Asp Gly Ser Ser Thr
                85              90              95

Tyr Gln Ser Glu Gly Ser Asn Ser Asp Met Tyr Leu Val Pro Ser Ala
            100             105             110

Met Phe Arg Asp Pro Phe Arg Arg Asp Pro Asn Lys Leu Val Leu Cys
        115             120             125

Glu Val Leu Lys Tyr Asn Arg Lys Pro Ala Glu Ser Asn Leu Arg His
    130             135             140

Ser Cys Gln Lys Ile Met Ser Met Ile Ala Asn Glu Tyr Pro Trp Phe
145             150             155             160

Gly Met Glu Gln Glu Tyr Thr Leu Leu Gly Thr Asp Gly His Pro Phe
            165             170             175

Gly Trp Pro Ser Asn Cys Phe Pro Gly Pro Gln Gly Pro Tyr Tyr Cys
        180             185             190

Gly Val Gly Ala Asp Lys Ala Tyr Gly Arg Asp Ile Val Glu Ala His
        195             200             205

Tyr Arg Ala Cys Leu Tyr Ala Gly Ile Glu Leu Ser Gly Thr Asn Ala
    210             215             220

Glu Val Met Ala Ala Gln Trp Glu Tyr Gln Val Gly Pro Cys Glu Gly
225             230             235             240

Ile Gln Met Gly Asp His Leu Trp Ile Ser Arg Phe Ile Leu His Arg
            245             250             255

Val Cys Glu Asp Phe Gly Ile Ile Ala Ser Phe Asp Pro Lys Pro Ile
            260             265             270

Pro Gly Asn Trp Asn Gly Ala Gly Cys His Thr Asn Phe Ser Thr Lys
        275             280             285

Ala Met Arg Asp Asp Gly Gly Leu Lys Tyr Ile Glu Asp Ser Ile Glu
    290             295             300

```
Lys Leu Gly Lys Arg His Gln Tyr His Ile Arg Ala Tyr Asp Pro Lys
305                 310             315                 320


Gly Gly Leu Asp Asn Ala Arg Ala Leu Thr Gly His His Glu Thr Ser
                325             330                 335


Asn Ile Asn Glu Phe Ser Ala Gly Val Ala Asn Arg Gly Ala Ser Ile
                340             345                 350


Arg Ile Pro Arg Ser Val Gly Gln Asp Lys Lys Gly Tyr Phe Glu Asp
            355             360             365


Arg Arg Pro Ser Ala Asn Cys Asp Pro Tyr Ala Val Thr Glu Ala Leu
        370             375             380


Val Arg Thr Cys Leu Leu Asp Glu Ser Gly Asp Lys Pro Ile Glu Tyr
385             390             395                 400


Asn Lys Asn
```

<210> 51
<211> 392
<212> PRT
<213> Xenopus laevis

<400> 51

```
Met Ser Val Ser His Ser Ser Arg Leu Asn Lys Gly Val Arg Glu Gln
1               5               10              15

Tyr Met Lys Leu Pro Gln Gly Glu Lys Val Gln Val Thr Tyr Val Trp
        20              25              30

Ile Asp Gly Thr Gly Glu Gly Val Arg Cys Lys Thr Arg Thr Leu Asp
            35              40              45

Gln Glu Pro Lys Thr Ile Asp Glu Ile Pro Glu Trp Asn Phe Asp Gly
    50              55              60

Ser Ser Thr His Gln Ala Glu Gly Ser Asn Ser Asp Met Tyr Leu Ile
65              70              75              80

Pro Val Gln Met Phe Arg Asp Pro Phe Cys Leu Asp Pro Asn Lys Leu
            85              90              95

Val Met Cys Glu Val Leu Lys Tyr Asn Arg Lys Ser Ala Glu Thr Asn
            100             105             110

Leu Arg His Thr Cys Lys Lys Ile Met Glu Met Val Asn Asp His Arg
        115             120             125
```

```
Pro Trp Phe Gly Met Glu Gln Glu Tyr Thr Leu Leu Gly Ile Asn Gly
    130                 135                 140

His Pro Tyr Gly Trp Pro Glu Asn Gly Phe Pro Gly Pro Gln Gly Pro
    145                 150                 155                 160

Tyr Tyr Cys Gly Val Gly Ala Asp Lys Val Tyr Gly Arg Asp Val Val
                165                 170                 175

Glu Ser His Tyr Lys Ala Cys Leu Tyr Ala Gly Ile Lys Ile Cys Gly
            180                 185                 190

Thr Asn Ala Glu Val Met Pro Ser Gln Trp Glu Phe Gln Val Gly Pro
        195                 200                 205

Cys Glu Gly Ile Asp Met Gly Asp His Leu Trp Met Ala Arg Phe Ile
    210                 215                 220

Leu His Arg Val Cys Glu Asp Phe Gly Val Val Ala Thr Leu Asp Pro
225                 230                 235                 240

Lys Pro Met Thr Gly Asn Trp Asn Gly Ala Gly Cys His Thr Asn Tyr
                245                 250                 255

Ser Thr Glu Ser Met Arg Val Glu Gly Gly Leu Lys His Ile Glu Asp
            260                 265                 270

Ala Ile Glu Lys Leu Gly Lys Arg His Asp Tyr His Ile Cys Val Tyr
        275                 280                 285

Asp Pro Arg Gly Gly Lys Asp Asn Ser Arg Arg Leu Thr Gly Gln His
    290                 295                 300

Glu Thr Ser Ser Ile His Glu Phe Ser Ala Gly Val Ala Asn Arg Gly
305                 310                 315                 320

Ala Ser Ile Arg Ile Pro Arg Gln Val Gly Gln Glu Gly Tyr Gly Tyr
                325                 330                 335

Phe Glu Asp Arg Arg Pro Ala Ala Asn Cys Asp Pro Tyr Ala Val Thr
            340                 345                 350

Glu Ala Leu Val Arg Thr Thr Ile Leu Asn Glu Thr Gly Ser Glu Thr
        355                 360                 365

Lys Asp Tyr Lys Asn Gly Ala Gly Phe Ser Arg Ala Ile Gly Met Ala
    370                 375                 380
```

```
Ser Pro Arg Asp Ala Ala Val Phe
385                 390
```

<210> 52
<211> 373
<212> PRT
<213> Gallus gallus

<400> 52

```
Ser Pro Arg Asp Ala Ala Val Phe
```

```
Met Ala Thr Ser Ala Ser Ser His Leu Ser Lys Ala Ile Lys His Met
1               5                   10                  15

Tyr Met Lys Leu Pro Gln Gly Glu Lys Val Gln Ala Met Tyr Ile Trp
        20                  25                  30

Ile Asp Gly Thr Gly Glu His Leu Arg Cys Lys Thr Arg Thr Leu Asp
        35                  40                  45

His Glu Pro Lys Ser Leu Glu Asp Leu Pro Glu Trp Asn Phe Asp Gly
        50                  55                  60

Ser Ser Thr Phe Gln Ala Glu Gly Ser Asn Ser Asp Met Tyr Leu Arg
65                  70                  75                  80

Pro Ala Ala Met Phe Arg Asp Pro Phe Arg Lys Asp Pro Asn Lys Leu
                85                  90                  95

Val Leu Cys Glu Val Phe Lys Tyr Asn Arg Gln Ser Ala Asp Thr Asn
            100                 105                 110

Leu Arg His Thr Cys Arg Arg Ile Met Asp Met Val Ser Asn Gln His
        115                 120                 125

Pro Trp Phe Gly Met Glu Gln Glu Tyr Thr Leu Leu Gly Thr Asp Gly
        130                 135                 140

His Pro Phe Gly Trp Pro Ser Asn Cys Phe Pro Gly Pro Gln Gly Pro
145                 150                 155                 160

Tyr Tyr Cys Gly Val Gly Ala Asp Lys Ala Tyr Gly Arg Asp Ile Val
            165                 170                 175

Glu Ala His Tyr Arg Ala Cys Leu Tyr Ala Gly Val Lys Ile Gly Gly
        180                 185                 190

Thr Asn Ala Glu Val Met Pro Ala Gln Trp Glu Phe Gln Val Gly Pro
        195                 200                 205

Cys Glu Gly Ile Glu Met Gly Asp His Leu Trp Ile Ala Arg Phe Ile
```

```
               210                    215                        220


      Leu His Arg Val Cys Glu Asp Phe Gly Val Ile Val Ser Phe Asp Pro
      225             230             235                     240


      Lys Pro Ile Pro Gly Asn Trp Asn Gly Ala Gly Cys His Thr Asn Phe
                      245             250                 255


      Ser Thr Lys Asn Met Arg Glu Asp Gly Gly Leu Lys His Ile Glu Glu
                  260             265                 270


      Ala Ile Glu Lys Leu Ser Lys Arg His Gln Tyr His Ile Arg Ala Tyr
                  275             280                 285


      Asp Pro Lys Gly Gly Leu Asp Asn Ala Arg Arg Leu Thr Gly Phe His
          290             295                 300


      Glu Thr Ser Ser Ile His Glu Phe Ser Ala Gly Val Ala Asn Arg Gly
      305             310             315                     320


      Ala Ser Ile Arg Ile Pro Arg Asn Val Gly His Glu Lys Lys Gly Tyr
                      325             330                 335


      Phe Glu Asp Arg Gly Pro Ser Ala Asn Cys Asp Pro Tyr Ala Val Thr
                  340             345                 350


      Glu Ala Leu Val Arg Thr Cys Leu Leu Asn Glu Thr Gly Asp Glu Pro
                  355             360                 365


      Phe Glu Tyr Lys Asn
                  370
```

<210> 53
<211> 373
<212> PRT
<213> Mus musculus

<400> 53

```
Met Ala Thr Ser Ala Ser Ser His Leu Asn Lys Gly Ile Lys Gln Met
1               5               10              15

Tyr Met Ser Leu Pro Gln Gly Glu Lys Val Gln Ala Met Tyr Ile Trp
        20              25              30

Val Asp Gly Thr Gly Glu Gly Leu Arg Cys Lys Thr Arg Thr Leu Asp
        35              40              45

Cys Glu Pro Lys Cys Val Glu Glu Leu Pro Glu Trp Asn Phe Asp Gly
        50              55              60
```

```
Ser Ser Thr Phe Gln Ser Glu Gly Ser Asn Ser Asp Met Tyr Leu His
65              70              75                  80

Pro Val Ala Met Phe Arg Asp Pro Phe Arg Arg Asp Pro Asn Lys Leu
                85              90                  95

Val Leu Cys Glu Val Phe Lys Tyr Asn Arg Lys Pro Ala Glu Thr Asn
            100             105             110

Leu Arg His Ile Cys Lys Arg Ile Met Asp Met Val Ser Asn Gln His
            115             120             125

Pro Trp Phe Gly Met Glu Gln Glu Tyr Thr Leu Met Gly Thr Asp Gly
        130             135             140

His Pro Phe Gly Trp Pro Ser Asn Gly Phe Pro Gly Pro Gln Gly Pro
145             150             155             160

Tyr Tyr Cys Gly Val Gly Ala Asp Lys Ala Tyr Gly Arg Asp Ile Val
            165             170             175

Glu Ala His Tyr Arg Ala Cys Leu Tyr Ala Gly Val Lys Ile Thr Gly
            180             185             190

Thr Asn Ala Glu Val Met Pro Ala Gln Trp Glu Phe Gln Ile Gly Pro
        195             200             205

Cys Glu Gly Ile Arg Met Gly Asp His Leu Trp Ile Ala Arg Phe Ile
        210             215             220

Leu His Arg Val Cys Glu Asp Phe Gly Val Ile Ala Thr Phe Asp Pro
225             230             235             240

Lys Pro Ile Pro Gly Asn Trp Asn Val Ala Gly Cys His Thr Asn Phe
            245             250             255

Ser Thr Lys Ala Met Arg Glu Glu Asn Gly Leu Lys Cys Ile Glu Glu
            260             265             270

Ala Ile Asp Lys Leu Ser Lys Arg His Gln Tyr His Ile Arg Ala Tyr
            275             280             285

Asp Pro Lys Gly Gly Leu Asp Asn Ala Arg Ala Leu Thr Gly Phe His
            290             295             300

Glu Thr Ser Asn Ile Asn Asp Phe Ser Ala Gly Val Ala Asn Arg Gly
305             310             315             320
```

```
        Ala Ser Ile Arg Ile Pro Arg Thr Val Gly Gln Glu Lys Lys Gly Tyr
                        325             330             335


        Phe Glu Asp Arg Arg Leu Arg Ala Asn Cys Asp Pro Tyr Ala Val Thr
                    340             345             350


        Glu Ala Ile Val Arg Thr Cys Leu Leu Asn Glu Thr Gly Asp Glu Pro
                    355             360             365


        Phe Gln Tyr Lys Asn
            370
```

<210> 54
<211> 373
<212> PRT
<213> Cricetulus griseus

<400> 54

Met Ala Thr Ser Ala Ser Ser His Leu Asn Lys Gly Ile Lys Gln Met
1             5                 10                15

Tyr Met Ser Leu Pro Gln Gly Glu Lys Val Gln Ala Met Tyr Ile Trp
        20                    25                30

Val Asp Gly Thr Gly Glu Gly Leu Arg Cys Lys Thr Arg Thr Leu Asp
        35                40                45

Cys Glu Pro Lys Cys Val Glu Glu Leu Pro Glu Trp Asn Phe Asp Gly
    50                55                60

Ser Ser Thr Phe Gln Ser Glu Ser Ser Asn Ser Asp Met Tyr Leu Ser
65                70                75                80

Pro Val Ala Met Phe Arg Asp Pro Phe Arg Lys Glu Pro Asn Lys Leu
            85                90                95

Val Phe Cys Glu Val Phe Lys Tyr Asn Gln Lys Pro Ala Glu Thr Asn
        100               105               110

Leu Arg His Thr Cys Lys Arg Ile Met Asp Met Val Ser Asn Gln His
        115               120               125

Pro Trp Phe Gly Met Glu Gln Glu Tyr Thr Leu Leu Gly Thr Asp Gly
    130               135               140

His Pro Phe Gly Trp Pro Ser Asp Gly Phe Pro Gly Pro Gln Gly Leu
145                   150                   155               160

Tyr Tyr Cys Gly Val Gly Ala Asp Lys Ala Tyr Arg Arg Asp Ile Met
            165               170               175

```
Glu Ala His Tyr Arg Ala Cys Leu Tyr Ala Gly Val Lys Ile Thr Gly
        180                 185                 190

Thr Tyr Ala Glu Val Lys His Ala Gln Trp Glu Phe Gln Ile Gly Pro
        195                 200                 205

Cys Glu Gly Ile Arg Met Gly Asp His Leu Trp Val Ala Arg Phe Ile
    210                 215                 220

Leu His Arg Val Cys Lys Asp Phe Gly Val Ile Ala Thr Phe Asp Ser
225                 230                 235                 240

Lys Pro Ile Pro Gly Asn Trp Asn Gly Ala Gly Cys His Thr Asn Phe
                245                 250                 255

Ser Thr Lys Thr Met Arg Glu Glu Asn Gly Leu Lys His Ile Lys Glu
        260                 265                 270

Ala Ile Glu Lys Leu Ser Lys Arg His Arg Tyr His Ile Arg Ala Tyr
        275                 280                 285

Asp Pro Lys Gly Gly Leu Asp Asn Ala Arg Arg Leu Thr Gly Phe His
    290                 295                 300

Lys Thr Ser Asn Ile Asn Asp Phe Ser Ala Gly Val Ala Asp Arg Ser
305                 310                 315                 320

Ala Ser Ile Arg Ile Pro Arg Thr Val Gly Gln Glu Lys Lys Gly Tyr
                325                 330                 335

Phe Glu Ala Arg Cys Pro Ser Ala Asn Cys Asp Pro Phe Ala Val Thr
                340                 345                 350

Glu Ala Ile Val Arg Thr Cys Leu Leu Asn Glu Thr Gly Asp Gln Pro
        355                 360                 365

Phe Gln Tyr Lys Asn
        370
```

<210> 55
<211> 373
<212> PRT
<213> Homo sapiens

<400> 55

```
Met Thr Thr Ser Ala Ser Ser His Leu Asn Lys Gly Ile Lys Gln Val
1               5                   10                  15
```

```
Tyr Met Ser Leu Pro Gln Gly Glu Lys Val Gln Ala Met Tyr Ile Trp
            20              25              30

Ile Asp Gly Thr Gly Glu Gly Leu Arg Cys Lys Thr Arg Thr Leu Asp
            35              40              45

Ser Glu Pro Lys Cys Val Glu Glu Leu Pro Glu Trp Asn Phe Asp Gly
    50              55              60

Ser Ser Thr Leu Gln Ser Glu Gly Ser Asn Ser Asp Met Tyr Leu Val
65              70              75              80

Pro Ala Ala Met Phe Arg Asp Pro Phe Arg Lys Asp Pro Asn Lys Leu
            85              90              95

Val Leu Cys Glu Val Phe Lys Tyr Asn Arg Arg Pro Ala Glu Thr Asn
            100             105             110

Leu Arg His Thr Cys Lys Arg Ile Met Asp Met Val Ser Asn Gln His
            115             120             125

Pro Trp Phe Gly Met Glu Gln Glu Tyr Thr Leu Met Gly Thr Asp Gly
    130             135             140

His Pro Phe Gly Trp Pro Ser Asn Gly Phe Pro Gly Pro Gln Gly Pro
145             150             155             160

Tyr Tyr Cys Gly Val Gly Ala Asp Arg Ala Tyr Gly Arg Asp Ile Val
            165             170             175

Glu Ala His Tyr Arg Ala Cys Leu Tyr Ala Gly Val Lys Ile Ala Gly
            180             185             190

Thr Asn Ala Glu Val Met Pro Ala Gln Trp Glu Phe Gln Ile Gly Pro
            195             200             205

Cys Glu Gly Ile Ser Met Gly Asp His Leu Trp Val Ala Arg Phe Ile
    210             215             220

Leu His Arg Val Cys Glu Asp Phe Gly Val Ile Ala Thr Phe Asp Pro
225             230             235             240

Lys Pro Ile Pro Gly Asn Trp Asn Gly Ala Gly Cys His Thr Asn Phe
            245             250             255

Ser Thr Lys Ala Met Arg Glu Glu Asn Gly Leu Lys Tyr Ile Glu Glu
            260             265             270

Ala Ile Glu Lys Leu Ser Lys Arg His Gln Tyr His Ile Arg Ala Tyr
```

143

                    275                    280                    285

        Asp Pro Lys Gly Gly Leu Asp Asn Ala Arg Arg Leu Thr Gly Phe His
            290                    295                    300

        Glu Thr Ser Asn Ile Asn Asp Phe Ser Ala Gly Val Ala Asn Arg Ser
        305                    310                    315                    320

        Ala Ser Ile Arg Ile Pro Arg Thr Val Gly Gln Glu Lys Lys Gly Tyr
                    325                    330                    335

        Phe Glu Asp Arg Arg Pro Ser Ala Asn Cys Asp Pro Phe Ser Val Thr
                    340                    345                    350

        Glu Ala Leu Ile Arg Thr Cys Leu Leu Asn Glu Thr Gly Asp Glu Pro
                    355                    360                    365

        Phe Gln Tyr Lys Asn
            370

<210> 56
<211> 80
<212> DNA
<213> Synthetic oligo

<400> 56


gaggatccca gaaccagcgc catcagcgtt accatggcac cagctacaac cttgaaccaa        60

ttaaccctca ctaaagggcg                                                    80


<210> 57
<211> 81
<212> DNA
<213> Synthetic oligo

<400> 57


gtggatccgc gatatcgtga aacagcgcgg cgatgaaaat cagctcagtt gacggcagta        60

atacgactca ctatagggct c                                                  81


<210> 58
<211> 80
<212> DNA
<213> Synthetic oligo

<400> 58

```
gaggatcctg cgcctgtttg aactgacgca gcgcctcaag ctgttgctct tcgtcatcaa      60

ttaaccctca ctaaagggcg                                                   80
```

<210> 59
<211> 81
<212> DNA
<213> Synthetic oligo

<400> 59

```
gtggatccag gtgttccagc tcattcgcgg cggacgcgaa ccgtcgctgc aatcgcgcta      60

atacgactca ctatagggct c                                                81
```